# EUROPÄISCHE PATENTSCHRIFT

(11) **EP 3 481 916 B1**
(45) Veröffentlichungstag und Bekanntmachung des Hinweises auf die Patenterteilung: **03.11.2021**
(21) Anmeldenummer: 17739520.9
(22) Anmeldetag: 04.07.2017
(51) Int. Cl.: H01L 27/28, H01L 51/05, C09K 19/60

(54) **ELEKTRONISCHES SCHALTELEMENT**
ELECTRONIC SWITCH
COMMUTATEUR ÉLECTRONIQUE

(30) Priorität: 07.07.2016 DE 102016008207
(43) Veröffentlichungstag der Anmeldung: 15.05.2019
(73) Patentinhaber: Merck Patent GmbH, 64293 Darmstadt (DE)
(72) Erfinder: KIRSCH, Peer, 64342 SEEHEIM-JUGENHEIM (DE); RUHL, Andreas, 64380 Rossdorf (DE); TORNOW, Marc, 81825 Muenchen (DE); BORA, Achyut, 10711 Berlin (DE)
(86) Internationale Anmeldenummer: PCT/EP2017/066534
(87) Internationale Veröffentlichungsnummer: WO 2018/007337

(56) Entgegenhaltungen:
- EP-A1- 1 958 999
- EP-A1- 2 380 945
- EP-A1- 2 607 451
- EP-A2- 0 393 462
- WO-A1-2013/004372
- WO-A1-2017/045740
- WO-A2-2007/111994
- CN-B- 103 214 353
- DE-A1- 10 157 674
- DE-A1-102011 108 708
- DE-A1-102015 002 298
- DE-B4- 19 927 627
- JP-A- 2002 338 690
- US-A1- 2015 115 200
- US-A1- 2015 144 198
- FAN YANG ET AL: "Structure-property relationship of naphthalene based donor-[pi]-acceptor organic dyes for dye-sensitized solar cells: remarkable improvement of open-circuit photovoltage", JOURNAL OF MATERIALS CHEMISTRY, Bd. 22, Nr. 42, 1. Januar 2012 (2012-01-01), Seite 22550, XP055415006, GB ISSN: 0959-9428, DOI: 10.1039/c2jm34363k
- CHEN YING-ZHE ET AL: "Construction of polyaromatics via photocyclization of 2-(fur-3-yl)ethenylarenes, using a 3-furyl group as an isopropenyl equivalent synthon", TETRAHEDRON, ELSEVIER SCIENCE PUBLISHERS, AMSTERDAM, NL, Bd. 70, Nr. 9, 25. Januar 2014 (2014-01-25), Seiten 1748-1762, XP028605962, ISSN: 0040-4020, DOI: 10.1016/J.TET.2014.01.035
- ANDREA OCCHIPINTI ET AL: "Effectiveness and mode of action of phosphonate inhibitors of plant glutamine synthetase", PEST MANAGEMENT SCIENCE, vol. 66, no. 1, 1 January 2010 (2010-01-01), pages 51-58, XP055713068, ISSN: 1526-498X, DOI: 10.1002/ps.1830
- E. MONTONERI ET AL: "Organosulphur Phosphorus Acid Compounds. Part 7. Preparation and Analytical Identification of Difluorobenzylphosphono-Sulfonic Acids", PHOSPHORUS, SULFUR AND SILICON AND THE RELATED ELEMENTS, vol. 134, no. 1, 1 February 1998 (1998-02-01), pages 99-108, XP055713071, US ISSN: 1042-6507, DOI: 10.1080/10426509808545455

## Beschreibung

Die Erfindung betrifft ein memristives Bauelement (10) enthaltend ein oder mehrere Schaltelemente (1) umfassend, in dieser Reihenfolge, eine erste Elektrode (20), eine molekulare Schicht (18), gebunden an ein Substrat, und eine zweite Elektrode (16), wobei die molekulare Schicht durch Chemisorption oder kovalent an das Substrat gebunden ist, und wobei das Substrat aus der ersten Elektrode (20) und optional einer oder mehreren darüberliegenden Schichten besteht, und wobei die molekulare Schicht im wesentlichen aus einer oder mehreren Verbindungen der Formel I, wie nachfolgend und in den Ansprüchen definiert, gebildet wird. Weitere Aspekte der Erfindung betreffen Verbindungen zur Verwendung in der molekularen Schicht, die Verwendung der molekularen Schicht sowie Verfahren zur Herstellung und zum Betrieb memristiver Bauelemente.

In der Computertechnologie werden Speichermedien benötigt, die einen schnellen Schreib- und Lesezugriff auf darin abgelegte Informationen erlauben. Dabei erlauben solid-state Speicher bzw. Halbleiterspeicher die Realisierung besonders schneller und zuverlässiger Speichermedien, da keinerlei bewegliche Teile erforderlich sind. Derzeit werden hauptsächlich Dynamic Random Access Memory (DRAM) Speicher eingesetzt. DRAM erlaubt einen schnellen Zugriff auf die gespeicherten Informationen, jedoch müssen diese regelmäßig aufgefrischt werden, so daß beim Ausschalten der Stromversorgung die gespeicherten Informationen verloren gehen.

Im Stand der Technik sind auch nicht-flüchtige Halbleiterspeicher wie Flash-Speicher oder Magnetoresistive Random Access Memory (MRAM) bekannt, bei denen die Informationen auch nach dem Ausschalten der Stromversorgung erhalten bleiben. Dabei ist am Flash-Speicher nachteilig, daß ein Schreibzugriff vergleichsweise langsam erfolgt und die Speicherzellen des Flash-Speichers nicht beliebig oft gelöscht werden können. Typischerweise ist die Lebensdauer von Flash-Speicher auf maximal eine Million Schreib-Lese-Zyklen begrenzt. MRAM kann ähnlich wie DRAM verwendet werden und weist eine lange Lebensdauer auf, jedoch konnte sich dieser Speichertyp aufgrund der schwierigen Herstellungsverfahren nicht durchsetzen.

Eine weitere Alternative stellen Speicher dar, die auf Basis von Memristoren arbeiten. Der Begriff Memristor ist dabei aus den Englischen Wörtern "memory" und "resistor" (Speicher und Widerstand) zusammengesetzt und bezeichnet ein Bauelement, welches seinen elektrischen Widerstand reproduzierbar zwischen einem hohen und einem geringen elektrischen Widerstand ändern kann. Dabei bleibt der jeweilige Zustand (hoher Widerstand oder geringer Widerstand) auch ohne eine Versorgungsspannung erhalten, so daß sich mit Memristoren nichtflüchtige Speicher realisieren lassen.

Eine wichtige alternative Anwendung elektrisch schaltbarer Bauelemente ergibt sich für das Gebiet des neuromorphen oder synaptischen Rechnens. In dort verfolgten Computer-Architekturen sollen die Informationen nicht mehr klassisch sequentiell verarbeitet werden. Vielmehr wird angestrebt, die Schaltkreise hoch dreidimensional vernetzt aufzubauen, um eine Informationsverarbeitung analog zum Gehirn realisieren zu können. In derartigen, künstlichen neuronalen Netzwerken werden dann die biologischen Verbindungen zwischen Nervenzellen (Synapsen) durch die memristiven Schaltelemente dargestellt. Hierbei können unter Umständen auch zusätzliche Zwischenzustände (zwischen den digitalen Zuständen "1" und "0") von besonderem Nutzen sein.

Aus beispielsweise WO 2012/127542 A1 und US 2014/008601 A1 sind organische molekulare Speicher bekannt, die zwei Elektroden und eine aktive Region aufweisen, die zwischen den beiden Elektroden angeordnet ist. Die aktive Region weist eine molekulare Schicht elektrisch leitfähiger aromatischer Alkine auf, deren Leitfähigkeit unter dem Einfluß eines elektrischen Feldes verändert werden kann. Ähnliche Bauteile auf Basis von redox-aktiven Bipyridiniumverbindungen werden in US 2005/0099209 A1 vorgeschlagen.

Nachteilig an den bekannten auf einer Leitfähigkeits- bzw. Widerstandsänderung basierenden Speichern ist, daß die durch den Stromfluß durch die Moleküle der Monolage gebildeten radikalischen Zwischenstufen grundsätzlich für Degradationsprozesse anfällig sind, was sich nachteilig auf die Lebensdauer der Bauelemente auswirkt.

Aufgabe war es daher, nach neuen elektronischen Bauelementen zu suchen, die sich zur Anwendung in memristiven Vorrichtungen eignen und insbesondere im Hinblick auf eine oder mehrere der folgenden Eigenschaften Verbesserungen bringen:
- die Selektier- und Auslesbarkeit von zwei bezüglich ihres elektrischen Widerstands ausreichend unterschiedlichen Zuständen ("0", "1"), mit Hilfe eines elektrischen Feldes oder Stroms;
- die Schaltspannung sollte im Bereich einiger 100 mV bis zu einigen V liegen;
- die Auslesespannung sollten wesentlich kleiner als die Schreibspannung sein(typisches Verhältnis 1:10 [V]);
- der Lesestrom sollte mindestens 100 nA in Zustand "1" betragen;
- das Widerstandsverhältnis zwischen High-Resistance-State (HRS) (entspricht "0") und Low-Resistance-State (LRS) (entspricht "1"): R_{HRS} : R_{LRS} sollte 10 oder mehr betragen, besonders bevorzugt sollte das Widerstandsverhältnis 1000 oder mehr betragen;
- die Zugriffszeiten für Schreib- und Lesevorgang sollten bei 100 ns oder weniger liegen;
- die Langzeitstabilität der selektierten Zustände bei Raumtemperatur ohne die Notwendigkeit, diese periodisch aufzufrischen und somit eine kontinuierliche Stromversorgung aufrecht zu erhalten, sollte auch bei kontinuierlicher Auslesung mehr als 10 Jahre betragen;
- ein breiter Temperaturbereich für Betrieb und Lagerung unter Erhalt der gespeicherten Information ist wünschenswert;
- ein hoher Grad an Reversibilität des Schaltvorgangs ohne "Ermüdungserscheinungen" (> 1,0 ^{·} 10⁶ Schaltzyklen) ist wünschenswert ("Endurance");
- das Potential zu hohen Integrationsdichten bis hinunter in den molekularen Größenmaßstab (< 10 nm) sollte gegeben sein;
- die Kompatibilität mit den Standardmethoden, -prozessen, Schaltungsparametern und -designregeln der Siliziumelektronik (CMOS) sollte möglich sein;
- eine einfache und damit kostengünstige Device-Architektur sollte möglich sein.

Es wurde nun gefunden, daß diese Aufgaben zumindest in Teilbereichen lösbar sind, wenn die Schaltelemente entsprechender Bauelemente eine molekulare Schicht aus mesogenen Verbindungen mit negativer dielektrischer Anisotropie enthalten, die durch Reaktion der Oberfläche des Schaltelements mit einer terminalen Doppelbindung der mesogenen Verbindungen fixiert werden oder die durch Wechselwirkung der Oberfläche mit einer terminalen polaren Ankergruppe der mesogenen Verbindungen fixiert werden.

Mesogene Verbindungen mit negativer dielektrischer Anisotropie sind dem Fachmann bekannt, darunter auch solche, die in einer Seitenkette eine terminale Doppelbindung enthalten. So ist beispielsweise in der DE 199 27 627 B4 die folgende Verbindung offengelegt: 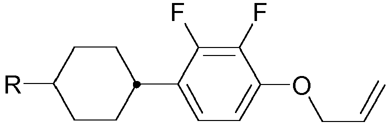 worin R u.a. einen Alkylrest bedeutet.

Eine verwandte Verbindung ist aus DE 10 2008 006875 A1 bekannt: 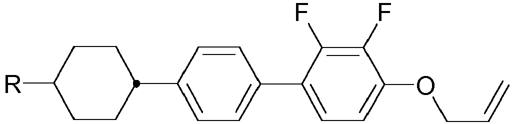 worin R u.a. einen Alkylrest bedeutet.

In der DE 10157674 A1 ist die folgende Verbindung offengelegt:

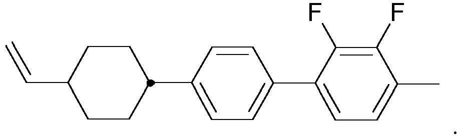

Eine weitere Klasse von Flüssigkristallen mit negativer dielektrischer Anisotropie bilden beispielsweise die in der DE 10 2015 002298 A1 offengelegten Difluordibenzofurane der nachfolgend gezeigten Struktur, worin ebenfalls Verbindungen mit terminaler C-C-Doppelbindung beschrieben sind:

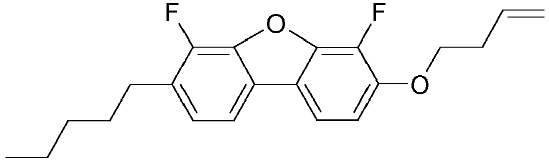

Mesogene Verbindungen mit terminaler polarer Ankergruppe sind aus dem Stand der Technik ebenfalls grundsätzlich bekannt. In der JP 2007 177051 A sind mesogene Verbindungen mit positiver dielektrischer Anisotropie beschrieben, die zur Derivatisierung von Eisenoxid-Nanopartikeln vorgeschlagen werden; die Bindung an die Partikel erfolgt hier durch am Ende der Seitenkette befindliche Phosphat-, Phosphonat- oder Carboxylatgruppen. In WO 2013/004372 A1 und WO 2014/169988 A1 sind mesogene Verbindungen offengelegt, die terminale Hydroxylgruppen tragen und zur Derivatisierung von Substraten für Flüssigkristallanzeigen mit dem Ziel einer homöotropen Ausrichtung des Flüssigkristalls dienen. Eine entsprechende Anwendung von dielektrisch neutralen und positiven mesogenen Verbindungen mit polaren Ankergruppen ist in der JP2005/002164 A offengelegt. Weitere Verbindungen und elektronische Bauelemente werden z.B in DE 199 27 627 B4 oder WO 2013/004372 A1 beschrieben.

In Angew. Chem. Int. Ed. 51 (2012), 4658 (H.J. Yoon et al.) und J. Am. Chem. Soc. 136 (2014) 16 - 19 (H.J. Yoon et al.) sind Anordnungen beschrieben, bei denen das elektronische Potential über Monoschichten von Alkylverbindungen mit polaren Endgruppen gemessen wird. Eine Eignung solcher Schichten zur Verwendung in Schaltelementen memristiver elektronischer Bauelemente lässt sich daraus nicht ableiten; mesogene Verbindungen oder Verbindungen mit negativer dielektrischer Anisotropie sind darin weder erwähnt noch ist deren Eignung nahegelegt.

Gegenstand der Erfindung ist daher ein memristives Bauelement (10) enthaltend ein oder mehrere Schaltelemente (1) umfassend, in dieser Reihenfolge, eine erste Elektrode (20),eine molekulare Schicht (18), gebunden an ein Substrat, und eine zweite Elektrode (16),wobei die molekulare Schicht durch Chemisorption oder kovalent an das Substrat gebunden ist, und wobei das Substrat aus der ersten Elektrode (20) und optional einer oder mehreren darüberliegenden Schichten besteht, und wobei die molekulare Schicht im wesentlichen aus einer oder mehreren Verbindungen der Formel I, wie nachfolgend definiert, gebildet wird:

R¹-(A¹-Z¹)ᵣ-B¹-(Z²-A²)ₛ-Sp-G (I)

worin
- R¹: H, einen Alkyl- oder Alkoxyrest mit 1 bis 15 C-Atomen, wobei in diesen Resten auch eine oder mehrere CH₂-Gruppen jeweils unabhängig voneinander durch -C≡C- , -CH=CH-, 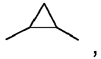 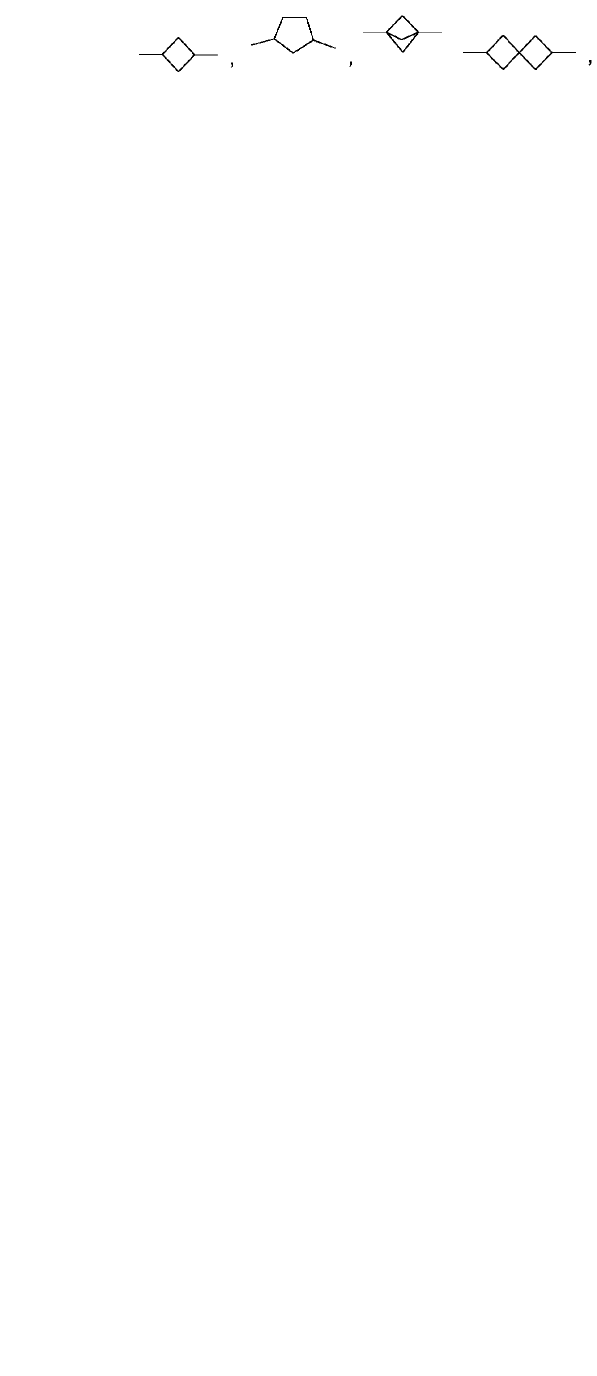 -O-, -S-, -CF₂O-, -OCF₂-, -CO-O-, -O-CO-, -SiR⁰R⁰⁰-, -NH-, -NR⁰-, oder
- -SO₂-,: so ersetzt sein können, daß O-Atome nicht direkt miteinander verknüpft sind, und worin auch ein oder mehrere H-Atome durch Halogen, CN, SCN oder SF₅ ersetzt sein können,
- R⁰, R⁰⁰: gleich oder verschieden einen Alkyl- oder Alkoxyrest mit 1 bis 15 C-Atomen, worin auch ein oder mehrere H-Atome durch Halogen ersetzt sein können,
- A¹, A²: bei jedem Auftreten gleich oder verschieden einen aromatischen, heteroaromatischen, alicyclischen or heteroaliphatischen Ring mit 4 bis 25 Ringatomen, der auch kondensierte Ringe enthalten kann und der ein- oder mehrfach durch Y substituiert sein kann^{,}
- Y: bei jedem Auftreten gleich oder verschieden F, Cl, CN, SCN, SF₅ oder geradkettiges oder verzweigtes, jeweils optional fluoriertes, Alkyl, Alkoxy, Alkylcarbonyl, Alkoxycarbonyl, Alkylcarbonyloxy oder Alkoxycarbonyloxy mit 1 bis 12 C-Atomen, bevorzugt F oder Cl,
- B¹: 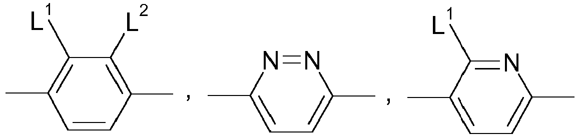 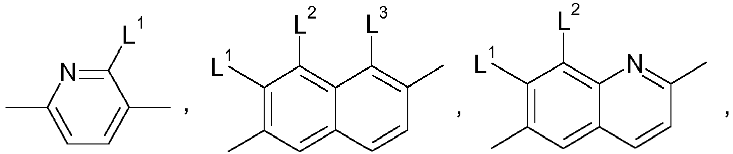 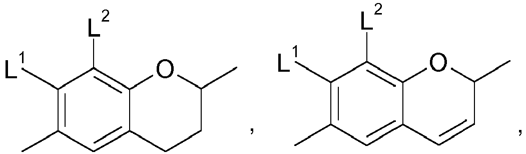 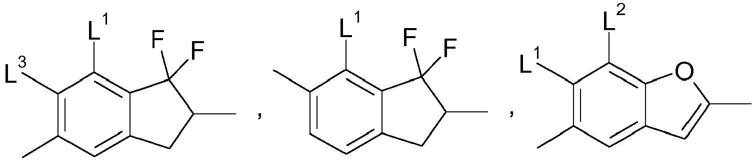 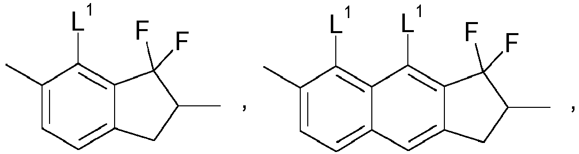 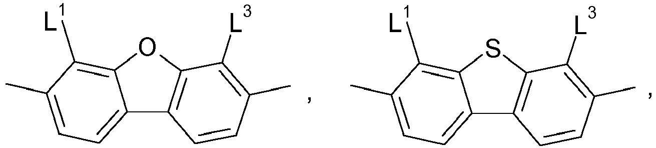 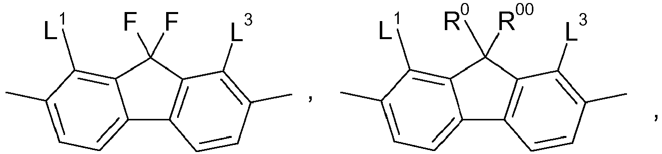 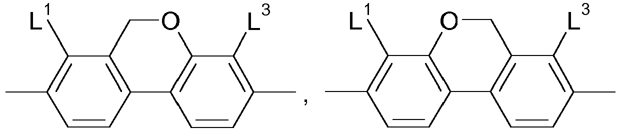 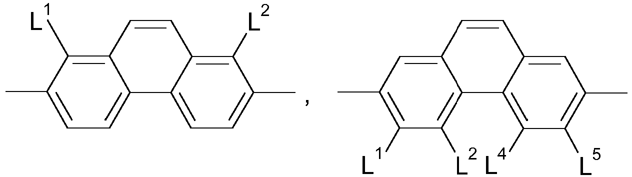 wobei die Gruppen in beide Richtungen orientiert sein können,
L¹bis L⁵ unabhängig voneinander F, Cl, Br, I, CN, SF₅, CF₃, OCF₃, bevorzugt Cl oder F, wobei L³ alternativ auch H bedeuten kann,
Z¹, Z² bei jedem Auftreten gleich oder verschieden eine Einfachbindung, -CF₂O-, -OCF₂-, -CF₂S-, -SCF₂-, -CH₂O-, -OCH₂- ,-C(O)O-, -OC(O)-, -C(O)S-, -SC(O)-, -CH₂-, -(CH₂)₂-, -(CH₂)₃-, -(CH₂)₄-, -CF₂-, -CF₂-CF₂-, -CF₂-CH₂-, -CH₂-CF₂-, -CH=CH-, -CF=CF- , -CF=CH-, -CH=CF-, -(CH₂)₃O-, -O(CH₂)₃-, -C≡C-, -O-, -S-, -C=N-, -N=C-, -N=N-, -N=N(O)-, -N(O)=N-, -N=C-C=N-,
Sp eine Abstandsgruppe oder eine Einfachbindung,
G -CH=CH₂, -OH, -SH, -SO₂OH, -OP(O)(OH)₂, -PO(OH)₂, -C(OH)(PO(OH)₂)₂, -COOH, -Si(OR^{x})₃, -SiCl₃,
R^{x} geradkettiges oder verzweigtes Alkyl mit 1 bis 6 C-Atomen, und
r und s unabhängig voneinander 0, 1, 2 oder 3,
wobei r + s ≤ 4 ist, bedeuten.

Die Schaltelemente sind insbesondere eingerichtet, zwischen einem Zustand mit hohem elektrischen Widerstand und einem Zustand mit geringem elektrischen Widerstand zu wechseln, wobei der Quotient zwischen hohem elektrischen Widerstand und niedrigem elektrischen Widerstand bevorzugt zwischen 10 und 100000 beträgt. Der elektrische Widerstand wird gemessen, indem eine Auslesespannung an das Schaltelement angelegt wird und der durch das Schaltelement fließende elektrische Strom gemessen wird. Der Wechsel zwischen den Zuständen erfolgt durch Anlegen einer Schaltspannung. Der Betrag der Auslesespannung ist geringer als der Betrag der Schaltspannung, wobei bevorzugt der Betrag der Auslesespannung maximal ein Zehntel des Betrags der kleinsten verwendeten Schaltspannung ist. Besonders bevorzugt ist es, wenn die Auslesespannung 10 mV oder mehr bis 300 mV oder weniger beträgt.

Gegenstand der Erfindung ist ein memristives Bauelement, insbesondere ein solches, das als Kreuzdrahtvorrichtung (engl.: "cross bar array") ausgeführt ist. Weiterer Gegenstand der Erfindung ist ein Verfahren zur Herstellung des erfindungsgemäßen memristiven Bauelements umfassend mindestens die folgenden Schritte:
i. Herstellung einer ersten Elektrode (20)
ii. Abscheidung einer Monolage (18) enthaltend eine oder mehrere Verbindungen der Formel I
iii. Aufbringen einer zweiten Elektrode (16).

Die Abscheidung der Monolage erfolgt mithilfe der Reinsubstanz oder aus Lösung, bevorzugt aus Lösung. Geeignete Abscheidungsverfahren und Lösungsmittel sind dem Fachmann bekannt; Beispiele sind Rotationsbeschichtung (engl. "spin coating") oder Tauchbeschichtung (engl.: "dip-coating").

In einer bevorzugten Ausführungsform wird das Substrat nach dem Abscheiden der Monolage getempert (engl.: "annealing"). Das Tempern erfolgt bei einer Temperatur von mehr als 20°C und weniger als 300°C, bevorzugt bei mehr als 50°C und weniger als 200°C, besonders bevorzugt bei mehr als 90°C und weniger als 150 °C. Die Zeitdauer des Temperns beträgt 1 bis 48 h, bevorzugt 4 bis 24 h, besonders bevorzugt 8 bis 16 h.

Die Herstellung und Strukturierung der Elektroden erfolgt mittels dem Fachmann bekannter Verfahren und wird nachfolgend anhand der Ausführungsbeispiele näher erläutert.

Weiterhin Gegenstand der Erfindung ist ein Verfahren zum Betrieb des erfindungsgemäßen memristiven Bauelements, dadurch gekennzeichnet, daß ein Schaltelement des elektronischen Bauelements in einen Zustand hohen elektrischen Widerstands geschaltet wird, indem eine korrespondierende erste Elektrode auf ein erstes elektrisches Potential und eine korrespondierende zweite Elektrode auf ein zweites elektrisches Potential gelegt wird, wobei der Betrag der Spannung zwischen den beiden Elektroden größer ist als eine erste Schaltspannung und das erste Potential größer als das zweite Potential ist, ein Schaltelement des elektronischen Bauelements in einen Zustand geringen elektrischen Widerstands geschaltet wird, indem eine korrespondierende erste Elektrode auf ein drittes elektrisches Potential und eine korrespondierende zweite Elektrode auf ein viertes elektrisches Potential gelegt wird, wobei der Betrag der Spannung zwischen den beiden Elektroden größer ist, als eine zweite Schaltspannung und das vierte Potential größer als das dritte Potential ist, und der Zustand eines Schaltelements bestimmt wird, indem zwischen korrespondierenden Elektroden eine Auslesespannung angelegt wird, deren Betrag kleiner als die erste und die zweite Schaltspannung ist und der fließende Strom gemessen wird.

Weiterhin Gegenstand der Erfindung ist die Verwendung von Verbindungen der Formel I als molekulare Schicht in Schaltelementen eines memristiven elektronischen Bauelements.

Weiterhin Gegenstand der Erfindung sind Verbindungen der Formeln Ia bis If, wie in Anspruch 19 angegeben, worin G -OP(O)(OH)₂, -P(O)(OH)₂, oder -C(OH)(P(O)(OH)₂)₂, bedeutet, und die übrigen Substituenten und Parameter die in Anspruch unter Formel I angegebenen Bedeutungen haben.

Weiterhin Gegenstand der Erfindung sind insbesondere Verbindungen der Formeln Ia bis If, wie in Anspruch 20 angegeben, worin
Sp -O(CF₂)ₚ₁- oder -(CF₂)ₚ₁- und
p1 eine ganze Zahl von 1 bis 12
bedeuten, und die übrigen Substituenten und Parameter die in Anspruch angegebenen Bedeutungen haben. Kurze Beschreibung der Figuren: Es zeigen
- Figur 1: eine erste Ausführungsform eines elektronischen Bauelements,
- Figur 2: eine zweite Ausführungsform eines elektronischen Bauelements,
- Figur 3: eine schematische Darstellung eines Versuchsaufbaus zur elektrischen Charakterisierung der Schaltelemente,
- Figur 4: ein Diagramm, welches eine zyklisch variierte Spannung darstellt,
- Figur 5: den aufgezeichneten Strom durch drei Proben mit jeweils einem aus der Verbindung CCY-5-O2V hergestellten Monolagensystem,
- Figur 6: den aufgezeichneten Strom durch eine Probe mit einem aus der Verbindung YY-4O-O2V hergestellten Monolagensystem mit permanenter zweiter Elektrode aus Pb/Ag,
- Figur 7: die angelegte Spannung und den gemessenen Widerstand als Funktion der Zeit für eine Probe mit einem aus der Verbindung YY-4O-O2V hergestellten Monolagensystem, (ein Messzyklus),
- Figur 8a: eine erfindungsgemäße Kreuzdrahtvorrichtung in der Draufsicht,
- Figur 8b: eine erfindungsgemäße Kreuzdrahtvorrichtung im Längsschnitt entlang einer zweiten Elektrode von der Seite
- Figur 9: eine schematische Darstellung der Kontaktierung eines erfindungsgemäßen Schaltelements,
- Figur 10a: den aufgezeichneten Strom durch eine Probe mit einem aus der Verbindung CCY-5-O3P hergestellten Monolagensystem mit permanenter zweiter Elektrode aus Pb
- Figur 10b: Ausschnittsvergrößerung aus der Figur 10a
- Figur 11: Widerstände in eingeschaltetem Zustand (ON) und ausgeschaltetem Zustand (OFF) eines erfindungsgemäßen Schaltelements für mehrere Schaltzyklen,
- Figur 12: Referenzmessung mit einer nicht schaltbaren, unpolaren Monolage,
- Figur 13: den aufgezeichneten Strom durch eine Probe mit einem aus der Verbindung CY-5-O11P hergestellten Monolagensystem auf Al₂O₃,
- Figur 14: Zustände hohen und niedrigen Widerstands einer Probe mit einem aus der Verbindung CY-5-O11P hergestellten Monolagensystem auf Al₂O₃,
- Figur 15: Referenzmessung mit einer nicht schaltbaren, nicht fluorierten, aus der Verbindung CP-5-O11P hergestellten Monolage,
- Figur 16: Zustände gleichbleibend hohen Widerstands einer Probe mit einem aus der Verbindung CP-5-O11P hergestellten Monolagensystem auf Al₂O_{3.}

Der Begriff "mesogene Gruppe " ist dem Fachmann bekannt und ist nach C. Tschierske, G. Pelzl, S. Diele, Angew. Chem. 2004, 116, 6340-6368 definiert als der Teil eines Moleküls oder Makromoleküls, der durch die Anisotropie seiner anziehenden und abstoßenden Wechselwirkungen wesentlich dazu beiträgt, daß niedermolekulare bzw. polymere Substanzen eine flüssigkristalline Mesophase bilden. Die Mehrzahl der mesogenen Gruppen besteht aus starren stab- oder scheibenförmigen Einheiten.

Ein mesogene Verbindung (kurz: "Mesogen") ist dadurch gekennzeichnet, daß sie eine oder mehrere mesogene Gruppen enthält. Dabei müssen die mesogenen Verbindungen nicht notwendigerweise selbst eine flüssigkristalline Phase aufweisen.

Die dielektrische Anisotropie Δε einer uniaxialen mesogenen Verbindung ist definiert als die Differenz der Dielektrizitätskonstanten parallel (ε_{∥}) und senkrecht (ε_{⊥}) zur Moleküllängsachse. Bei dielektrisch negativen Verbindungen gilt folglich Δε = (ε_{∥} ― ε_{⊥} ) < 0.

Eine Ankergruppe im Sinne der vorliegenden Erfindung ist eine funktionelle Gruppe, mittels derer die mesogene Verbindung durch Physisorption, Chemisorption oder durch chemische Reaktion an die Oberfläche des Substrats adsorbiert oder gebunden wird.

Eine Abstandsgruppe im Sinne der vorliegenden Erfindung ist eine flexible Kette zwischen mesogener Gruppe und Ankergruppe, die einen Abstand zwischen diesen Substrukturen des Moleküls bewirkt und dabei aufgrund ihrer Flexibilität die Beweglichkeit der mesogenen Gruppe nach Bindung an ein Substrat verbessert.

Sofern R¹ einen Alkylrest darstellt, ist dieser geradkettig oder verzweigt und hat 1 bis 15 C-Atome. Vorzugsweise ist R¹ geradkettig und hat, soweit nicht anders angegeben, 1, 2, 3, 4, 5, 6 oder 7 C-Atome und ist demnach vorzugsweise Methyl, Ethyl, Propyl, Butyl, Pentyl, Hexyl oder Heptyl.

Sofern R¹ einen Alkoxyrest darstellt, sind dieser geradkettig oder verzweigt und enthält 1 bis 15 C-Atome. Vorzugsweise ist R¹ geradkettig und hat, soweit nicht anders angegeben, 1, 2, 3, 4, 5, 6 oder 7 C-Atome und ist demnach vorzugsweise Methoxy, Ethoxy, Propoxy, Butoxy, Pentoxy, Hexoxy oder Heptoxy.

R¹ in Formel I kann ferner ein Alkenylrest mit 2 bis 15 C-Atomen sein, der geradkettig oder verzweigt ist und wenigstens eine C-C-Doppelbindung aufweist. Vorzugsweise ist er geradkettig und hat 2 bis 7 C-Atome. Er ist demnach vorzugsweise Vinyl, Prop-1- oder Prop-2-enyl, But-1-, 2- oder But-3-enyl, Pent-1-, 2-, 3- oder Pent-4-enyl, Hex-1-, 2-, 3-, 4- oder Hex-5-enyl, Hept-1-, 2-, 3-, 4-, 5- oder Hept-6-enyl. Sind die beiden C-Atome der C-C-Doppelbindung substituiert, kann der Alkenylrest als E- und/oder Z-Isomer (trans/cis) vorliegen. Im Allgemeinen sind die jeweiligen E-Isomere bevorzugt. Unter den Alkenylresten sind besonders bevorzugt Prop-2-enyl, 2- oder But-3-enyl, und 3- oder Pent-4-enyl.

R¹ in Formel I kann auch ein Alkinylrest mit 2 bis 15 C-Atomen sein, der geradkettig oder verzweigt ist und wenigstens eine C-C-Dreifachbindung aufweist. Bevorzugt ist 1- oder 2 Propinyl und 1-, 2- oder 3- Butinyl.

Bevorzugte Arylgruppen sind beispielsweise abgeleitet von den Grundkörpern Benzol, Naphthalin, Tetrahydronaphthalin, 9,10-Dihydrophenanthren, Fluoren, Inden, Indan.

Bevorzugte Heteroarylgruppen sind beispielsweise fünfgliedrige Ringe Furan, Thiophen, Selenophen, Oxazol, Isoxazol, 1,2-Thiazol, 1,3-Thiazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, sechsgliedrige Ringe wie beispielsweise Pyridin, Pyridazin, Pyrimidin, Pyrazin, 1,3,5-Triazin, 1,2,4-Triazin, 1,2,3-Triazin, oder kondensierte RInge wie beispielsweise Indol, Isoindol, Indolizin, Indazol, Benzimidazol, Benzotriazol, Purin, Naphthimidazol, Benzoxazol, Naphthoxazol, Benzothiazol, Benzofuran, Isobenzofuran, Dibenzofuran, Thieno[2,3b]-thiophen, Thieno[3,2b]thiophen, Dithienothiophen, Isobenzothiophen, Dibenzothiophen, Benzothiadiazothiophen, 2H-Chromen (2H-1-benzopyran), 4H-Chromen (4H-1-benzopyran), Cumarin (2H-chromen-2-on) oder Kombinationen dieser Gruppen.

Bevorzugte cycloaliphatische Gruppen sind Cyclobutan, Cyclopentan, Cyclohexan, Cyclohexen, Cycloheptan, Decahydronaphthalin, Bicyclo-[1.1.1]pentan, Bicyclo[2.2.2]octan, Spiro[3.3]heptan, Octahydro-4,7-methanoindan.

Bevorzugte heteroaliphatische Gruppen sind Tetrahydrofuran, Dioxolan, Tetrahydrothiofuran, Pyran, Dioxan, Dithian, Silinan, Piperidin, Pyrrolidin.

Besonders bevorzugt sind A¹ und A², unabhängig voneinander und bei jedem Auftreten gleich oder verschieden, ausgewählt aus den folgenden Gruppen:
a) 1,4-Phenylen worin auch eine oder zwei CH-Gruppen durch N ersetzt sein können und worin auch ein oder mehrere H-Atome durch Y ersetzt sein können,
b) der Gruppe bestehend aus trans-1,4-Cyclohexylen und 1,4-Cyclohexenylen, worin auch eine oder mehrere nicht benachbarte CH₂-Gruppen durch -O- und/oder -S- ersetzt sein können und worin auch ein oder mehrere H-Atome durch Y ersetzt sein können, und
c) der Gruppe bestehend aus 1,3-Dioxolan-2,4-diyl, Tetrahydrofuran-2,5-diyl, Cylcobutan-1,3-diyl, 1,4-Bicyclo[2,2,2]octandiyl, Piperidin-1,5-diyl, Thiophen-2,5-diyl, welche auch ein oder mehrfach durch Y ersetzt sein können,
wobei Y die oben unter Formel I angegebene Bedeutung hat und bevorzugt F, Cl, CN oder CF₃ bedeutet.

In Formel I bedeutet Sp bevorzugt eine Abstandsgruppe.

Bevorzugte Abstandsgruppen Sp sind ausgewählt aus der Formel Sp'-X', so daß der Rest G-Sp- der Formel G-Sp'-X'- entspricht, wobei
- Sp': geradkettiges oder verzweigtes Alkylen mit 1 bis 20, vorzugsweise 1 bis 12 C-Atomen bedeutet, welches optional durch F, Cl, Br, I oder CN ein- oder mehrfach substituiert ist, und worin auch eine oder mehrere nicht benachbarte CH₂-Gruppen jeweils unabhängig voneinander so durch -O-, -S-, -NH-, -NR⁰-, -SiR⁰⁰R⁰⁰⁰-, -CO-, -COO-, -OCO-, -OCO-O-, -S-CO-, -CO-S-,-NR⁰⁰-CO-O-, -O-CO-NR⁰⁰-, -NR⁰⁰-CO-NR⁰⁰-, -CH=CH- oder -C≡C- ersetzt sein können, daß O- und/oder S-Atome nicht direkt miteinander verknüpft sind,
- X': -O-, -S-, -CO-, -COO-, -OCO-, -O-COO-, -CO-NR⁰⁰-, -NR⁰⁰-CO-, -NR⁰⁰-CO-NR⁰⁰-, -OCH₂-, -CH₂O-, -SCH₂-, -CH₂S-, -CF₂O-, -OCF₂-, -CF₂S-, -SCF₂-, -CF₂CH₂-, -CH₂CF₂-, -CF₂CF₂-, -CH=N-, -N=CH-, -N=N-, -CH=CR⁰⁰-, -CY^{x}=CY^{x'}-, -C≡C-, -CH=CH-COO-, -OCO-CH=CH- oder eine Einfachbindung bedeutet,
- R⁰⁰ und R⁰⁰⁰: jeweils unabhängig voneinander H oder Alkyl mit 1 bis 12 C-Atomen bedeuten, und
- Y^{x} und Y^{x'}: jeweils unabhängig voneinander H, F, Cl oder CN bedeuten.

X' ist vorzugsweise -O-, -S -CO-, -COO-, -OCO-, -O-COO-, -CO-NR⁰-, -NR⁰-CO-, -NR⁰-CO-NR⁰- oder eine Einfachbindung.

Typische Abstandsgruppen Sp' sind beispielsweise -(CH₂)ₚ₁-, -(CF₂)ₚ₁-, -(CH₂CH₂O)_{q1}-CH₂CH₂-, -(CF₂CF₂O)_{q1}-CF₂CF₂-, -CH₂CH₂-S-CH₂CH₂-,-CH₂CH₂-NH-CH₂CH₂- oder -(SiR⁰⁰R⁰⁰⁰-O)ₚ₁₋, worin p1 eine ganze Zahl von 1 bis 12 ist, q1 eine ganze Zahl von 1 bis 3 ist, und R⁰⁰ und R⁰⁰⁰ die oben angegebenen Bedeutungen besitzen.

Besonders bevorzugte Gruppen -X'-Sp'- sind -(CH₂)ₚ₁-, -O-(CH₂)ₚ₁-, -(CF₂)ₚ₁-, -O(CF₂)ₚ₁-, -OCO-(CH₂)ₚ₁-, -OC(O)O-(CH₂)ₚ₁-, worin p1 die oben angegebene Bedeutung hat

Besonders bevorzugte Gruppen Sp' sind beispielsweise jeweils geradkettiges Ethylen, Propylen, Butylen, Pentylen, Hexylen, Heptylen, Octylen, Nonylen, Decylen, Undecylen, Dodecylen, Octadecylen, Perfluorethylen, Perfluorpropylen, Perfluorbutylen, Perfluorpentylen, Perfluorhexylen, Perfluorheptylen, Perfluoroctylen, Perfluornonylen, Perfluordecylen, Perfluorundecylen, Perfluordodecylen, Perfluoroctadecylen, Ethylenoxyethylen, Methylenoxybutylen, Ethylenthioethylen, Ethylen-N-methyl-iminoethylen, 1-Methylalkylen, Ethenylen, Propenylen und Butenylen.

Besonders bevorzugte Unterformeln der Formel I sind die nachfolgend aufgeführten Unterformeln Ia bis If:

| | |
|---|---|
| R¹-B¹-Sp-G | Ia |
| R¹-(A¹-Z¹)-B¹-Sp-G | Ib |
| R¹-(A¹-Z¹)₂-B¹-Sp-G | Ic |
| R¹-B¹-( -Z²-A²)-Sp-G | Id |
| R¹-B¹-( Z²-A²)₂-Sp-G | Ie |
| R¹-(A¹-Z¹)-B¹-( Z²-A²-)-Sp-G | If |

worin R¹, A¹, A², B¹, Z¹, Z², Sp und G die oben abgegebenen Bedeutungen haben und bevorzugt
- A¹ und A²: 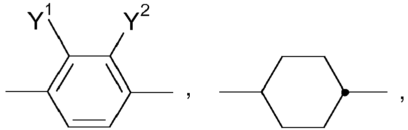
- B¹: 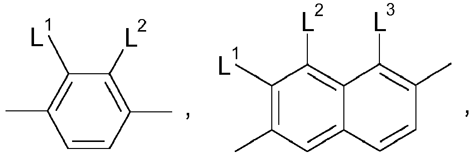 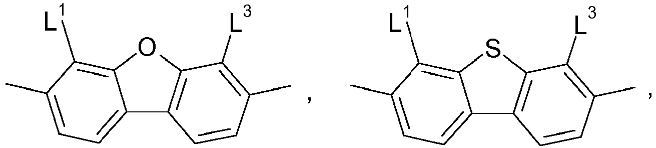 wobei die Gruppen in beide Richtungen orientiert sein können,
- R¹: Alkyl mit 1-15 C-Atomen, bevorzugt mit 1-7 C-Atomen, insbesondere CH₃, C₂H₅, n-C₃H₇, n-C₄H₉, n-C₅H₁₁, n-C₆H₁₃, n-C₇H₁₅.
- L¹ und L²: unabhängig voneinander Cl oder F, wobei mindestens einer der Reste L¹ und L² F bedeutet,
- L³: F,
- Y¹ und Y²: unabhängig voneinander H, Cl oder F,
- Z¹, Z²: unabhängig voneinander eine Einfachbindung, -CF₂O-, -OCF₂-, -CH₂O-, OCH₂-, -CH₂CH₂-,
- Sp: unverzweigtes 1,ω-Alkylen mit 1 bis 12 C-Atomen,
- G: -CH=CH₂, -OH, -SH, -SO₂OH, -OP(O)(OH)₂, -PO(OH)₂, -COH(PO(OH)₂)₂, -COOH, -Si(OR)₃, -SiCl₃,
bedeuten.

In einer weiteren bevorzugten Ausführungsform bedeutet in den Verbindungen der Formeln Ia bis If
- Sp: unverzweigtes 1,ω-Perfluoralkylen mit 1 bis 12 C-Atomen,
wobei R¹, A¹, A², B¹, Z¹, Z² und G die oben angegebenen Bedeutungen haben.

Ganz besonders bevorzugte Unterformeln der Formel I sind die Unterformeln Ia, Ib und Id.

Beispiele für bevorzugte Verbindungen der Formeln Ia bis If sind nachfolgend aufgeführt: 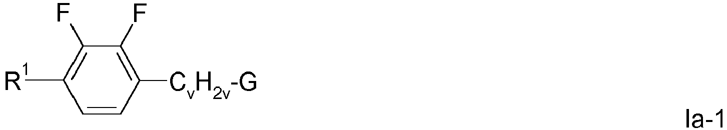 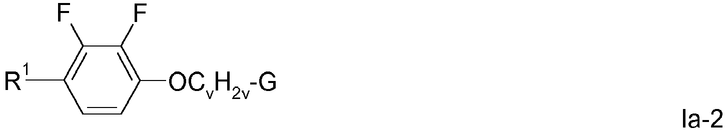 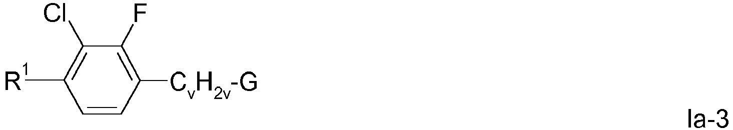 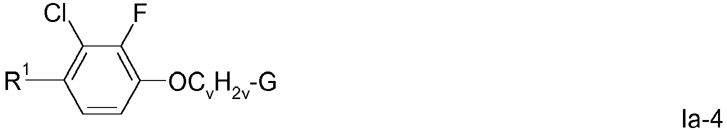 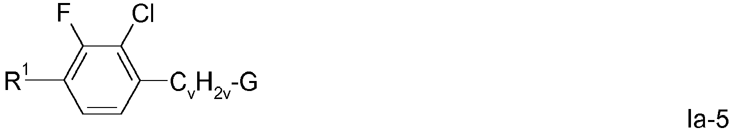 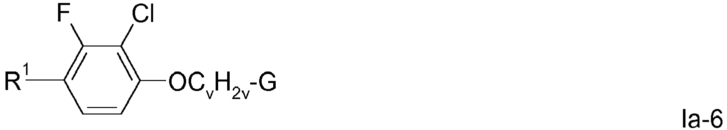 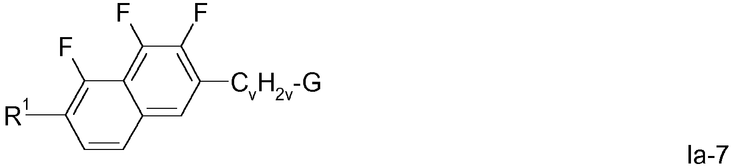 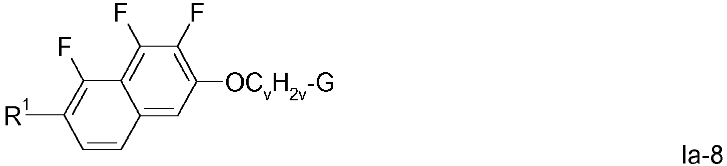 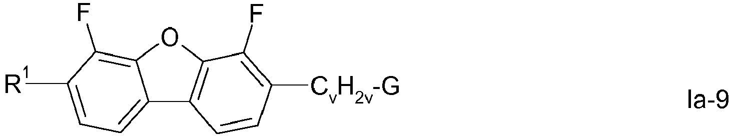 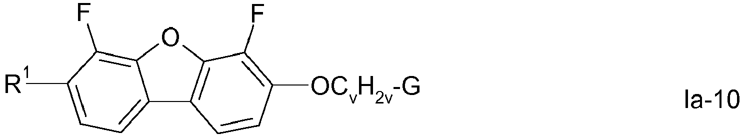 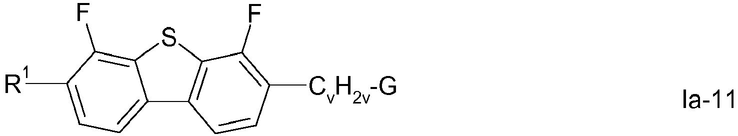 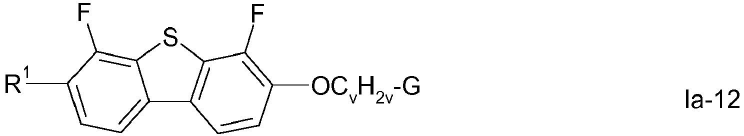 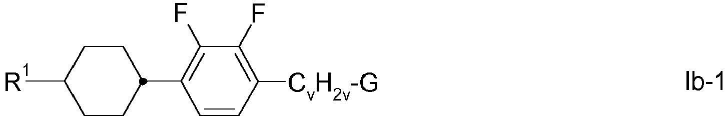 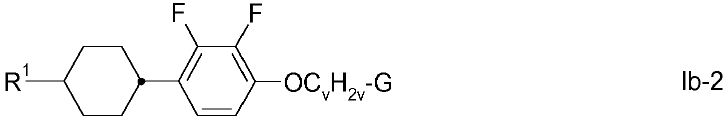 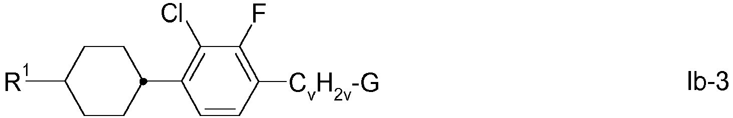 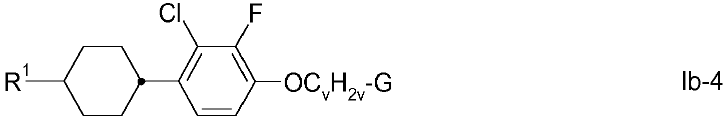 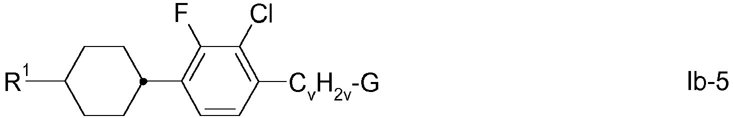 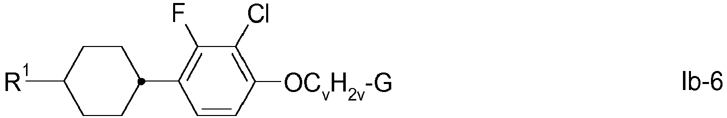 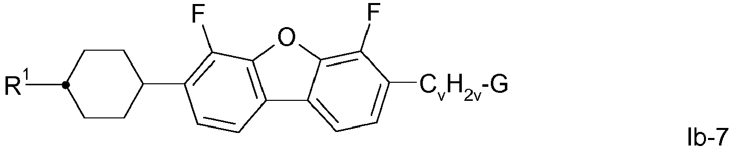 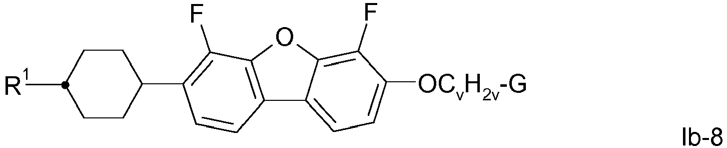 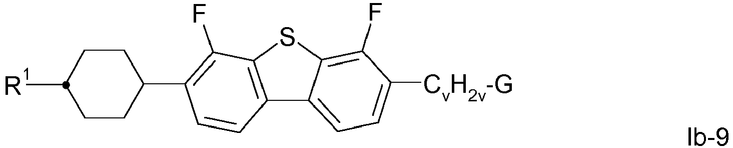 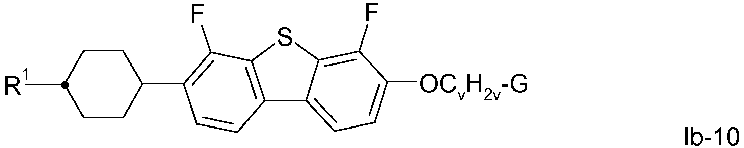 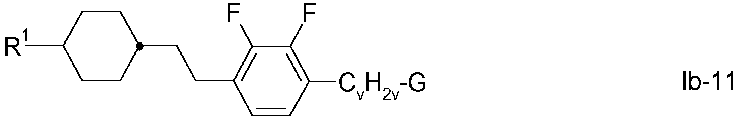 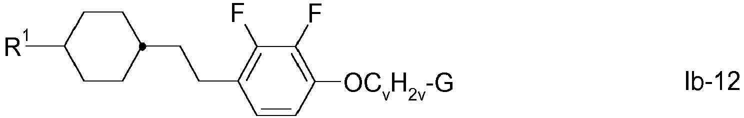 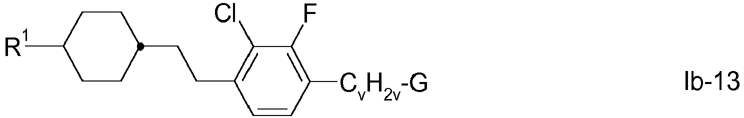 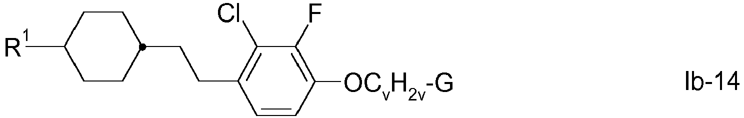 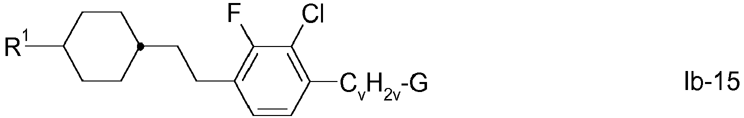 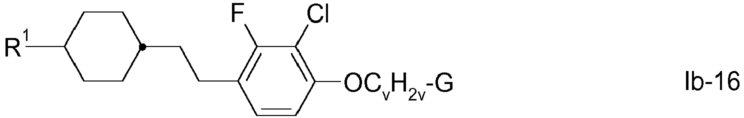 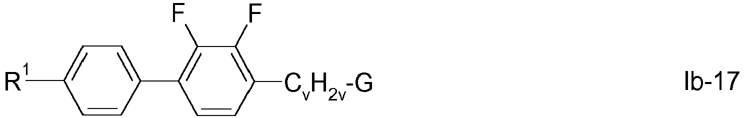 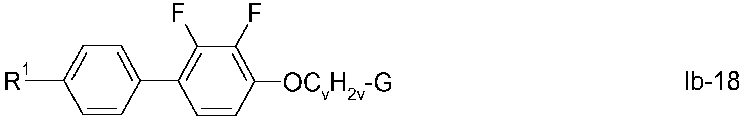 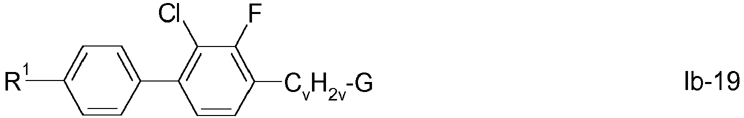 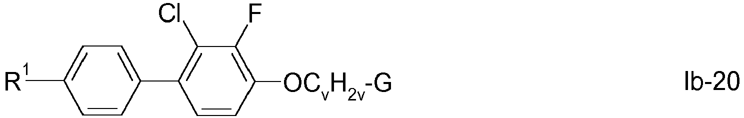 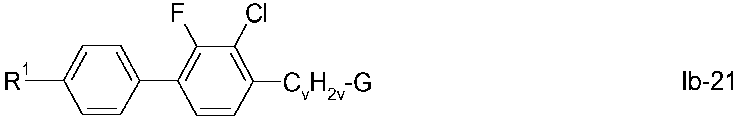 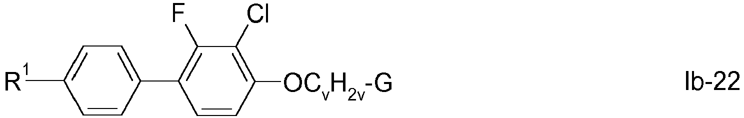 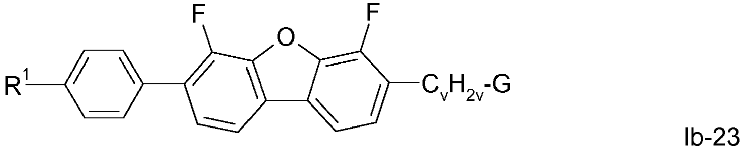 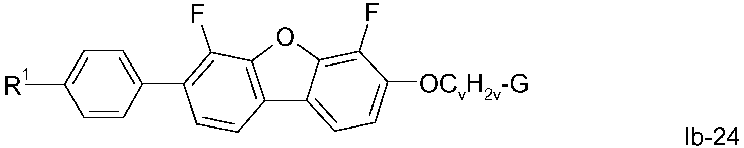 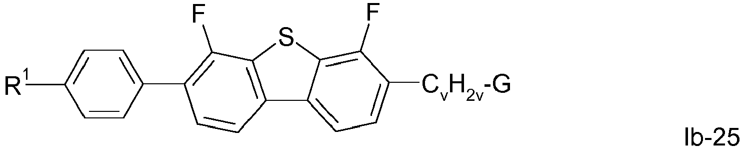 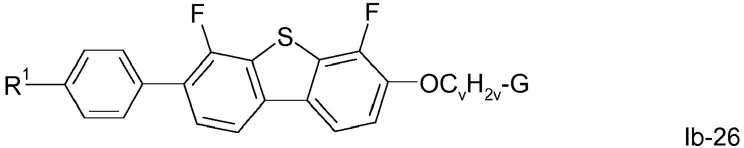 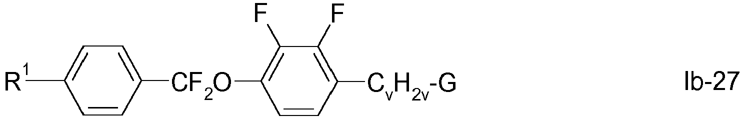 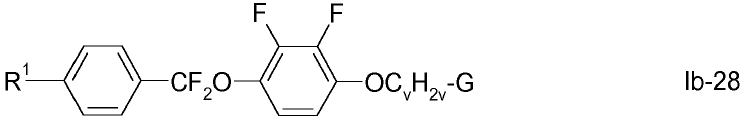 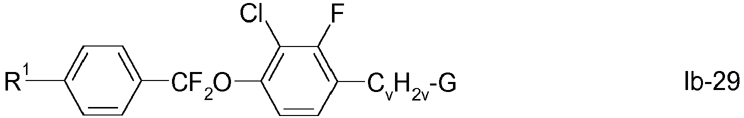 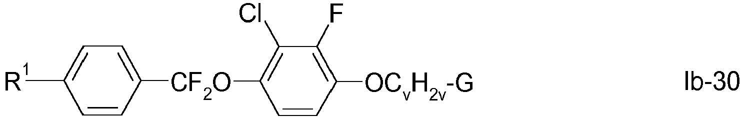 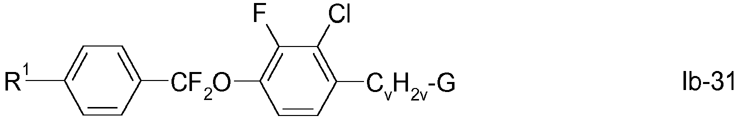 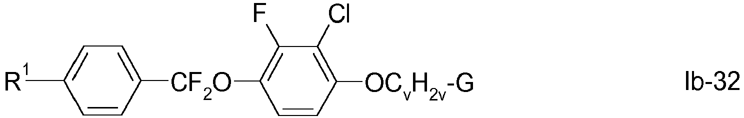 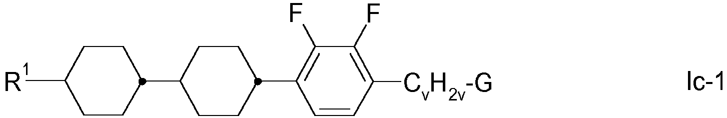 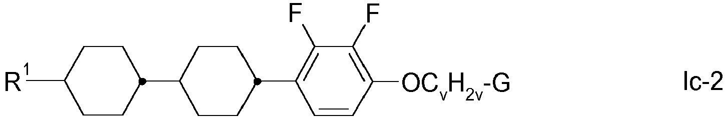 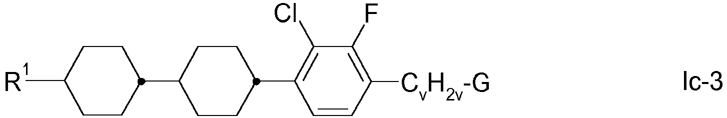 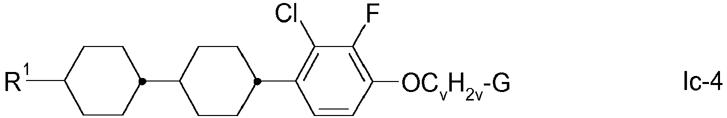 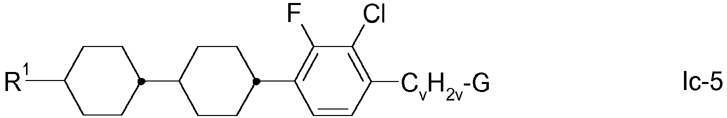 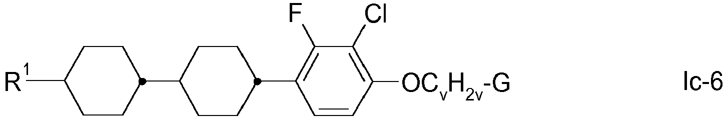 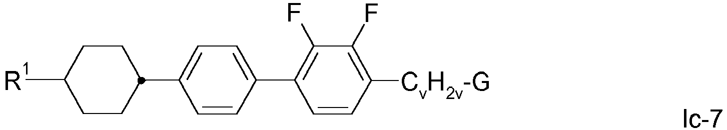 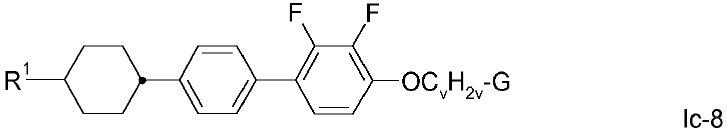 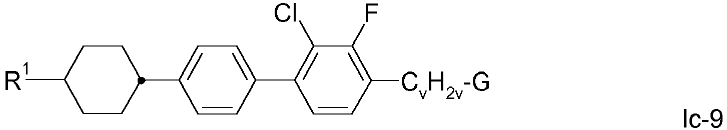 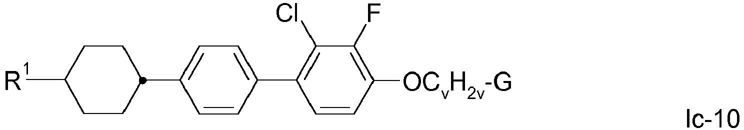 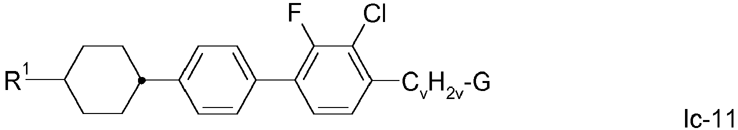 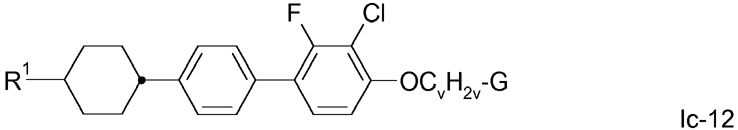 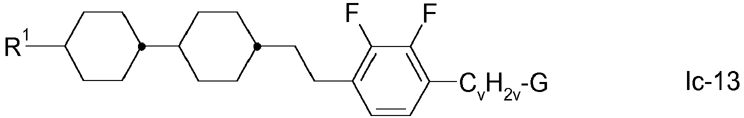 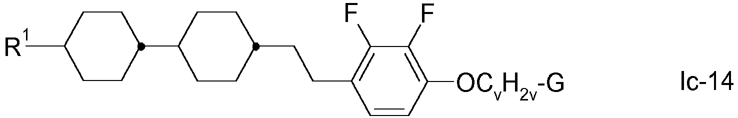 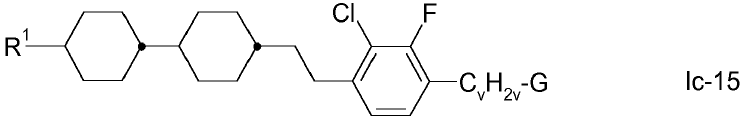 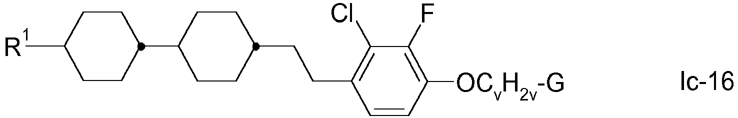 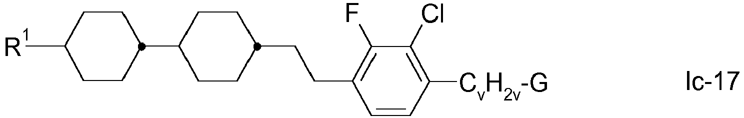 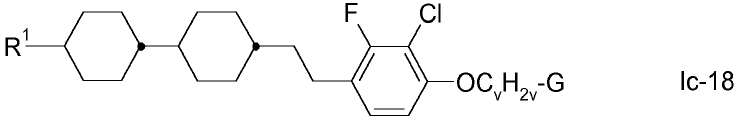 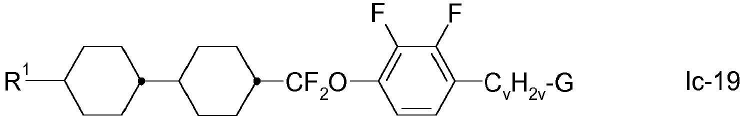 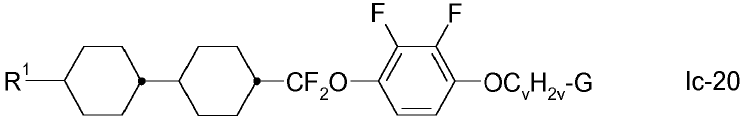 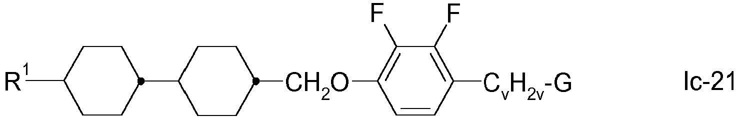 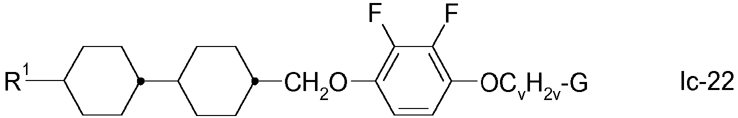 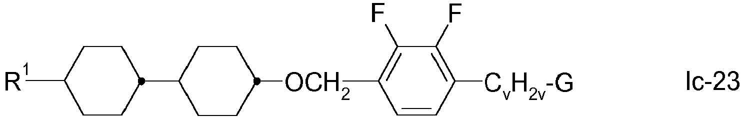 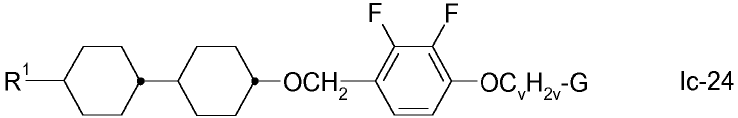 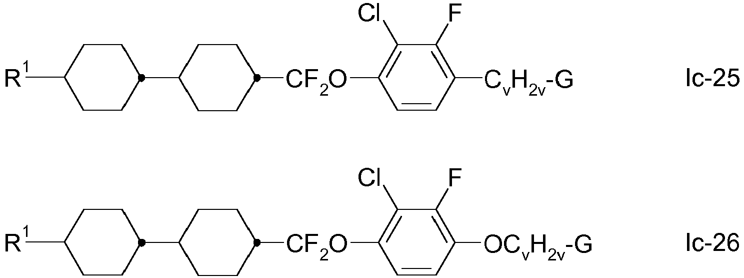 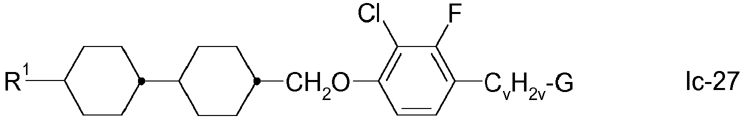 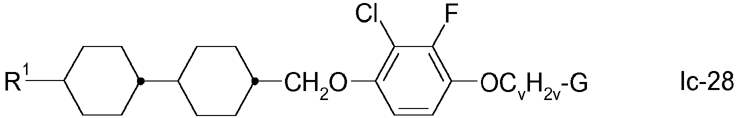 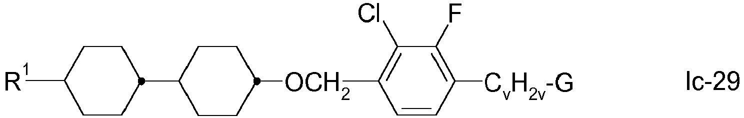 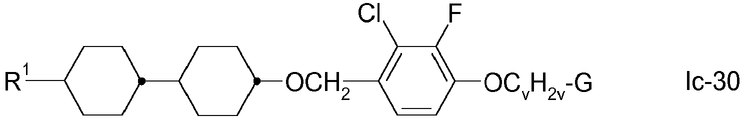 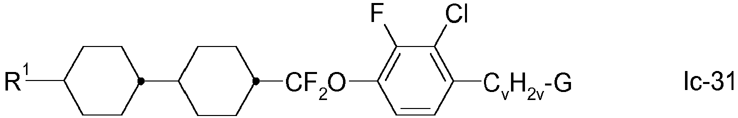 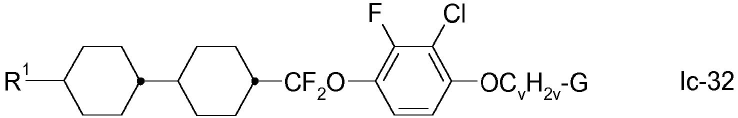 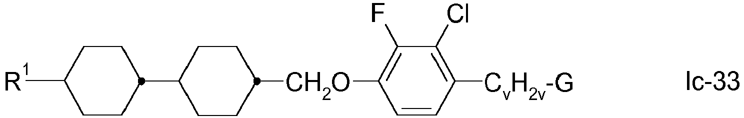 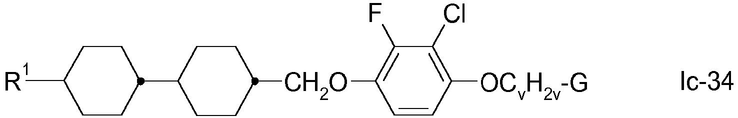 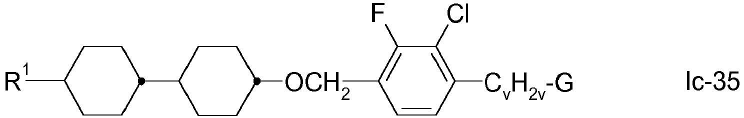 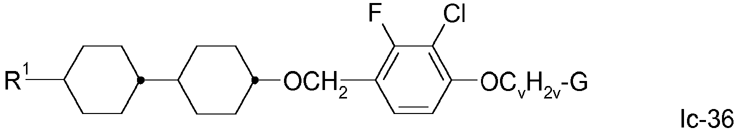 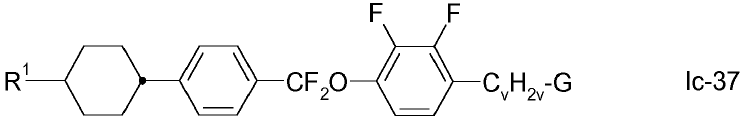 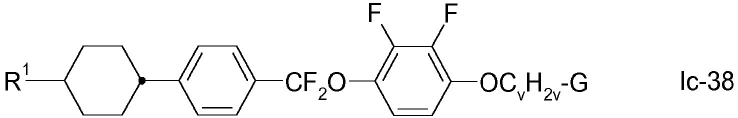 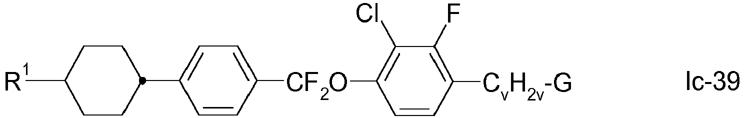 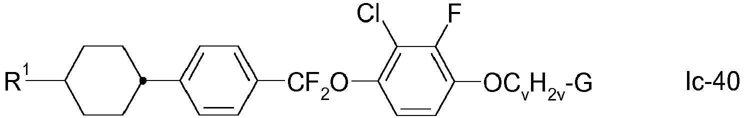 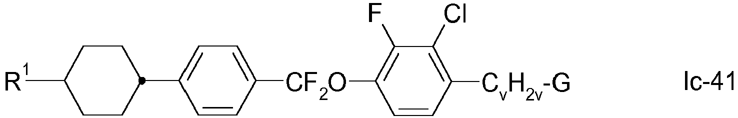 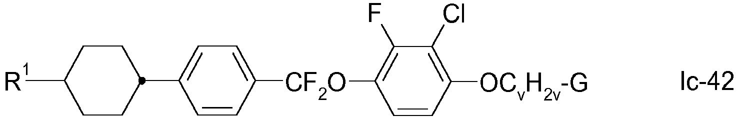 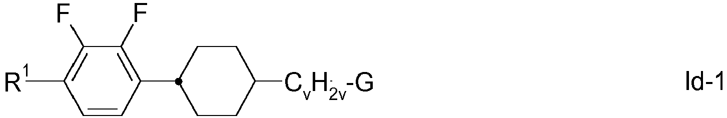 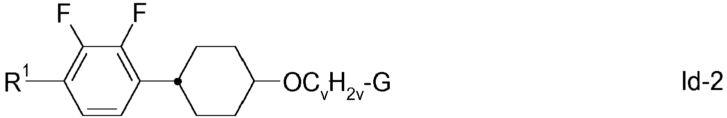 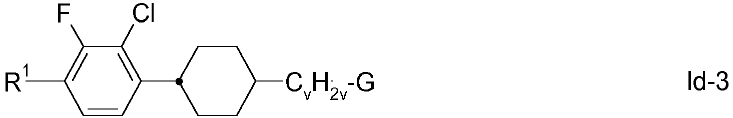 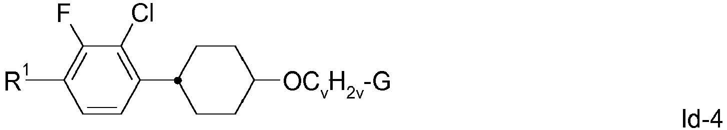 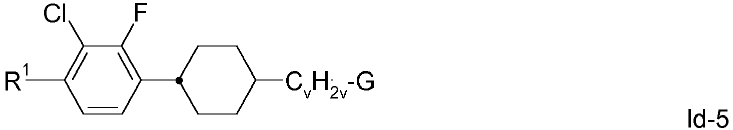 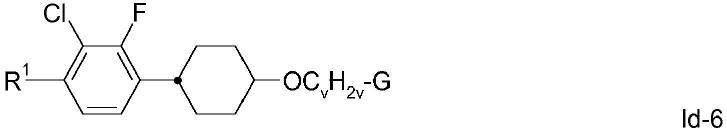 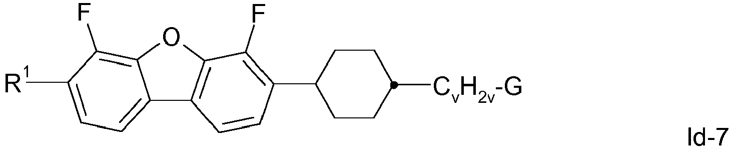 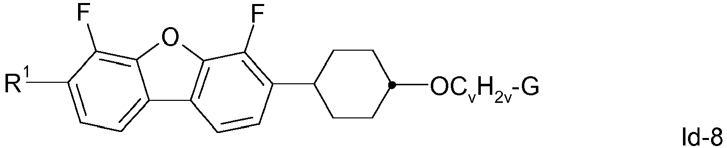 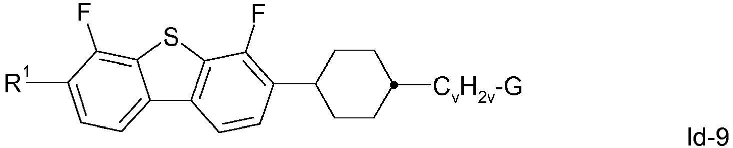 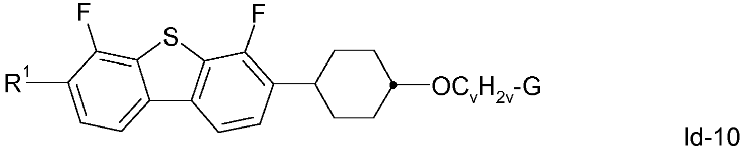 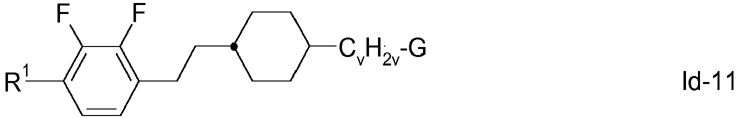 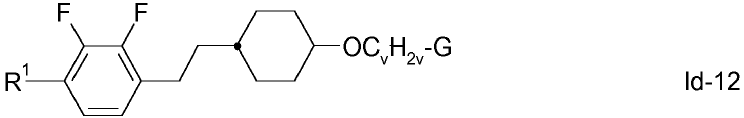 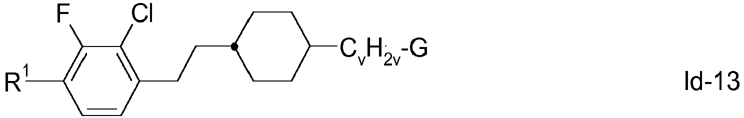 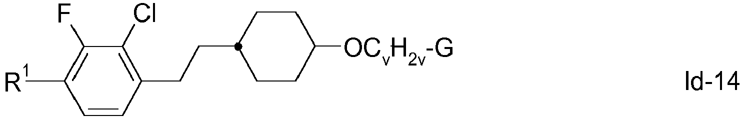 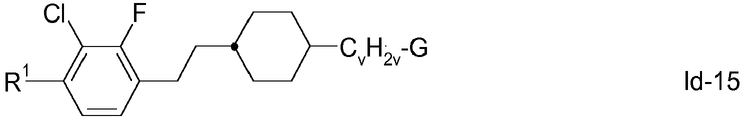 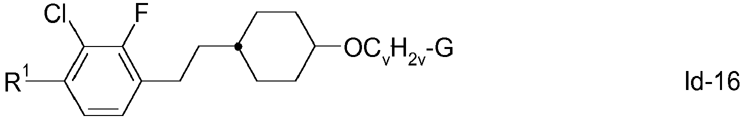 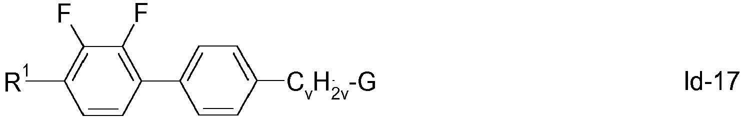 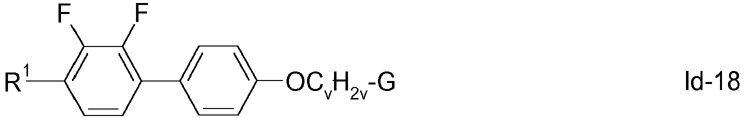 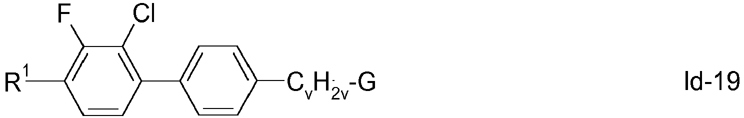 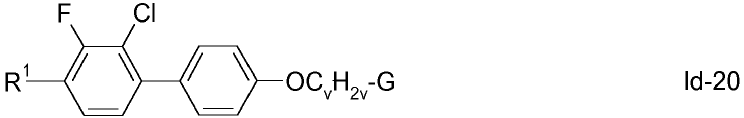 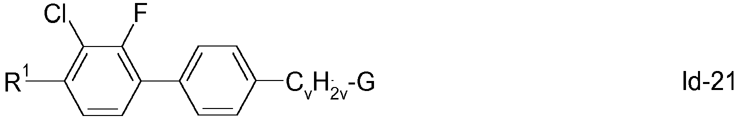 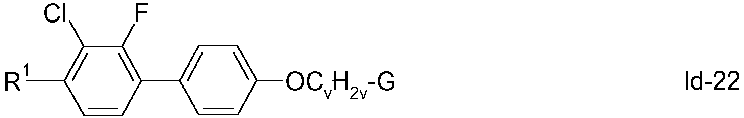 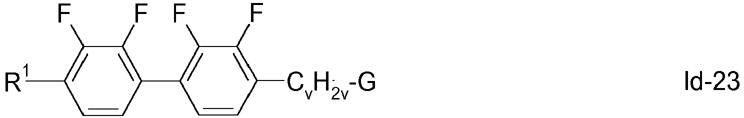 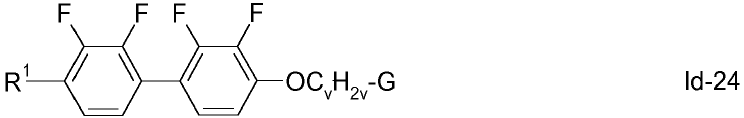 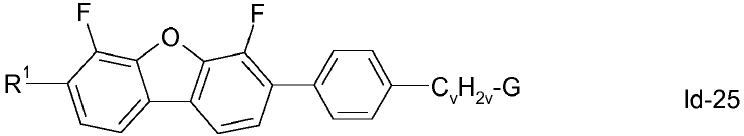 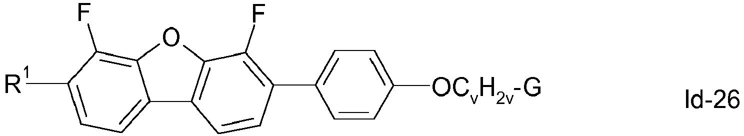 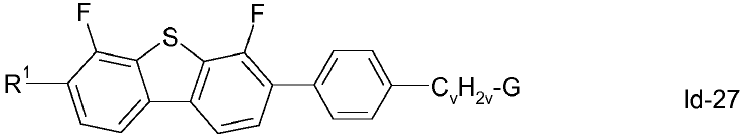 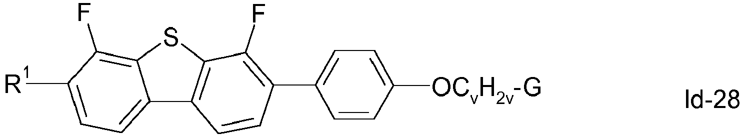 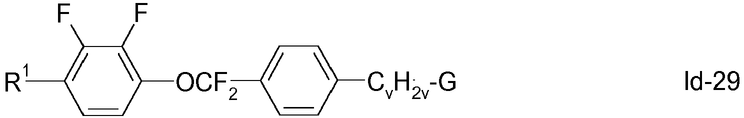 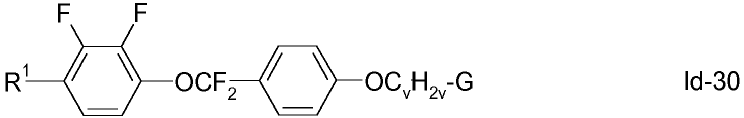 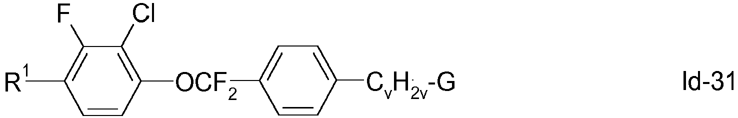 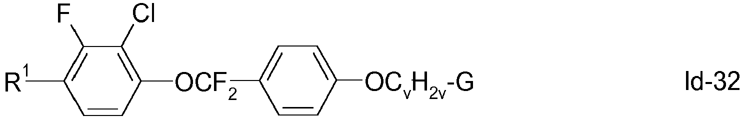 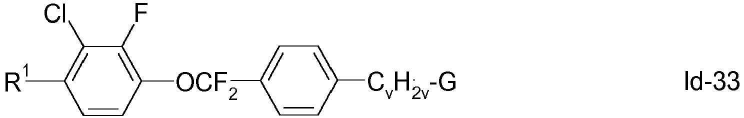 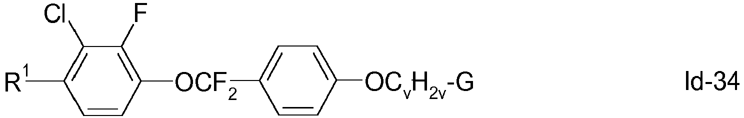 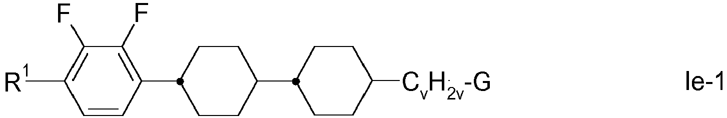 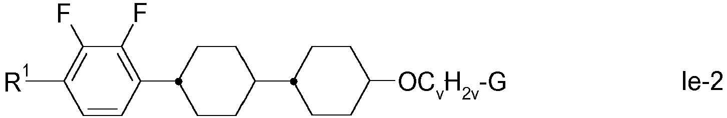 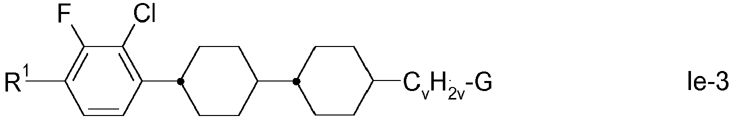 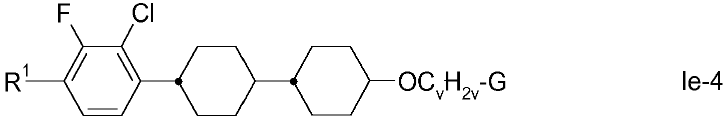 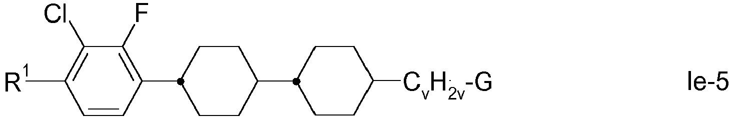 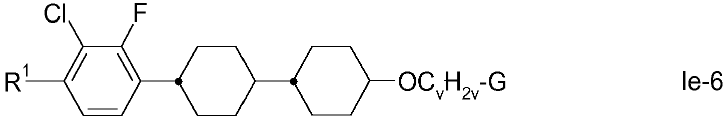 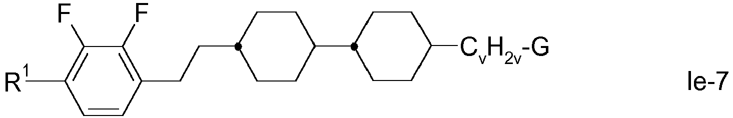 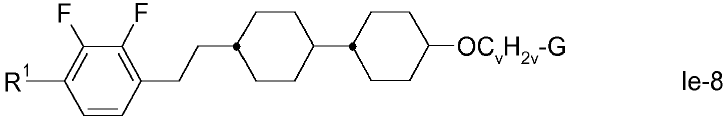 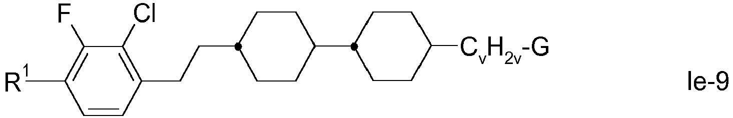 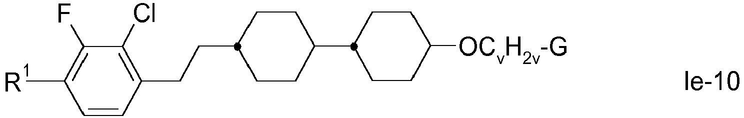 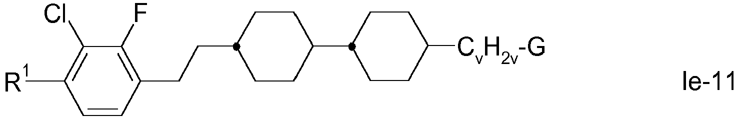 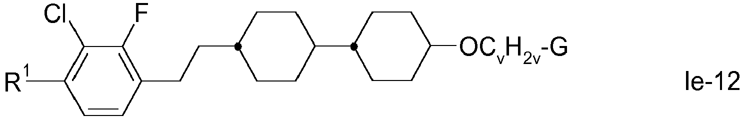 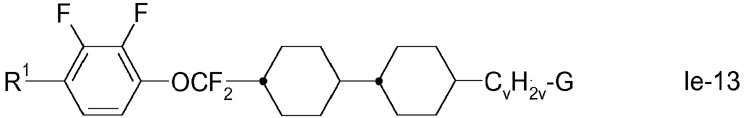 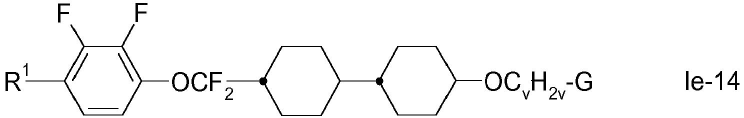 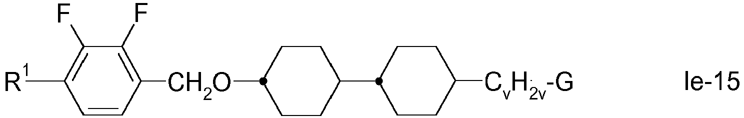 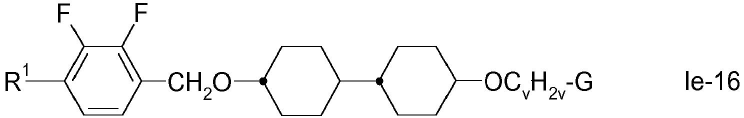 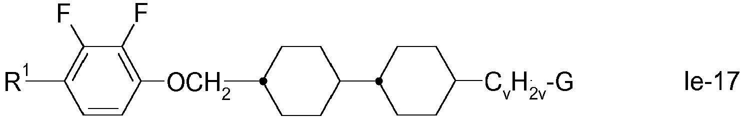 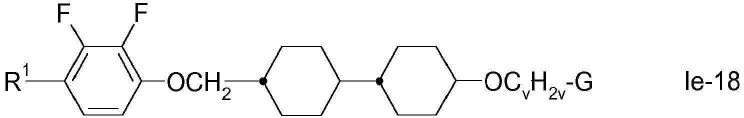 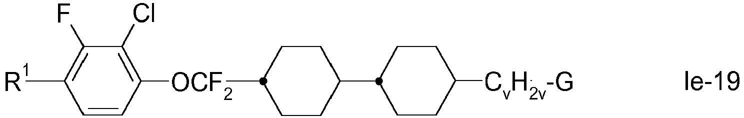 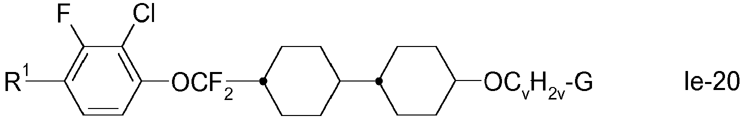 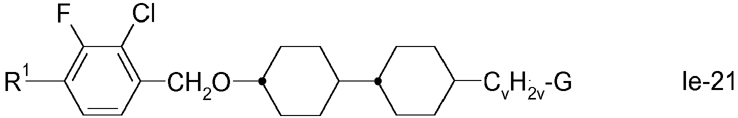 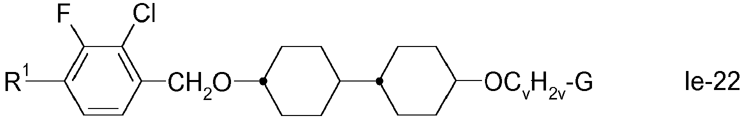 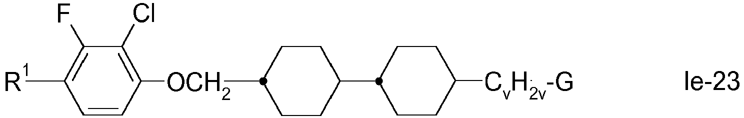 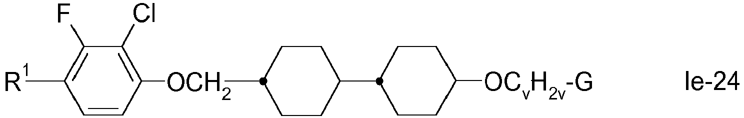 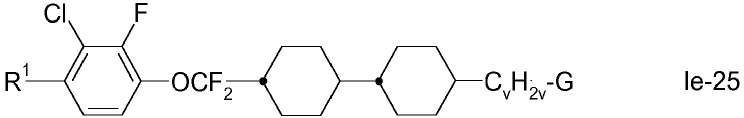 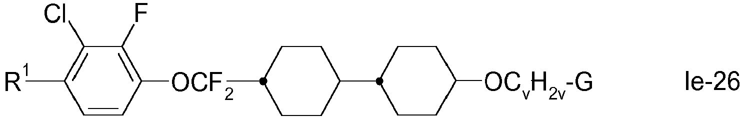 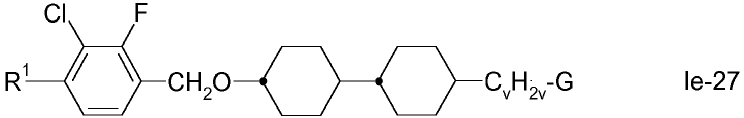 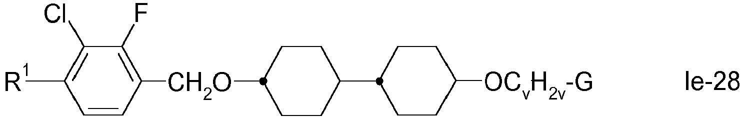 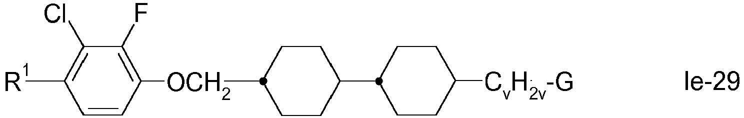 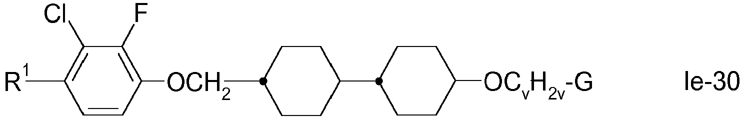 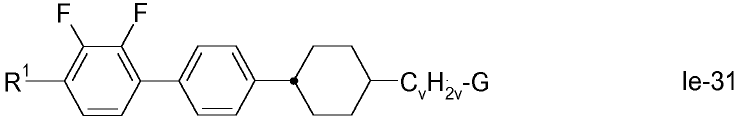 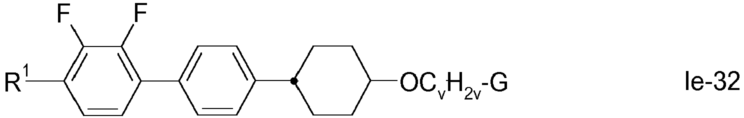 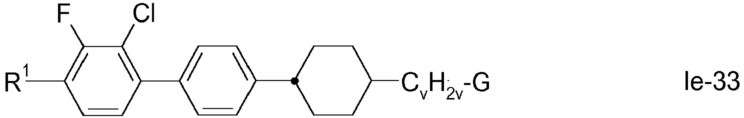 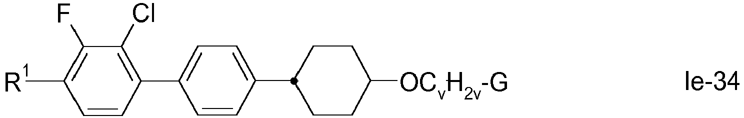 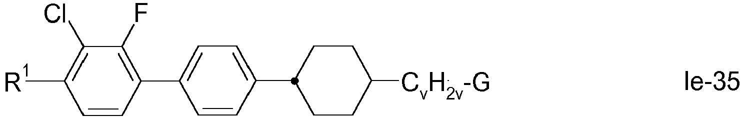 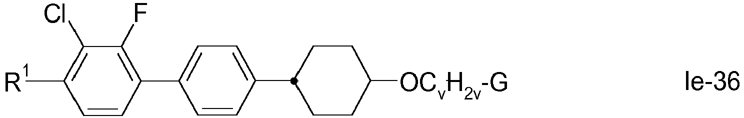 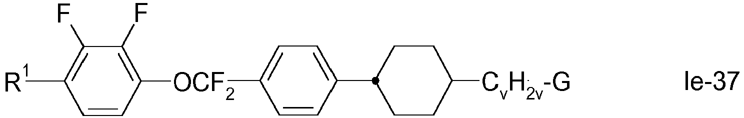 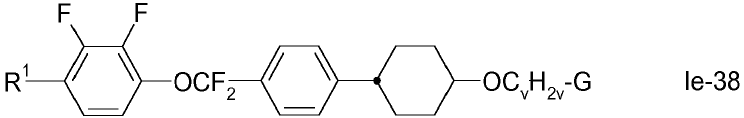 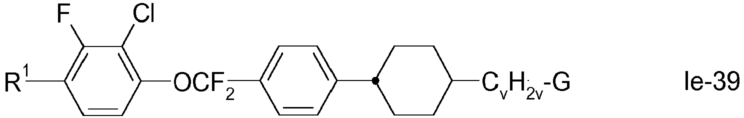 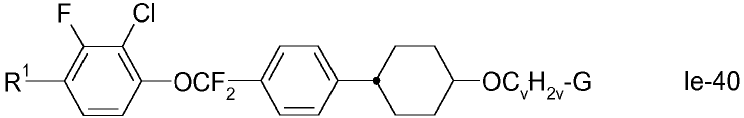 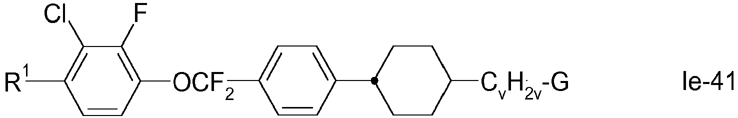 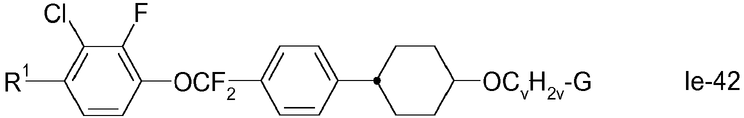 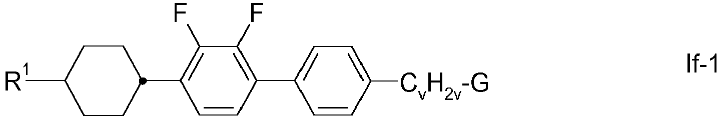 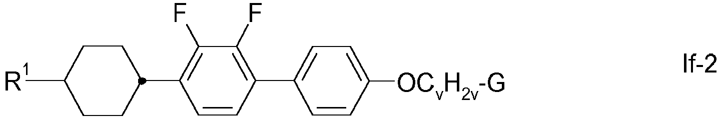 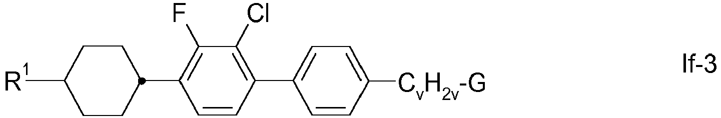 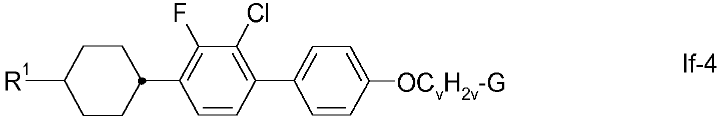 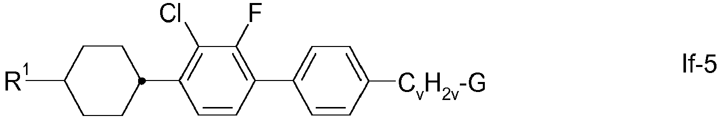 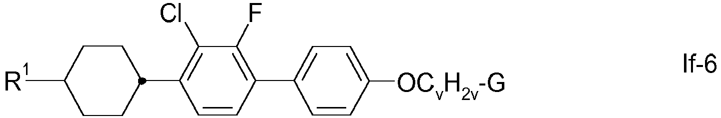 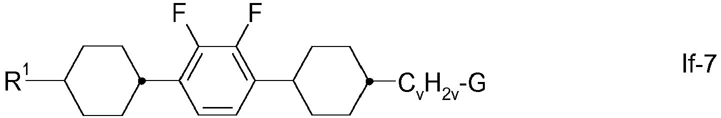 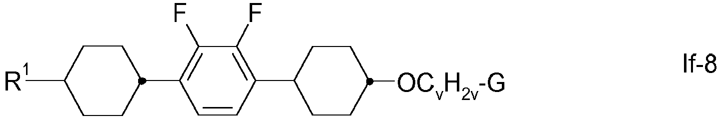 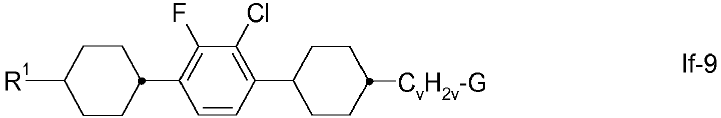 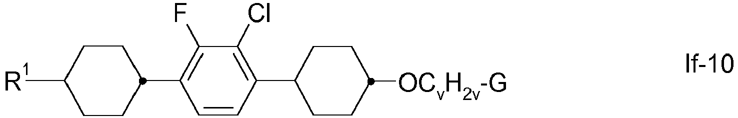 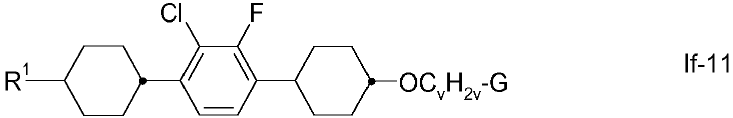 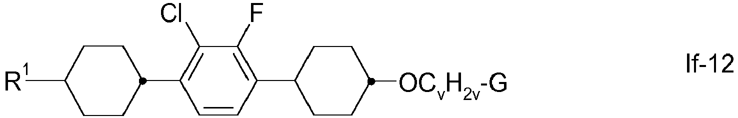 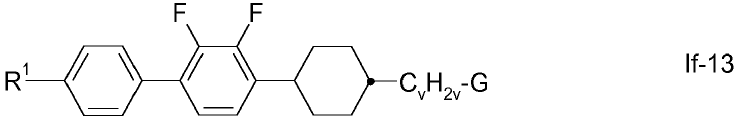 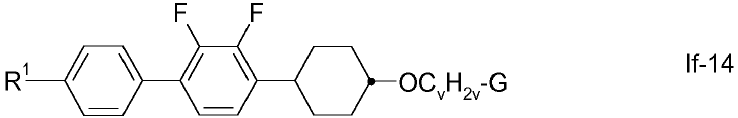 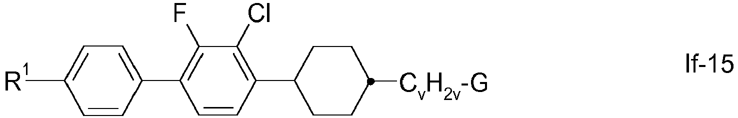 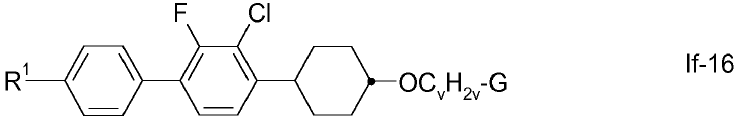 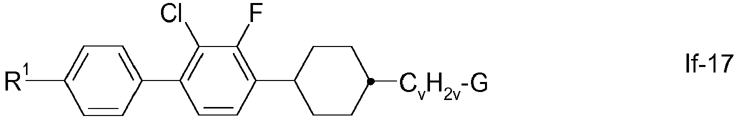 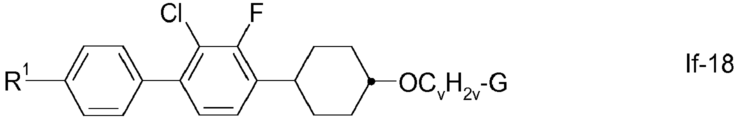 worin R¹ und G die oben genannten Bedeutungen haben und bevorzugt
- R¹: Alkyl mit 1 bis 7 C-Atomen und
- G: -CH=CH₂, -P(O)(OH)₂ oder -COH(P(O)(OH)₂)₂,
bedeuten, und
- v: eine ganze Zahl von 1 bis 12, bevorzugt von 2 bis 7 bedeutet.

Von der Beschreibung umfaßt sind auch solche Verbindungen der Formel I, bei denen in den Unterformeln Ia-1 bis Ia-12, Ib-1 bis Ib-32, Ic-1 bis Ic-42, Id-1 bis Id-34, Ie-1 bis Ie-42 und If-1 bis If-18 die Gruppe -CᵥH₂ᵥ-durch -CᵥF₂ᵥ- ersetzt ist.

Erfindungsgemäß in dem elektronischen Bauelement eingesetzte Schaltelemente umfassen eine molekulare Schicht, enthaltend eine oder mehrere Verbindungen der Formel I.

Die molekulare Schicht der vorliegenden Erfindung ist eine Schicht elektrisch isolierender, nicht leitender und nicht halbleitender organischer Verbindungen.

Bevorzugt enthält die molekulare Schicht Moleküle der Formel I oder, besonders bevorzugt, besteht sie aus Molekülen der Formel I.

Bevorzugt beträgt die Dicke der Schicht 10 nm oder weniger, besonders bevorzugt 5 nm oder weniger, ganz besonders bevorzugt 2 nm oder weniger.

Die molekulare Schicht kann aus einer, zwei, drei oder mehr Moleküllagen enthaltend Verbindungen der Formel I bestehen.

Die erfindungsgemäß eingesetzte molekulare Schicht ist bevorzugt eine molekulare Monoschicht.

In einer Ausführungsform handelt es sich dabei um eine selbstorganisierte Monoschicht (SAM, self assembled monolayer).

Die Herstellung von selbstorganisierten Monoschichten ist dem Fachmann bekannt; ein Überblick ist beispielsweise in A. Ulman, Chem. Rev. 1996, 96, 1533-1554 gegeben.

In einer weiteren Ausführungsform wird die molekulare Schicht durch Chemisorption, insbesondere durch eine Additionsreaktion oder Kondensationsreaktion an das Substrat gebunden.

In einer weiteren Ausführungsform ist die molekulare Schicht durch Physisorption an das Substrat gebunden.

Der Bedeckungsgrad des Substrats ist vorzugsweise 90% oder mehr bis 100%, besonders bevorzugt 95% oder mehr bis 100%, ganz besonders bevorzugt 98% oder mehr bis 100%.

In einer weiteren Ausführungsform ist die molekulare Schicht mit 1 bis 10, bevorzugt 1 bis 5, besonders bevorzugt 1 bis 3 weiteren Lagen an organischen oder anorganischen Adsorbaten bedeckt. Geeignet sind beispielsweise Schichten aus Dielektrika, zum Beispiel oxidische, fluoridische oder nitridische Materialien wie TiO₂, Al₂O₃,HfO₂, SiO₂, LiF und Si₃N₄, oder Metallen wie Pt, Pd, Pb, Au, Ag, Cu, Al und Mg sowie deren eutektische Verbindungen wie z.B. PdAu 20:80. Solche Schichten können durch definierte und hochpräzise Abscheidung, beispielsweise durch ALD-(atomic layer deposition) Verfahren, in einer Dicke von wenigen Nanometern aufgebaut werden.

Bevorzugt sind die Moleküle der molekularen Schicht kovalent an das Substrat gebunden. Die Anbindung erfolgt nach bekannten, dem Fachmann geläufigen Methoden, beispielsweise durch Addition einer Verbindung der Formel I oder durch Veresterung mit an der Oberfläche des Substrats befindlichen Hydroxylgruppen.

Für Additionsreaktionen kann beispielsweise ein geeignetes Substrat, bevorzugt eine Siliziumoberfläche - nach entsprechender Vorbehandlung mit wäßriger NH₄F-Lösung - behandelt werden, um eine wasserstoffterminierte Oberfläche zu erhalten. Die so behandelte Oberfläche kann dann bei erhöhter Temperatur unter Sauerstoffausschluß entweder direkt mit einer geeigneten, flüssigen Verbindung der Formel I oder einer Lösung der Verbindung der Formel I in einem geeigneten Lösungsmittel hergestellt werden.

Für Kondensationsreaktionen kann beispielsweise ein geeignetes Substrat, bevorzugt eine Siliziumoberfläche, mit Sauerstoffplasma behandelt werden, um eine hydrophile, mit Hydroxylgruppen bedeckte oxidische Oberfläche zu erhalten. Die so behandelte Oberfläche kann dann bei erhöhter Temperatur entweder direkt mit einer geeigneten, flüssigen Verbindung der Formel I oder einer Lösung der Verbindung der Formel I in einem geeigneten Lösungsmittel hergestellt werden. Es ist klar, daß eine solche oxidische Oberfläche lediglich der Oberflächenmodifizierung dient mit dem Ziel einer möglichen Derivatisierung über Kondensationsreaktionen und nicht eine Isolatorschicht (14) im eigentlichen Sinne darstellt. Ausreichend große Tunnelströme durch diese oxidische Oberfläche hindurch sind aufgrund der geringen Dicke in der Größenordnung von 1 nm möglich.

In den erfindungsgemäßen Schaltelementen sind die Moleküle der molekularen Schicht an ein Substrat oder eine zwischen molekularer Monoschicht und Substrat befindliche Zwischenschicht gebunden. Das erfindungsgemäße Substrat kann je nach Aufbau der Schaltelemente unterschiedliche Funktionen wahrnehmen. Beispielsweise kann ein leitfähiges Substrat als erste Elektrode dienen. Ebenso kann das Substrat eine Schicht einer Diode darstellen.

Geeignete Substrate sind dem Fachmann bekannt. Besonders geeignete Substrate sind ausgewählt aus:
- Elementhalbleiter, insbesondere Si, Ge, C (Diamant, Graphit, Graphen, Fulleren), α-Sn, B, Se und Te;
- Verbindungshalbleiter, bevorzugt
   - Gruppen III-V-Halbleiter, insbesondere GaAs, GaP, InP, InSb, InAs, GaSb, GaN, TaN, TiN, MoN, WN, AlN, InN, AlₓGa₁₋ₓAs und InₓGa₁₋ₓNi,
   - Gruppen II-VI-Halbleiter, insbesondere ZnO, ZnS, ZnSe, ZnTe, CdS, CdSe, CdTe, Hg₍₁₋ₓ₎Cd₍ₓ₎Te, BeSe, BeTeₓ und HgS;
   - Gruppen III-VI-Halbleiter, insbesondere GaS, GaSe, GaTe, InS, InSeₓ und InTe,
      Gruppen I-III-VI-Halbleiter, insbesondere CuInSe₂, CuInGaSe₂, CuInS₂ und CuInGaS₂;
   - Gruppen IV - IV-Halbleiter, insbesondere SiC und SiGe,
   - Gruppen IV-VI-Halbleiter, insbesondere SeTe;
- organische Halbleiter, insbesondere Polythiophen, Tetracen, Pentacen, Phthalocyanine, PTCDA, MePTCDI, Chinacridon, Acridon, Indanthron, Flaranthron, Perinon, AlQ₃- und Mischsysteme wie PEDOT/PSS und Polyvinylcarbazol/TLNQ-Komplexe;
- Metalle, insbesondere Ta, Ti, Co, Mo, Pt, Ru, Au, Ag, Cu, Al, W und Mg;
- leitfähige oxidische Materialien, insbesondere Indiumzinnoxid (ITO), Indiumgalliumoxid (IGO), InGa-α-ZnO (IGZO), aluminiumdotiertes Zinkoxid (AZO), zinndotiertes Zinkoxid (TZO), fluordotiertes Zinnoxid (FTO) und Antimonzinnoxid.

Die molekulare Schicht kann optional auch an eine dünne (bevorzugt 0.5 - 5 nm dick) oxidische oder fluoridische Zwischenschicht, z.B. TiO₂, Al₂O₃, HfO₂, SiO₂ oder LiF gebunden sein, die sich auf dem Substrat befindet.

Die Gegenelektrode bzw. zweite Elektrode besteht aus einem leitenden oder halbleitenden Material oder einer Kombination (Schichtstapel) mehrerer dieser Materialien. Beispiele sind die als Substratmaterial aufgeführten Materialien. Bevorzugt sind Hg, In, Ga, InGa, Ag, Au, Cr, Pt, PdAu, Pb, Al, Mg, W, Yb, Zn, CNT (Kohlenstoffnanoröhren, engl.: "carbon nanotubes") , Graphen und leitfähige Polymere (wie PEDOT:PSS).

In der nachfolgenden Beschreibung der Ausführungsbeispiele der Erfindung werden gleiche oder ähnliche Komponenten und Elemente mit gleichen oder ähnlichen Bezugszeichen bezeichnet, wobei auf eine wiederholte Beschreibung dieser Komponenten oder Elemente in Einzelfällen verzichtet wird. Die Figuren stellen den Gegenstand der Erfindung nur schematisch dar.

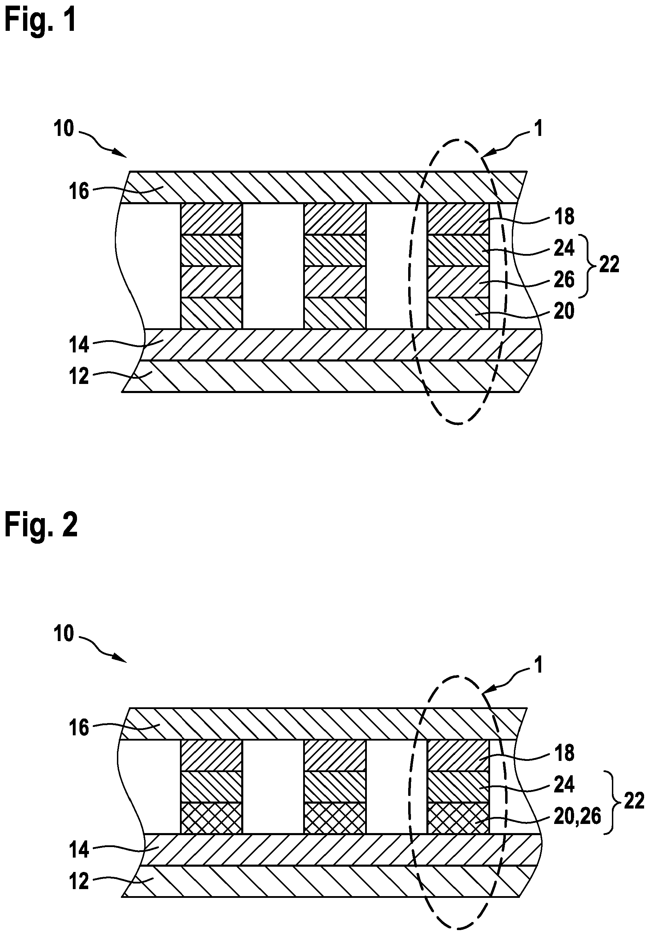

Figur 1 zeigt eine erste Ausführungsform eines elektronischen Bauelements in einer Schnittdarstellung von der Seite.

Das in Figur 1 dargestellte elektronische Bauelement (10) ist auf einem äußeren Substrat (12) angeordnet, welches beispielsweise ein Wafer sein kann, der an seiner den übrigen Komponenten des elektronischen Bauelements (10) zugewandten Seite mit einem Isolator (14) versehen wurde. Das äußere Substrat (12) besteht aus den oben beschriebenen Materialien und ist beispielsweise ausgewählt aus einem Elementhalbleiter wie Silizium (Si), Germanium (Ge), Kohlenstoff in Form von Diamant, Graphit oder Graphen, aus einem Verbindungshalbleiter, insbesondere einem II-VI Verbindungshalbleiter wie Cadmium-Selenid (CdSe), Zinksulfid (ZnS), aus einem Metall wie beispielsweise Gold, Silber, Kupfer, Aluminium, Magnesium oder aus einem leitfähigen oxidischen Material wie Indiumzinnoxid (ITO), Indium-Galliumoxid (IGO), Indium-Gallium-Zinkoxid (IGZO), Aluminiumdotiertes Zinkoxid (AZO) oder fluordotiertes Zinnoxid (FTO). Bevorzugt wird kristallines Silizium als Substrat verwendet, wobei Siliziumwafer mit (100) Oberfläche besonders bevorzugt werden. Siliziumwafer, deren Oberfläche (100) orientiert ist, werden in der Mikroelektronik als übliches Substrat eingesetzt und sind in hoher Qualität und mit geringen Oberflächendefekten verfügbar.

Der Isolator (14) kann beispielsweise ein Oxid sein, wobei dieses zum Beispiel bei Verwendung eines Siliziumsubstrats mittels Ionenimplantation von Sauerstoff-Ionen in das Substrat erhalten werden kann. Auf den Isolator sind zweite Elektroden (20) angeordnet, die in der Ausführungsform der Figur 1 in Form von Leiterbahnen ausgeführt sind, die senkrecht zur Zeichenebene verlaufen. In der in Figur 1 dargestellten Ausführungsform sind die ersten Elektroden (20) als metallische Elektroden ausgeführt. Auf den ersten Elektroden (20) sind Dioden 22 angeordnet, die beispielsweise als Zener-Dioden ausgeführt sind und jeweils eine hoch p-dotierte Schicht 26 und eine hoch n-dotierte Schicht 24 umfassen. Am Übergang der p-dotierten Schicht 26 zur n-dotierten Schicht 24 bildet sich ein pn Übergang der Diode 22 aus.

Auf der den ersten Elektroden (20) abgewandten Seite der Diode (22), welche in dieser Ausführungsform der Erfindung das erfindungsgemäße Substrat bildet, ist die molekulare Schicht (18) angeordnet. Die molekulare Schicht (18) ist bevorzugt als molekulare Monoschicht ausgeführt und damit genau eine Moleküllage dick.

Auf der der Diode (22) abgewandten Seite der molekularen Schicht (18) ist eine zweite Elektrode (16) (Gegenelektrode) angeordnet, die wie die erste Elektrode (20) als Leiterbahn ausgeführt ist. Die zweite Elektrode (16) ist gegenüber der ersten Elektrode (20) jedoch um 90° gedreht, so daß eine kreuzförmige Anordnung entsteht. Diese Anordnung wird auch Crossbar-Array (Kreuzdrahtvorrichtung) genannt, wobei der 90° Winkel hier als ein Beispiel gewählt ist und auch vom rechten Winkel abweichende Anordnungen denkbar sind, bei denen sich zweite Elektroden (16) und erste Elektroden (20) kreuzen. An jeder Kreuzungsstelle zwischen einer zweiten Elektrode (16) und einer ersten Elektrode (20) ist ein Schaltelement (1) angeordnet, welches aus einem Schichtsystem mit in dieser Reihenfolge zweiten Elektrode (16), molekularer Schicht (18) und ersten Elektrode (20) gebildet wird. In der in Figur 1 dargestellten Ausführungsform ist jedem Schaltelement (1) auch eine Diode (22) zugeordnet.

Durch das Crossbar-Array kann jedes Schaltelement (1) elektrisch angesteuert werden, indem eine Spannung zwischen der korrespondierenden ersten Elektrode (20) und zweiten Elektrode (16) angelegt wird. Über die Dioden (22) wird dabei verhindert, daß Leckströme über benachbarte Schaltelemente (1) fließen können.

Aufgrund der bipolaren Schaltcharakteristik der Schaltelemente (1) müssen die Dioden (22) eine nichtlineare Charakteristik für beide Polaritäten aufweisen. Hierzu werden die Dioden (22) beispielsweise als Zener-Dioden ausgeführt, wobei dazu sowohl die p-dotierte Schicht (26) als auch die n-dotierte Schicht (24) hoch dotiert werden.

Die Herstellung der Strukturen der Elektroden (16, 20) kann mittels dem Fachmann aus der Mikroelektronik bekannten Strukturierungsverfahren erfolgen. Beispielsweise kann zur Herstellung der ersten Elektroden 20 ein Lithographieverfahren eingesetzt werden. Dabei wird eine Metallschicht auf den Isolator (14) mittels Aufdampfen aufgebracht. Anschließend wird die Metallschicht mit einem Photoresist belackt, der mit den herzustellenden Strukturen belichtet wird. Nach dem Entwickeln und eventuellem Ausbacken des Resists werden die nicht benötigten Teile der Metallschicht beispielsweise durch nasschemisches Ätzen entfernt. Anschließend wird der restliche Resist beispielsweise mit einem Lösungsmittel abgetragen.

Die Strukturen der zweiten Elektroden (16) können auch mit einem Druckverfahren hergestellt werden, bei dem ähnlich wie beim konventionellen Druck ein leitfähiges Material auf das Bauelement (10) bzw. auf die molekularen Schicht (18) aufgetragen wird. Hierzu sind beispielsweise leitfähige Polymere wie Poly(3,4-ethylendioxythiophen)/Polystyrolsulfonat (PEDEOT:PSS) geeignet.

Eine weitere Möglichkeit zur Herstellung der Elektroden (16, 20), insbesondere der zweiten Elektroden (16), ist das Aufdampfen mit Hilfe einer Schattenmaske. Dabei wird eine Maske, deren Öffnungen der Form der herzustellenden Elektroden (16, 20) entsprechen, auf das Bauelement (10) aufgelegt und anschließend ein Metall aufgedampft. Der Metalldampf kann sich nur in den Bereichen, die nicht von der Maske verdeckt wurden, auf dem Bauelement (10) niederschlagen und die Elektrode (16, 20) ausbilden.

In Figur 2 ist eine weitere Ausführungsform des elektronischen Bauelements (10) dargestellt. In der Ausführungsform der Figur 2 bestehen die ersten Elektroden (20) aus einem Halbleitermaterial, welches dotiert wird, um gleichzeitig als ein Teil der Diode (22) zu fungieren. Diese Ausführungsform ist insbesondere bei Verwendung von Silicon-on-Insulator Wafern als äußeres Substrat (12) vorteilhaft. Ein Silicon-on-Insulator Wafer umfasst einen Schichtaufbau, wobei in dieser Reihenfolge Schichten aus Silizium, Siliziumdioxid und stark dotiertem Silizium angeordnet sind. Ein solches Substrat kann beispielsweise hergestellt werden, indem zunächst mittels Ionenimplantation mit Sauerstoff-Ionen in einer Tiefe zwischen 100 nm und 10 µm in das Siliziumsubstrat implantiert werden. Dicht an der Oberfläche werden Dotieratome implantiert, um eine p-Leitung oder n-Leitung einzustellen. Nach anschließender Wärmebehandlung entsteht dann in der Tiefe eine Schicht aus Siliziumdioxid, da sich die implantierten Sauerstoff-Ionen mit dem Silizium verbinden. Das Silizium wird als Substrat (12) verwendet, während die Siliziumdioxidschicht als Isolator (14) dient. Aus der dotierten Siliziumschicht werden die ersten Elektroden (20) mittels üblicher aus der Mikroelektronik grundsätzlich bekannter Strukturierungsverfahren hergestellt. In Fortbildung dieses Verfahrens kann auch direkt ein pn-Übergang an der Oberfläche des Silicon-on-Insulator Wafers erzeugt werden. Dazu werden mehrere Ionenimplantationsschritte durchgeführt, wobei in einem ersten Schritt über eine Volumenimplantation beispielsweise eine p-dotierte Schicht erzeugt wird und nachfolgend mit einer flachen, oberflächlichen Implantation eine n-dotierte Schicht erzeugt wird.

Auf den ersten Elektroden (20), die auch als Teil der Diode (22) fungieren, ist in der Ausführungsform der Figur 2 eine n-dotierte Schicht (24) angeordnet. Die ersten Elektroden (20) sind somit p-dotiert, um zusammen mit der n-dotierten Schicht (24) die Diode (22) mit einem pn-Übergang auszubilden. In dieser Ausführungsform bildet die n-dotierte Schicht (24) das erfindungsgemäße Substrat.

Die weiteren Schichten sind wie bereits zu Figur 1 beschrieben angeordnet, wobei wiederum jeweils ein Schaltelement (1) an einem Kreuzungspunkt einer ersten Elektrode (20) und einer zweiten Elektrode (16) ausgebildet wird.

Die Verbindungen der allgemeinen Formel I können nach an sich bekannten Methoden dargestellt werden, wie sie in der Literatur (z.B. in den Standardwerken wie Houben-Weyl, Methoden der organischen Chemie, Georg-Thieme-Verlag, Stuttgart) beschrieben sind und zwar unter Reaktionsbedingungen, die für die genannten Umsetzungen bekannt und geeignet sind. Dabei kann man von an sich bekannten, hier nicht näher erwähnten Varianten Gebrauch machen.

Die Ausgangsstoffe können gegebenenfalls auch in situ gebildet werden, derart, daß man sie aus dem Reaktionsgemisch nicht isoliert, sondern sofort weiter zu den Verbindungen der allgemeinen Formel I umsetzt.

Die Synthesen erfindungsgemäßer Verbindungen der allgemeinen Formel I werden in den Beispielen exemplarisch beschrieben. Die Ausgangssubstanzen sind nach allgemein zugänglichen Literaturvorschriften oder käuflich zu erhalten.

Besonders geeignete Synthesewege zu den erfindungsgemäßen Verbindungen werden im Folgenden anhand der Schemata 1, 2 und 3 veranschaulicht und anhand der Ausführungsbeispiele näher erläutert.

Die erfindungsgemäßen Phosphonsäuren werden vorzugsweise durch Michaelis-Arbuzov-Reaktion und anschließende säurekatalysierte Hydrolyse hergestellt (Schema 1).

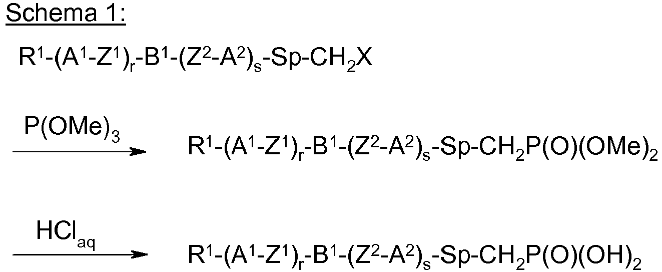

In Schema 1 bedeutet X eine Abgangsgruppe, vorzugsweise Cl, Br, I, Toluolsulfonyl, Methansulfonyl, besonders bevorzugt Br.

Bevorzugte Syntheseverfahren zur Herstellung der erfindungsgemäßen Hydroxybisphosphonsäuren sind in M. Egorov, Eur. J. Org. Chem. 2011, 7148-7154 beschrieben; in einem besonders bevorzugten Verfahren werden Carbonsäuren zunächst mit Katecholboran derivatisiert und anschließend unter Decarboxylierung mit Tris(trimethylsilyl)phosphit gefolgt von einer Methanolyse zu den erfindungsgemäßen Hydroxybisphosphonaten umgesetzt (Schema 2). Verbindungen mit perfluorierten Spacergruppen -Sp- werden bevorzugt nach A. Budinskä, J.Václavík, V. Matousek, und P. Beier, Org. Lett. 2016, 18, 5844-5847, und wie in Schema 3 veranschaulicht, hergestellt. Andere Kettenlängen als -CF₂CF₂- sind analog zugänglich.

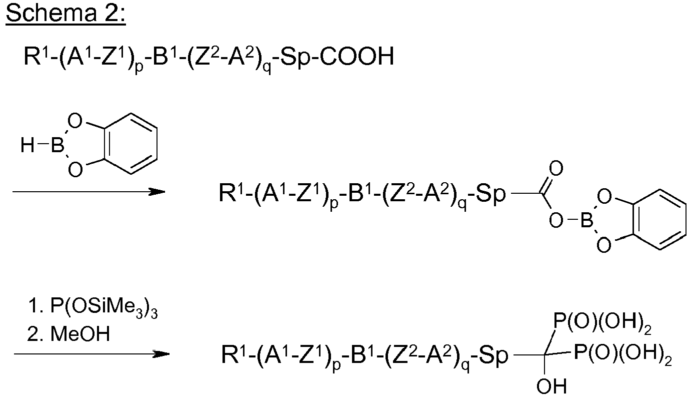

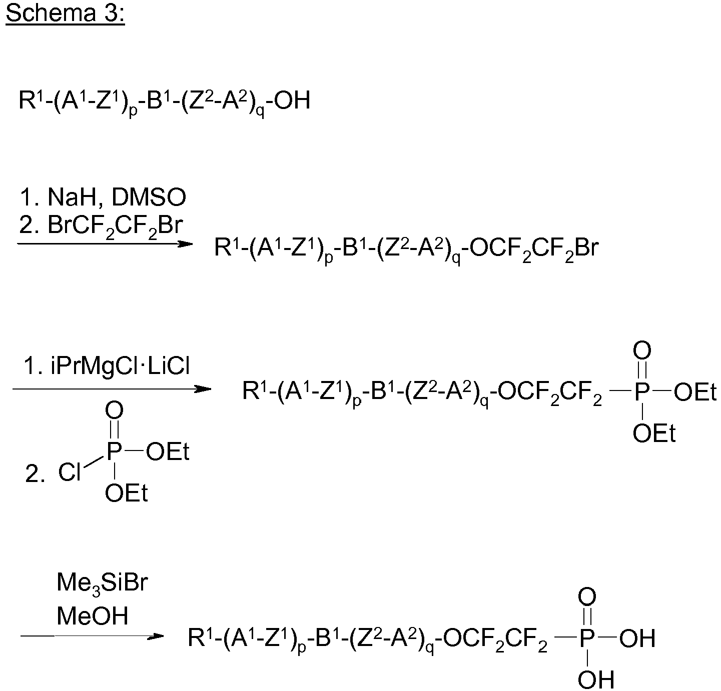

Die Erfindung ist nicht auf die hier beschriebenen Ausführungsbeispiele und die darin hervorgehobenen Aspekte beschränkt. Vielmehr sind innerhalb des durch die Ansprüche angegebenen Bereichs eine Vielzahl von Abwandlungen möglich, die im Rahmen fachmännischen Handelns liegen.

Die Erfindung wird durch die Beispiele näher erläutert, ohne sie dadurch einzuschränken.

Alle physikalischen Eigenschaften werden und wurden nach "Merck Liquid Crystals, Physical Properties of Liquid Crystals", Status Nov. 1997, Merck KGaA, Darmstadt, Deutschland bestimmt und gelten für eine Temperatur von 20°C und Δn wird bei 589 nm und Δε bei 1 kHz bestimmt, sofern nicht jeweils explizit anders angegeben.

Die flüssigkristallinen Eigenschaften der Einzelverbindungen werden, soweit nicht anders angegeben, in der nematischen Wirtsmischung ZLI-4792 (kommerziell erhältlich von Merck KGaA, Darmstadt) bei einer Konzentration von 10% bestimmt.

### Beispiele

### 1. Synthesebeispiele

Substanzbeispiel 1: 1-But-3-enoxy-2,3-difluor-4-[4-(4-methylcyclohexyl)-cyclohexyl]benzol **(CCY-5-O2V)**

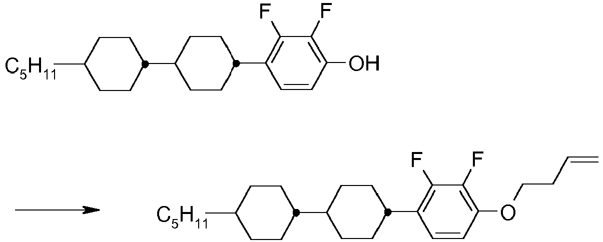

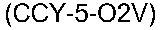

14,6 g (40 mmol) 2,3-Difluor-4-[4-(4-pentylcyclohexyl)cyclohexyl]phenol werden in 100 ml Methanol vorgelegt, zunächst 8,9 ml einer 30proz. Lösung von Natriummethanolat in Methanol und anschließend bei 50°C 6,7g (48 mmol) 4-Brom-1-buten hinzugefügt und der Ansatz wird 6 h unter Rückfluß und danach über Nacht bei Raumtemp. rühren gelassen. Das Lösungsmittel wird i. Vak. entfernt, der Rückstand mit Toluol über Kieselgel filtriert und das Rohprodukt aus Ethanol umkristallisiert. Man erhält 1-But-3-enoxy-2,3-difluor-4-[4-(4-methylcyclohexyl)-cyclohexyl]benzol als farblosen Feststoff.
Phasensequenz: K 41 SmB 131 N 159 I.
Δε = -5,8

### Substanzbeispiel 2: 1-But-3-enoxy-4-(4-butoxy-2,3-difluor-phenyl)-2,3-difluorbenzol (YY-4O-O2V)

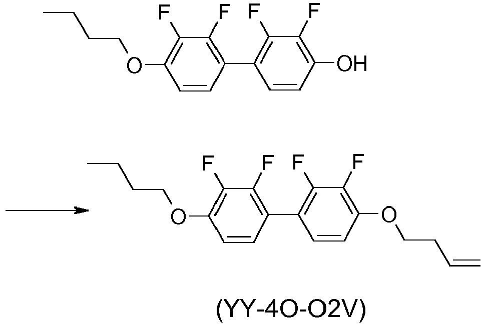

Analog der Synthese von Substanzbeispiel 1 erhält man aus 4-(4-Butoxy-2,3-difluorphenyl)-2,3-difluorphenol das 1-But-3-enoxy-4-(4-butoxy-2,3-difluorphenyl)-2,3-difluorbenzol als farblosen Feststoff vom Schmp. 73 °C. Δε = -11,9

In Analogie zu Substanzbeispiel 1 werden die Substanzbeispiele 3 bis 5 hergestellt.

### Substanzbeispiel 3

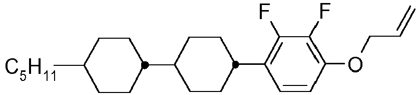

Phasensequenz Tg - 75 K 58 SmB 120 N 175 I
Δε = -5,7

### Substanzbeispiel 4

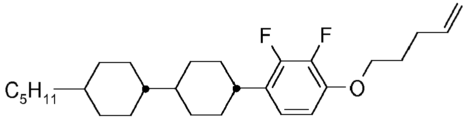

Phasensequenz Tg - 83 K 53 SmA1 127 SmA2 135 N 167 I

### Substanzbeispiel 5

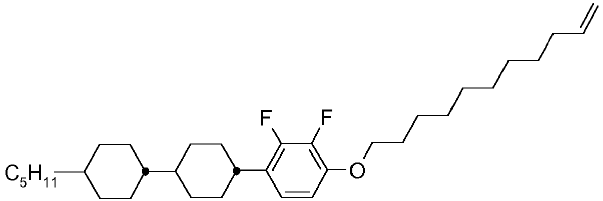

Phasensequenz K 59 SmA 124 N 136 I

### Substanzbeispiel 6: 3-[2,3-Difluor-4-[4-(4-pentylcyclohexyl)-cyclohexyl]phenoxy]-propylphosphonsäure

### Stufe 1: 1-(3-Brompropoxy)-2,3-difluor-4-[4-(4-pentylcyclohexyl)-cyclohexyl]benzol (CCY-5-O3P)

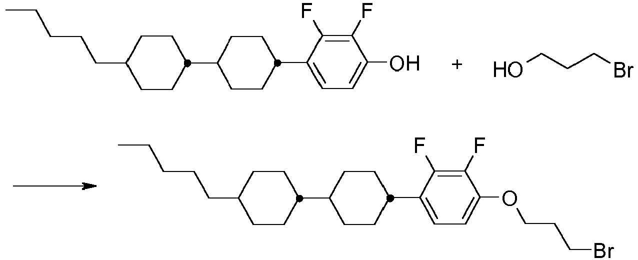

9,10 g (25,0 mmol) 2,3-Difluor-4-[4-(4-pentylcyclohexyl)cyclohexyl]phenol, 2,4 ml (27,6 mmol) 3-Brom-1-propanol und 6,89 g (26 mmol) Triphenylphosphin werden in 150 ml THF gelöst, unter Eiskühlung tropfenweise mit 5,50 ml (28 mmol) Diisopropylazodicarboxylat versetzt und über Nacht bei Raumtemp. rühren gelassen. Der Ansatz wird anschließend mit 200 ml Wasser und 100 ml MTB-Ether versetzt, die wäßrige Phase abgetrennt und dreimal mit MTB-Ether extrahiert. Die vereinigten org. Phasen werden mit Wasser und ges. Natriumchloridlsg. gewaschen und über Natriumsulfat getrocknet. Das Lösungsmittel wird i. Vak. entfernt und der Rückstand mit n-Heptan an Kieselgel chromatographisch gereinigt. Kristallisation aus Ethanol liefert 1-(3-Brompropoxy)-2,3-difluor-4-[4-(4-pentylcyclohexyl)-cyclohexyl]benzol als farblose Kristalle.

### Stufe 2: 1-(3-Diethoxyphosphorylpropoxy)-2,3-difluor-4-[4-(4-pentylcyclohexyl)-cyclohexyl]benzol

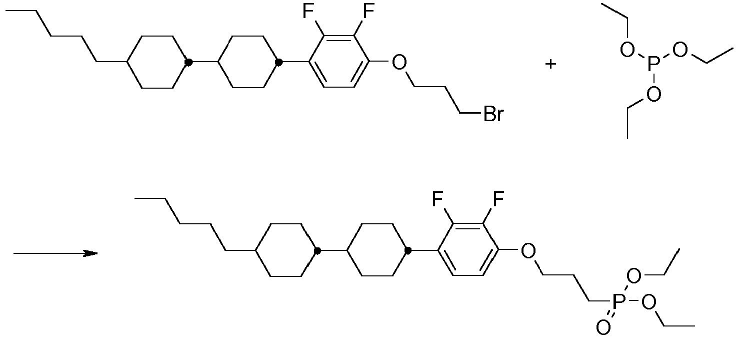

1,00 g (2,06 mmol) 1-(3-Brompropoxy)-2,3-difluor-4-[4-(4-pentylcyclohexyl)cyclohexyl]benzol und 1,1 ml (6,2 mmol) Triethylphosphit werden 18 h auf 120 °C und 8 h auf 130 °C erhitzt. Anschließend wird überschüssiges Triethylphosphit im Kugelrohr abdestilliert und der Rückstand mit Toluol/Essigester (1:1) und anschließend Essigester an Kieselgel chromatographiert. Man erhält 1-(3-Diethoxyphosphorylpropoxy)-2,3-difluor-4-[4-(4-pentylcyclohexyl)-cyclohexyl]benzol als amorphen farblosen Feststoff.

### Stufe 3: 3-[2,3-Difluor-4-[4-(4-pentylcyclohexyl)cyclohexyl]phenoxy]-propylphosphonsäure

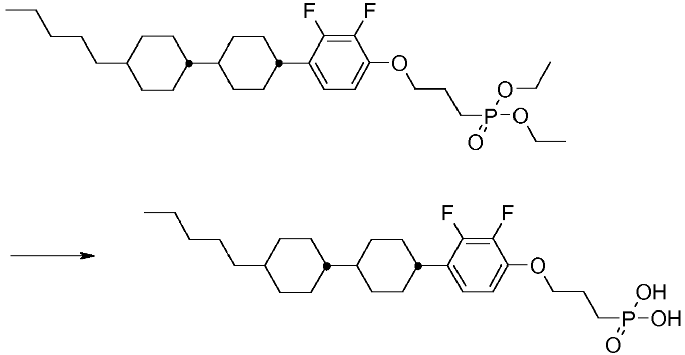

500 mg (0,921 mmol) 1-(3-Diethoxyphosphorylpropoxy)-2,3-difluor-4-[4-(4-pentylcyclohexyl)-cyclohexyl]benzol wird in 8 ml konz. Salzsäure über Nacht bei 100 °C rühren gelassen. Die Suspension wird anschließend i. Vak. zum Rückstand eingeengt, mit kaltem Wasser sowie Aceton digeriert und i. Vak. getrocknet. Man erhält 3-[2,3-Difluor-4-[4-(4-pentylcyclohexyl)-cyclohexyl]phenoxy]propylphosphonsäure als farblosen Feststoff. Phasensequenz K 117 SmX 220 (Zers.)

In Analogie zu Beispiel 6 werden die Substanzbeispiele 7 bis 20 hergestellt.

### Substanzbeispiel 7

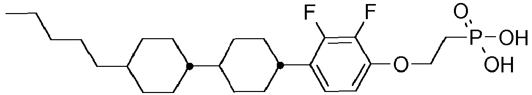

Phasensequenz K 116 (Zers.)

### Substanzbeispiel 8

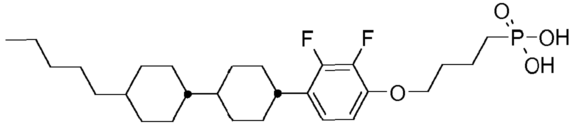

Phasensequenz K 115 (Zers.)

### Substanzbeispiel 9

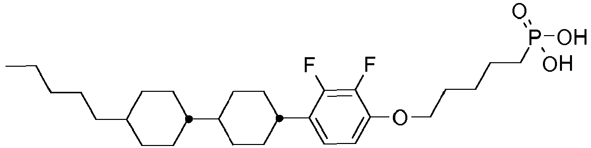

Phasensequenz K 154 (Zers.)

### Substanzbeispiel 10

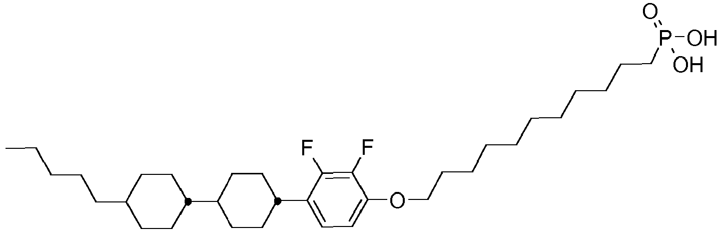

Phasensequenz K 126 (Zers.)

### Substanzbeispiel 11

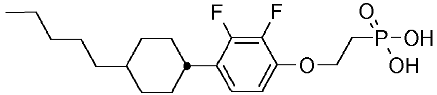

Phasensequenz K 62 SmX (Zers.)

### Substanzbeispiel 12

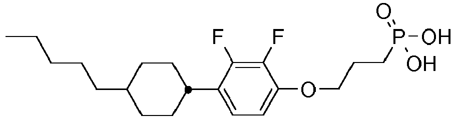

Phasensequenz K 115 SmX (Zers.)

### Substanzbeispiel 13

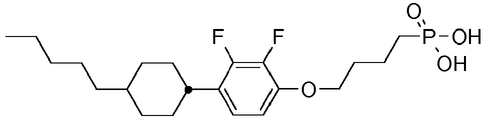

Phasensequenz Tg -17 K 84 SmX (Zers.)

### Substanzbeispiel 14

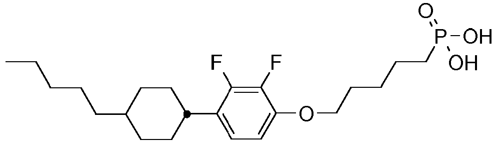

Phasensequenz Tg -17 K 84 SmX (Zers.)

### Substanzbeispiel 15

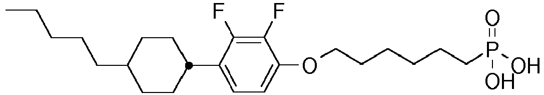

Phasensequenz K 137 SmX 197 I (Zers.)

### Substanzbeispiel 16

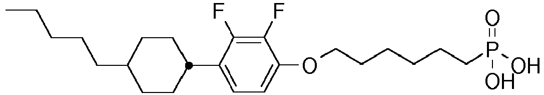

Phasensequenz K 137 SmX 197 I (Zers.)

### Substanzbeispiel 17

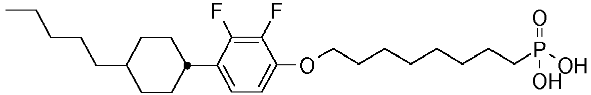

Phasensequenz K 88 SmX 161 I

### Substanzbeispiel 18

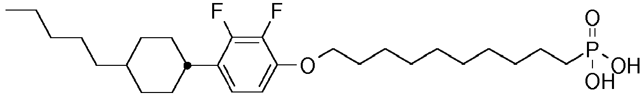

Phasensequenz K 114 SmX 145 I

### Substanzbeispiel 19

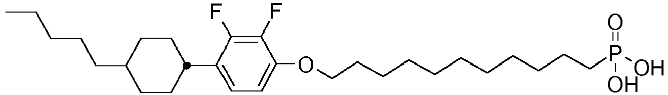

Phasensequenz K 105 SmX (Zers.)

### Substanzbeispiel 20

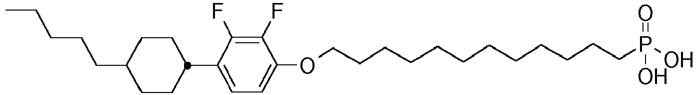

Phasensequenz K 104 SmX 135 I

### Substanzbeispiel 21: 5-[2,3-Difluor-4-[4-(4-pentylcyclohexyl)-cyclohexyl]phenoxy]butansäure

### Stufe 1: 5-[2,3-Difluor-4-[4-(4-pentylcyclohexyl)-cyclohexyl]-phenoxy]butansäureethylester

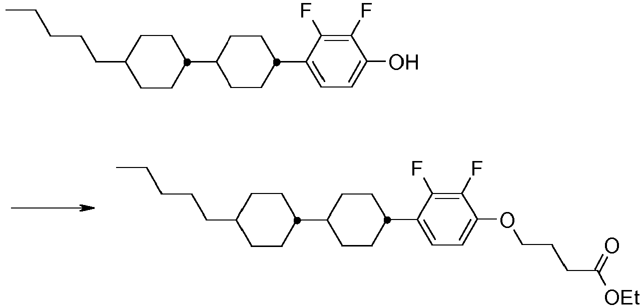

2,3-Difluor-4-[4-(4-pentylcyclohexyl)cyclohexyl]phenol (6.0 g, 16.5 mmol) werden in Aceton (60 ml) vorgelegt und nach Zugabe von Ethyl-4-brombutyrate (6.4 g, 32.9 mmol) und K₂CO₃ (4.5 g, 32.9 mmol) 16 h unter Rückfluß erhitzt. Anschließend wird der Ansatz filtriert, eingeengt, der Rückstand mit Dichlormathan/Heptan (1:1) über Kieselgel filtriert und aus Heptan umkristallisiert. Man erhält 5-[2,3-Difluor-4-[4-(4-pentylcyclohexyl)-cyclohexyl]-phenoxy]butansäureethylester als farblose Kristalle.

### Stufe 2: 5-[2,3-Difluor-4-[4-(4-pentylcyclohexyl)-cyclohexyl]phenoxy]butansäure

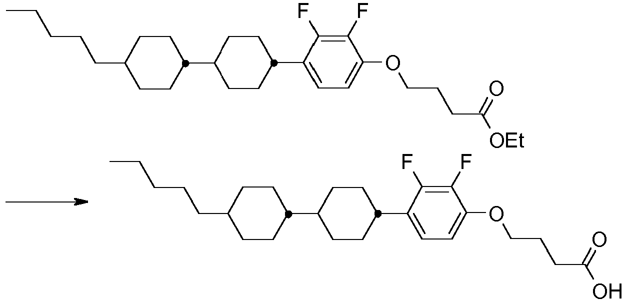

6.3 g (13.2 mmol) 5-[2,3-Difluor-4-[4-(4-pentylcyclohexyl)-cyclohexyl]-phenoxy]butansäureethylester werden in THF (250 ml) gelöst, LiOH 1M (40 ml, 3 eqv.) hinzugefügt und der Ansatz wird 16 h bei 60°C rühren gelassen. Anschließend werden 250 ml Wasser hinzugefügt und mit 3 Äquivalenten 2N Salzsäure angesäuert. Die Mischung wird mit MTB-Ether extrahiert und die vereinigten org. Phasen werden mit Wasser gewaschen und über Magnesiumsulfat getrocknet. Das Lösungsmittel wird i. Vak entfernt und der Rückstand aus einer Mischung von 60 ml Dichlormethan und 100 ml Methanol umkristallisiert. man erhält 5-[2,3-Difluor-4-[4-(4-pentylcyclohexyl)-cyclohexyl]phenoxy]butansäure als farblose Kristalle.
Phasensequenz: K 102 SmX 102 SmB 195 X 200 l (Zers.)

Analog zu Substanzbeispiel 21 wird erhalten:

### Substanzbeispiel 22: 5-[2,3-Difluor-4-[4-(4-pentylcyclohexyl)-cyclohexyl]phenoxy]pentansäure

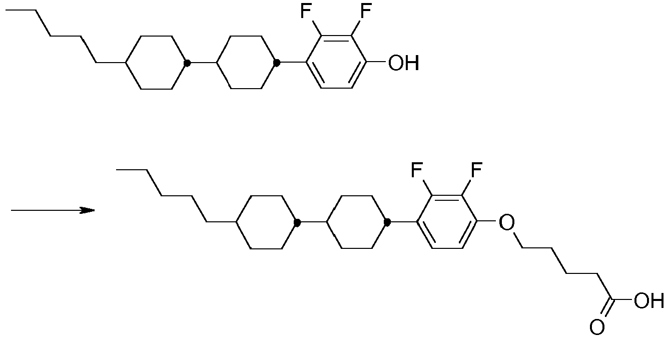

Phasensequenz: K 151 SmX 202 N 221 I (Zers.)

### Substanzbeispiel 23: [3-[2,3-Difluor-4-[4-(4-pentylcyclohexyl)-cyclohexyl]phenoxy]-1-hydroxy-1-phosphono-propyl]phosphonsäure

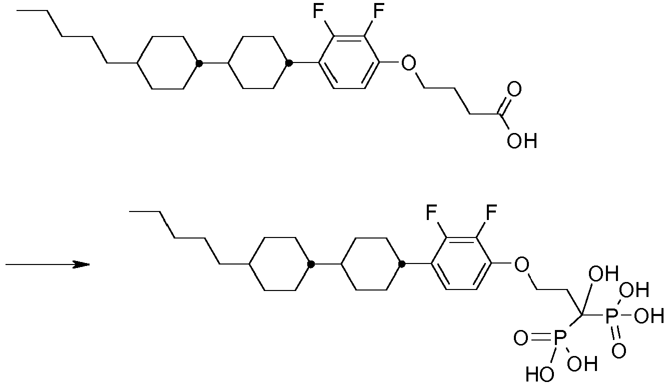

Eine 1 M Lösung von Katecholboran in THF (1,35 mL, 1,35 mmol) wird bei Raumtemp. unter Argon zu 546 mg (1,23 mmol) 5-[2,3-Difluor-4-[4-(4-pentylcyclohexyl)-cyclohexyl]phenoxy]butansäure gegeben. Der Ansatz wird etwa 1 h bei Raumtemp. rühren gelassen bis keine Gasentwicklung mehr zu beobachten ist. Anschließend werden 770 mg (2,58 mmol) Tristrimethylsilylphosphit hinzugefügt und der Ansatz über Nacht bei Raumtemp. rühren gelassen. Nach Zugabe von 4 ml Methanol wird noch 1 h gerührt und das Lösungsmittel i. Vak. entfernt. Der Rückstand wird mit Dichlormethan überschichtet und das Lösungsmittel dekantiert. Das abgeschiedene Öl wird in wenig Methanol aufgenommen, mit Ether verdünnt und das ausgefallene Produkt wird abgesaugt, mit Ether gewaschen und getrocknet. Man erhält [3-[2,3-Difluor-4-[4-(4-pentyl-cyclohexyl)-cyclohexyl]phenoxy]-1-hydroxy-1-phosphono-propyl]-phosphonsäure als farblose Kristalle.

### Substanzbeispiel 24: (4-(2,3-Difluor-4-(4'-pentyl-[1,1'-bi(cyclohexan)]-4-yl)-phenoxy)-1-hydroxybutan-1,1-diyl)diphosphonsäure Stufe 1:4-(2,3-Difluor-4-(4'-pentyl-[1,1'-bi(cyclohexan)]-4-yl)-phenoxy)butanoylchlorid

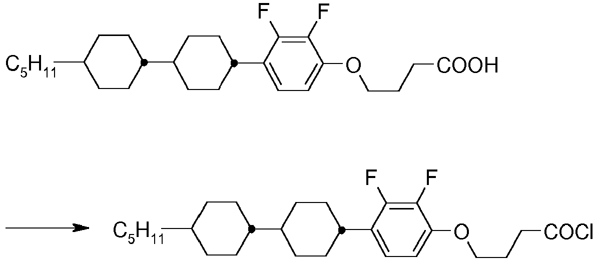

5.0 g (12 mmol) 4-(2,3-Difluor-4-(4'-pentyl-[1,1'-bi(cyclohexan)]-4-yl)-phenoxy)butansäure und 7.2 g Thionylchlorid (4.4 mL, 60 mmol) werden in 20 ml 1,2-Dichlorethan (DCE) 16 h unter Rückfluß erhitzt. Anschließend wird der Ansatz i.Vak. eingeengt und das 4-(2,3-Difluor-4-(4'-pentyl-[1,1'-bi(cyclohexan)]-4-yl)-phenoxy)butanoylchlorid ohne weitere Aufreinigung weiter umgesetzt.

### Stufe 2: (4-(2,3-Difluor-4-(4'-pentyl-[1,1'-bi(cyclohexan)]-4-yl)-phenoxy)-1-hydroxybutan-1,1-diyl) diphosphonsäure.

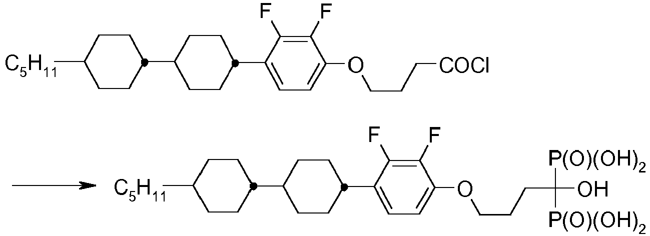

5.2 g (12 mmol) 4-(2,3-Difluor-4-(4'-pentyl-[1,1'-bi(cyclohexan)]-4-yl)-phenoxy)-butanoylchlorid werden in 30 ml Tetrahydrofuran vorgelegt und unter Eiskühlung 7.9 g (26 mmol) Tris(trimethylsilyl)phosphit zutropfen gelassen. Nach 2 h wird die Kühlung entfernt und der Ansatz 8 h bei Raumtemp. gerührt. Die Lösung wird i. Vak. eingeengt und der Rückstand 4 h mit Methanol digeriert. Der erhaltene Niederschlag wird abfiltriert, zweimal mit Methanol gewaschen und i. Vak. getrocknet. Man erhält (4-(2,3-Difluor-4-(4'-pentyl-[1,1'-bi(cyclohexan)]-4-yl)-phenoxy)-1-hydroxybutan-1,1-diyl)diphosphonsäure als farblosen Feststoff vom Schmp. 135°C

In analoger Weise erhält man

### Substanzbeispiel 25

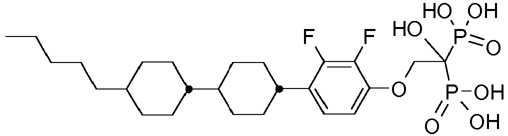

Phasensequenz K 170 (Zers.)

### Substanzbeispiel 26

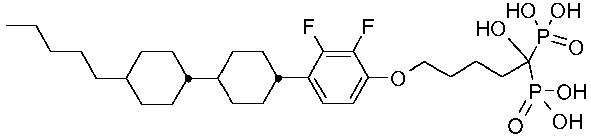

### Substanzbeispiel 27

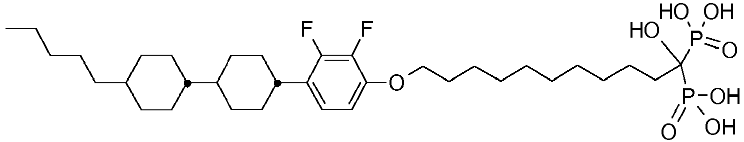

Phasensequenz K 48 SmX 120 (Zers.)

In Analogie zu den oben beschriebenen Synthesen erhält man aus 2,3-Difluorhydrochinonmonoethylether die folgende Verbindung: Substanzbeispiel 28 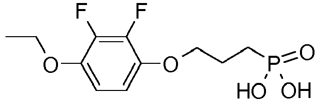 Schmp. 93°C.

### Substanzbeispiel 29: [2-[2,3-Difluor-4-(4-pentylcyclohexyl)phenoxy]-1,1,2,2-tetrafluorethyl]phosphonsäure

### Stufe 1: 1-(2-Brom-1,1,2,2-tetrafluorethoxy)-2,3-difluor-4-(4-pentylcyclohexyl)benzol

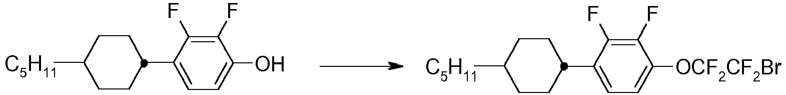

6.0 g (21 mmol) 2,3-Difluor-4-(4-pentylcyclohexyl)phenol werden unter Argon in 40 ml Dimethylsulfoxid vorgelegt und Natriumhydrid (1.0 g, 25 mmol, 60% Dispersion in Mineralöl) portionsweise bei Raumtemp. zugegeben. Nach beendeter Zugabe wird der Ansatz 30 min rühren gelassen, 1,2-Dibromtetrafluorethan (10.9 g) langsam hinzugefügt und anschließend 6 h auf 60°C erwärmt. Nach dem Abkühlen wird der Ansatz mit 120 ml Wasser verdünnt und dreimal mit 50 ml Petrolether extrahiert. Die vereinigten org. Phasen werde i. Vak eingeengt und das Rohprodukt mit Petrolether an Kieselgel chromatographiert. Man erhält 1-(2-Brom-1,1,2,2-tetrafluorethoxy)-2,3-difluor-4-(4-pentylcyclohexyl)benzol als farblosen Feststoff.

### Stufe 2: 1-(2-Diethoxyphosphoryl-1,1,2,2-tetrafluorethoxy)-2,3-difluor-4-(4-pentylcyclohexyl)benzol

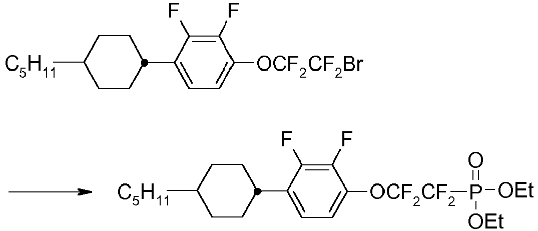

2,0 g (4,3 mmol) 1-(2-Brom-1,1,2,2-tetrafluorethoxy)-2,3-difluor-4-(4-pentylcyclohexyl)benzol wird in 20 ml Tetrahydrofuran (THF) bei -78°C vorgelegt und mit einer 1.3 M Lösung von *i*-PrMgCl·LiCl in THF versetzt. Nach 3 min werden 1.1 Äquivalente Diethylchlorphosphonat in THF hinzugefügt, die Kühlung entfernt und der Anstz 1 h bei Raumtemp. rühren gelassen. Nach wäßriger Aufarbeitung wird das Rohprodukt mit Petrolether/Essigesster (3:1) and Kieselgel chromatographiert. Man erhält 1-(2-Diethoxyphosphoryl-1,1,2,2-tetrafluorethoxy)-2,3-difluor-4-(4-pentylcyclohexyl)benzol als gelbes Öl.

### Stufe 3: [2-[2,3-Difluor-4-(4-pentylcyclohexyl)phenoxy]-1,1,2,2-tetrafluorethyl]phosphonsäure

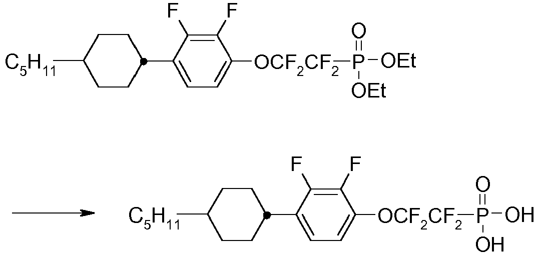

Bromtrimethylsilan (5,20 g, 34 mmol) wird unter Argon tropfenweise zu 1-(2-Diethoxyphosphoryl-1,1,2,2-tetrafluorethoxy)-2,3-difluor-4-(4-pentylcyclohexyl)benzol (1,63 g, 3,4 mmol) gegeben und 12 h unter Rückfluß erhitzt. Anschließend werden die flüchtigen Bestandteile im Feinvakuum entfernt und das Rohprodukt bei Raumtemp. 8 h mit Methanol digeriert. Anschließend wird das Lösungsmittel i. Vak. entfernt und man erhält [2-[2,3-Difluor-4-(4-pentylcyclohexyl)phenoxy]-1,1,2,2-tetrafluorethyl]phosphonsäure als farblosen Feststoff.
Phasensequenz K 65 SmX 112 N 140 I

Eine Referenzverbindung der Formel 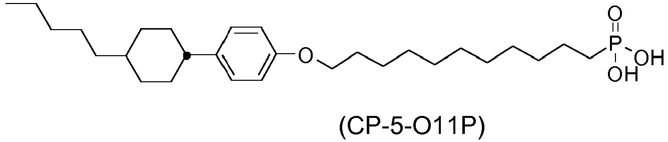 wird wie Substanzbeispiel 19 hergestellt.

### 2. Anwendungsbeispiele

### Derivatisierung von Siliciumoberflächen durch Si-C-Verknüpfung

Im Prinzip erfolgt hier die Derivatisierung von Siliziumoberflächen analog zu O. Seitz et al., Langmuir 22 (2006), 6915-6922. Das Siliziumsubstrat wird zunächst mit Aceton im Ultraschallbad von organischen Verunreinigungen gereinigt und dann mit "Piranha-Lösung" (konz. H₂SO₄/30% H₂O₂ 70:30) behandelt. Nach dem Spülen mit Wasser wird das Substrat unter Sauerstoffausschluss mit wäßriger NH₄F-Lösung behandelt und anschließend mit sauerstofffreiem Wasser gewaschen. Das nun wasserstoffterminierte Substrat wird für 12 h bei 120°C unter strengem Sauerstoffausschluss mit einer 10%igen Lösung des Derivatisierungsreagenzes in 1,2-Dichlorbenzol behandelt. Flüssige Derivatisierungsreagenzien können auch ohne Lösungsmittel verwendet werden. Anschließend wäscht man das derivatisierte Substrat mit Aceton im Ultraschallbad, spült mit Isopropanol nach und trocknet in staubfreier Umgebung mit einem Stickstoffstrahl.

### Durchführung

Ein frisch hergestellter, Wasserstoff-terminierter Chip (8 mm × 8 mm × 575±25 µm, 100-Orientierung, hochgradig Bor-dotiert) wird in einem mit Argon gespülten Schlenk-Gefäß mit entgastem Derivatisierungsreagenz (z.B. einer 10proz. (w/w) Lösung von 1-But-3-enoxy-2,3-difluor-4-[4-(4-methylcyclohexyl)-cyclohexyl]benzol aus Substanzbeispiel 1 in 1,2-Dichlorbenzol) für 18 h auf 110°C erhitzt. Der nun organomodifizierte Chip wurde aus dem Reaktionsgefäß entnommen, 5 min mit Aceton im Ultraschallbad gespült, mit Aceton und Isopropanol nachgespült und im Stickstoffstrom getrocknet. Der derivatisierte Chip wird in einem Eppendorf-Gefäß aufbewahrt.

### Derivatisierung von Siliziumoberflächen durch Si-O-Verknüpfung

Die Derivatisierung von Siliziumoberflächen durch Ausbildung einer Si-O-Verknüpfung erfolgt bevorzugt über eine Hydrophilisierung mit Sauerstoffplasma zur Erzeugung einer Hydroxylgruppen-haltigen Siliziumoxidoberfläche und anschließende Veresterung mit geeigneten Derivatisierungsreagenzien, wie Phosphonsäuren, Phosphorsäuren, Carbonsäure, Alkoholen, Trialkoysilanen, Trichlorsilanen, usw. Eine solche Behandlung ist im folgenden am Beispiel der Umsetzung mit Phosphonsäuren näher erläutert.

### Derivatisierung von Siliziumoberflächen mittels einer Zwischenschicht aus Aluminiumoxid

Hier wird ein Silizium-Wafer durch ein ALD-Verfahren (engl.: atomic layer deposition) mit einer beispielsweise 2 nm dicken Al₂O₃ Schicht beschichtet, die in einem zweiten Schritt mit geeigneten, bereits für Siliziumdioxidoberflächen beschriebenen Derivatisierungsreagenzien derivatisiert werden kann. In einem bevorzugten Verfahren wird naßchemisch mit Hilfe der Vorläufersubstanzen (precursor) Trimethyl-Aluminium und Wasser Al₂O₃ auf der Waferoberfläche abgeschieden. Eine solche Behandlung ist im folgenden am Beispiel der Umsetzung mit Phosphonsäuren näher erläutert.

### Topographische und elektrische Charakterisierung

Ein memristives Schaltverhalten wurde für mehrere dipolare Monoschichtsysteme gemessen, so daß diese als erfindungsgemäße Ausführungsbeispiele verifiziert werden konnten. Alle Schichten wurden auf p+ Si (100)-Substraten präpariert. Es wurden dabei die in der dritten Spalte der nachfolgenden Tabelle angegebenen organischen Gruppen als Monolagen erzielt, wobei dafür die in der zweiten Spalte angegebenen Vorstufen eingesetzt wurden.

| Beispiel | Vorstufe | Monolage |
|---|---|---|
| 1 | Substanzbeispiel 1 (CCY-5-O2V) | 2,3-Difluor-4-[4-(4-pentylcyclohexyl)cyclo-hexyl]-1-phenoxybutyl (CCY-5-O4) |
| 2 | Substanzbeispiel 2 (YY-4O-O2V) | 1-But-3-enoxy-4-(4-butoxy-2,3-difluorphenyl)-2,3-difluorbenzol (YY-4O-O4) |
| 3 | Substanzbeispiel 6 (CCY-5-O3P) | 2,3-Difluor-4-[4-(4-pentylcyclohexyl)-cyclohexyl]-1-phenoxy-propylphosphonat (CCY-5-O3P) |
| 4 | Substanzbeispiel 19 (CY-5-O11P) | 11-[2,3-Difluor-4-(4-pentylcyclohexyl)phenoxy]undec ylphosphonsäure (CY-5-O11P) |

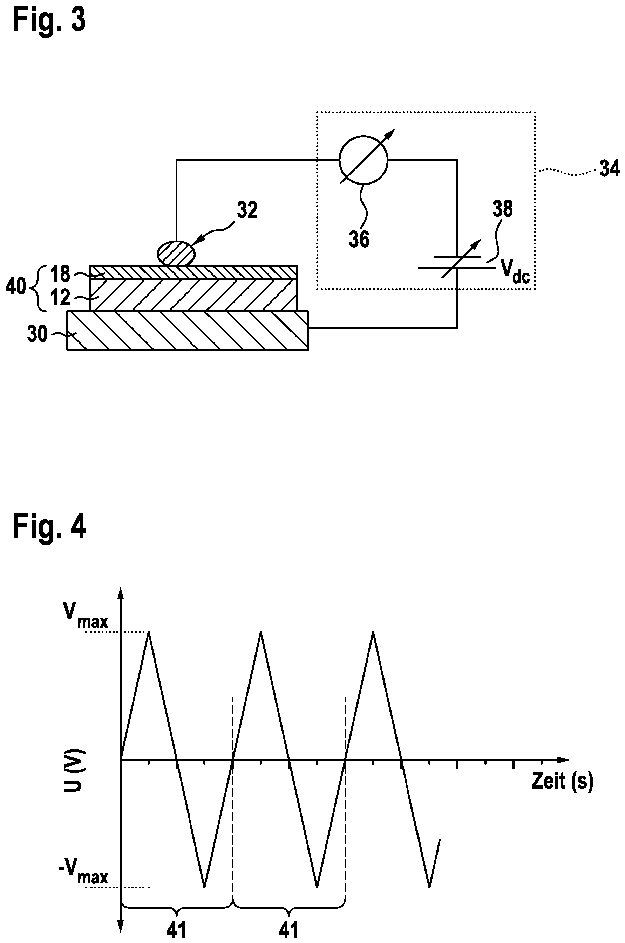

Die elektrischen Messungen an verschiedenen Proben werden nachfolgend mit Bezug zu den Figuren 3 bis 16 beschrieben.

Figur 3 zeigt einen Versuchsaufbau zur Charakterisierung der elektrischen Eigenschaften einer molekularen Schicht auf einem Substrat.

In Figur 3 ist schematisch dargestellt, wie die elektrischen Eigenschaften einer Probe (40) ermittelt werden. Die Probe (40) umfaßt das Substrat (12), auf das eine molekulare Schicht (18) aufgebracht wurde. Das Substrat (12) ist elektrisch leitend und dient hierbei als eine Elektrode, um die molekulare Schicht (18) elektrisch zu kontaktieren. Die elektrische Verbindung zu einem Meßgerät (34) wird dabei über eine bewegliche Kupferplatte (30) hergestellt. Die Probe (40) wird dazu auf der Kupferplatte (30) plaziert und kann durch Bewegen der Kupferplatte (30) relativ zu einer weiteren Elektrode bewegt werden. Als weitere Elektrode zur elektrischen Kontaktierung der Oberseite der molekularen Schicht (18) dient ein hängender Quecksilbertropfen (32), der über seine Aufhängung ebenfalls mit dem Meßgerät (34) in Verbindung steht. Der Durchmesser des Quecksilbertropfens (32) beträgt typischerweise etwa 150 µm.

Nach dem Plazieren der Probe (40) auf der Kupferplatte (30) wird diese in Relation zum Quecksilbertropfen (32) so bewegt, daß der Quecksilbertropfen (32) die Oberfläche der molekularen Schicht (18) berührt. Dies ermöglicht eine zerstörungsfreie und wechselwirkungsfreie Prüfung der elektrischen Leitfähigkeitseigenschaften der Probe (40).

Für die elektrischen Messungen ist das Meßgerät (34) bevorzugt als Source-Measure Unit ausgeführt, das heißt das Meßgerät (34) stellt über eine Spannungsquelle (38) eine Ausgangspannung bereit und mißt gleichzeitig über eine Strommeßeinheit (36) den resultierenden elektrischen Strom.

Für die Messungen wird zwischen der Kupferplatte (30) und dem Quecksilbertropfen (32) eine elektrische Spannung angelegt und variiert, gleichzeitig wird der elektrische Strom durch die Probe (40) gemessen. Dabei variiert die Spannung zyklisch zwischen einem vorgegebenen Maximalwert Vₘₐₓ und einem vorgegebenen Minimalwert Vₘᵢₙ, wie in Figur 4 dargestellt. Für die Versuche wurde eine Source-Measure-Unit vom Typ Keithley 2635 eingesetzt.

Figur 4 zeigt beispielhaft mehrere Zyklen (41), in denen die angelegte Spannung zyklisch zwischen Vₘₐₓ und Vₘᵢₙ variiert wird, wobei ein sägezahnförmiger Spannungsverlauf entsteht.

An die beiden Elektroden (das Substrat (12) und der Quecksilbertropfen (32), vergleiche Figur 3) wird über das Meßgerät (34) eine Gleichspannung angelegt, welche über die Zeit in einer zyklischen Abfolge zwischen einem maximalen negativen und maximalen positiven Spannungswert bei konstanter Rate, beispielsweise 5 mV/s variiert. In der Figur 4 sind drei solcher Zyklen (41) eingezeichnet. Der resultierende Strom durch die zu charakterisierende Probe 10 wird gemessen und aufgezeichnet.

Die aufgezeichneten Ströme für verschiedene Proben werden in den folgenden Figuren dargestellt und in der dazugehörigen Beschreibung näher erläutert.

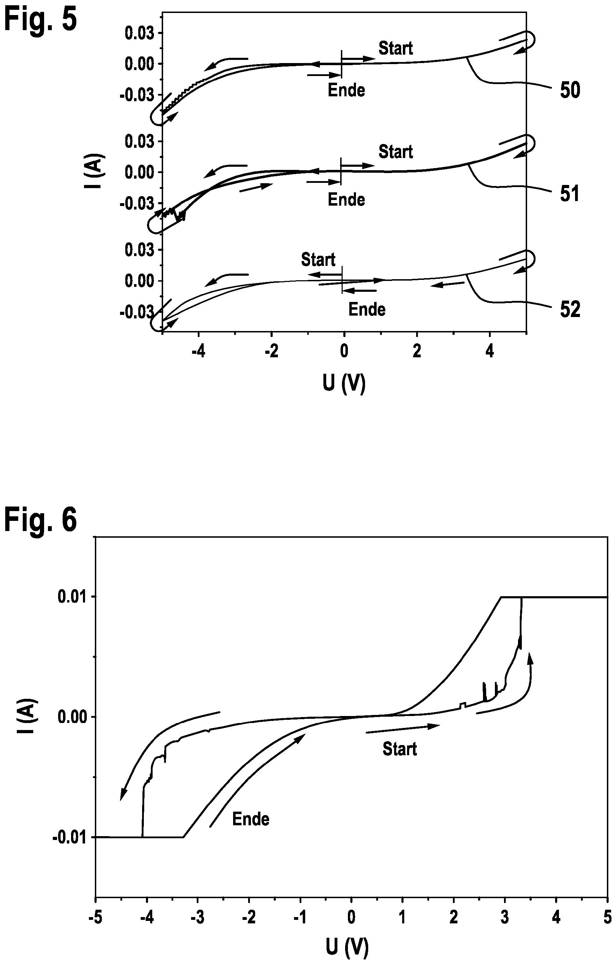

Figur 5 zeigt den aufgezeichneten Strom in Abhängigkeit der angelegten Spannung durch drei verschiedene Proben mit dem Monolagensystem CCY-5-O4.

Man erkennt ein charakteristisches, schwach ausgeprägtes hysteretisches Verhalten im Bereich negativer Vorspannungen des Substrates.

Die hier gemessenen, geringen Ströme können besonders vorteilhaft für Speicheranwendungen sein. Typisch bei Memristoren auf Grundlage der Bildung von Metallfilamenten gemessene ON-Ströme sind sehr hoch (100 mA ― Bereich) und stellen eine besondere Problematik in den elektronischen Schaltungen dar (z.B. Leistungsverbrauch, Wärmeentwicklung). Das mittels Hg-Elektrode gemessene R_{HRS}:R_{LRS}-Verhältnis des Systems CCY-5-O4 liegt bei ca. 1.4 (Auslesespannung -4 V).

### Elektrische Messungen mit permanenter zweiter Elektrode

Per Schattenbedampfungstechnik werden anstelle der Hg-Elektrode (vgl. Figur 3) permanente, dünne Metallfilme aus Pb (200 nm) mit nachfolgend Ag (150 nm) auf eine YY-4O-O4-Probe aufgebracht. Die so präparierten oberen Elektroden (80 µm × 80 µm) werden mit einer Meßnadel kontaktiert. Im Übrigen verlaufen die elektrischen Messungen analog zur oben beschriebenen Anordnung bzw. Vorgehensweise.

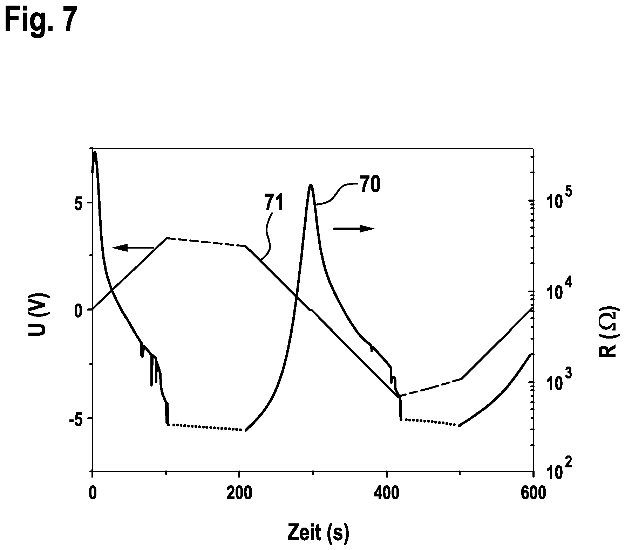

Figur 6 zeigt das charakteristische, ausgeprägt hysteretische Schaltverhalten für dieses System, welche anhand von mind. 4 auseinander folgenden Testzyklen reproduziert werden kann, ohne daß eine nennenswerte Degradation des Effekts bzw. der Probe auftritt. Um die memristive Struktur gegen mögliche Degradation oder Zerstörung durch zu hohe Ströme zu schützen, wird der maximale Strom auf +/- 10 mA begrenzt ("current compliance"). Das OFF-ON Verhältnis der beiden Widerstandswerte R_{HRS}:R_{LRS} beträgt ca. ~2 (bei einer Auslesespannung von +0.05V) bzw. ~70 (bei -0.05V). Figur 7 zeigt die charakteristische Variation des Widerstands (70) als Funktion der Zeit über einen Spannungszyklus (71).

### Herstellung und Charakterisierung einer Kreuzdrahtvorrichtung (Engl.: "cross bar array" ) unter Verwendung von Phosphonsäuren Bauteilherstellung

Die Herstellung der Bauteile umfassen mindestens die folgenden Schritte:
i. Wafer-Behandlung zur Herstellung der ersten Elektroden (20)
ii. Abscheidung der Phosphonsäure-Monolage (18)
iii. Aufbringen einer zweiten Elektrode (16)

Im folgenden werden die Prozeßschritte im Detail beschrieben:

### i. Wafer-Behandlung zur Herstellung der ersten Elektroden (20)

Ausgangsmaterial ist ein Silizium-auf-Isolator-Wafer (engl.: "silicon-oninsulator (SOI) wafer") mit einem Durchmesser von 6 Zoll, mit einer 110 nm dicken Siliziumschicht mit [100]-Orientierung auf einer 200 nm dicken Siliziumoxidschicht auf einem 525 µm dicken Wafer aus leicht Bordotiertem Silizium mit [100]-Orientierung und einem spezifischen Widerstand von ca. 20 Ω·cm.

Die obere Siliziumschicht ist durch lonenimplantation hoch Bor-dotiert (Dotier-Konzentration c ~ 5×10¹⁹ cm⁻³ , spezifischer Widerstand p ~1.4 mΩ.cm). Nach der Dotierung wird der Wafer in quadratische Teile ("Chips") der Größe 8mm × 8mm zerteilt. Die Chips werden jeweils 10 min im Ultraschallbad zunächst in Aceton und anschließend in Isopropanol gereinigt.

Die Strukturierung zur Herstellung der Silizium-Leiterbahnen für die ersten Elektroden (20) erfolgt durch Photolithographie und anschließendes selektives Trockenätzen mittels reaktiver Ionen. Im vorliegenden Beispiel werden acht Siliziumleiterbahnen hergestellt

Zur Kontaktierung der Siliziumleiterbahnen werden in einem zweiten analogen Photolithographieschritt quadratische Kontaktflächen 200 bestehend aus einer Schicht Chrom (10 nm) gefolgt von Gold (130 nm ) alternierend an die Enden der Leiterbahnen durch Elektronenstrahlverdampfen aufgedampft und der Photolack entfernt. Es werden verschiedene Chips mit einer Breite der Siliziumleiterbahnen von jeweils 25 µm, 35 µm und 50 µm hergestellt.

### ii. Abscheidung der Phosphonsäure-Monolage (18)

Die wie oben beschrieben frisch hergestellten Chips werden erneut im Ultraschallbad jeweils 5 min in Aceton und Isopropanol gereinigt und anschließend bei 70 °C mit Piranha-Lösung behandelt. Nach dem Spülen mit entionisiertem Wasser werden die Chips mit Sauerstoffplasma (200 W, 7 min) behandelt, wodurch eine mit Hydroxylgruppen belegte Siliziumdioxidschicht auf den Siliziumleiterbahnen erzeugt wird, was die Oberfläche hydrophilisiert und reaktiv gegenüber beispielsweise Phosphonsäuren macht.

Anschließend werden die Chips zur Abscheidung der Phosphonsäure-Monolage (18) auf die Siliziumleiterbahnen (20) mit einer 250 µM Lösung von CCY-5-O3P (Substanzbeispiel 6) in Tetrahydrofuran tauchbeschichtet, danach über Nacht im Ofen auf 120 °C erhitzt und danach mit Ethanol gewaschen. Dieses Verfahren entspricht im wesentlichen der literaturbekannten "TBAG"-Methode und liefert eine selbstorganisierte Monoschicht (SAM) (18) von im vorliegenden Beispiel CCY-5-O3P.

### iii. Aufbringen einer zweiten Elektrode

Auf die wie unter den Schritten i. und ii. beschrieben hergestellte Monoschicht (18) auf der ersten Elektrode (20) werden durch eine Schattenmaske zweite Elektroden (16) aus Blei der Dicke 200 nm mit einer Abscheidungsrate von 5 Å/s aufgedampft. Die Schattenmaske steht in verschiedenen Ausführungsformen zur Verfügung und besitzt parallele Schlitze der Breite von wahlweise 25 µm, 35 µm oder 50 µm, entsprechend der Breite der Siliziumstreifen der ersten Elektroden (20), die an den Enden größere quadratische Aussparungen aufweisen, wodurch in demselben Verfahrensschritt zur späteren Kontaktierung an den Enden der Leiterbahnen quadratische Kontaktflächen (160) ebenfalls aus Blei hergestellt werden. Im vorliegenden Beispiel werden acht Bleileiterbahnen senkrecht zu den acht Siliziumleiterbahnen aufgedampft, wodurch sich 64 erfindungsgemäße Schaltelemente (1) an den jeweiligen Kreuzungspunkten ergeben.

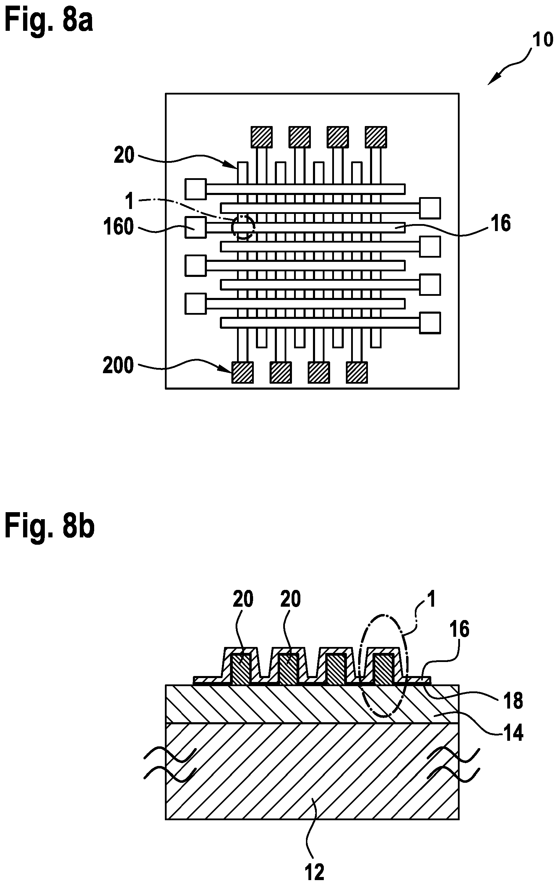

In Figur 8a ist eine auf die oben beschriebene Weise hergestellte Kreuzdrahtvorrichtung in der Draufsicht schematisch dargestellt, wobei aus Gründen der Übersichtlichkeit auf die Darstellung der molekularen Schicht (18) verzichtet wurde. Figur 8b zeigt dieselbe Kreuzdrahtvorrichtung im Längsschnitt entlang einer zweiten Elektrode (16) von der Seite.

### Elektrische Messungen und Charakterisierung

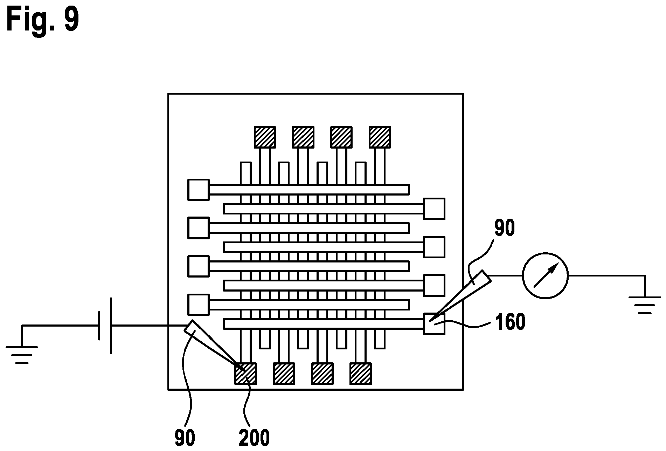

Eine Meßvorrichtung ist zur Kontaktierung der erfindungsgemäßen Kreuzdrahtvorrichtung mit Beryllium-Kupfer-Kontaktnadeln (90) mit einem Durchmesser an der Spitze von 25 µm ausgestattet. Durch Kontaktierung von jeweils einer ersten Elektrode (20) (im vorliegenden Beispiel aus Silizium) an der entsprechenden Kontaktfläche (200) mit einer ersten Kontaktnadel (90) und einer zweiten Elektrode (16) (im vorliegenden Beispiel aus Blei) an der entsprechenden Kontaktfläche (160) mit einer zweiten Kontaktnadel (90) können nacheinander alle 64 Schaltelemente (1) vermessen werden. Eine solche Meßanordnung ist in Figur 9 beispielhaft dargestellt. Zur elektrischen Charakterisierung der erfindungsgemäßen Kreuzdrahtvorrichtung werden unter Vakuum (~3·10⁻⁶ mbar, 20 °C) mit einer Keithley 2635 SMU zyklische Strom-Spannungs-Kurven aufgenommen. Die jeweilige zweite Elektrode (16) wurde geerdet und die Vorspannung an die jeweilige erste Elektrode (20) angelegt.

### Meßergebnisse

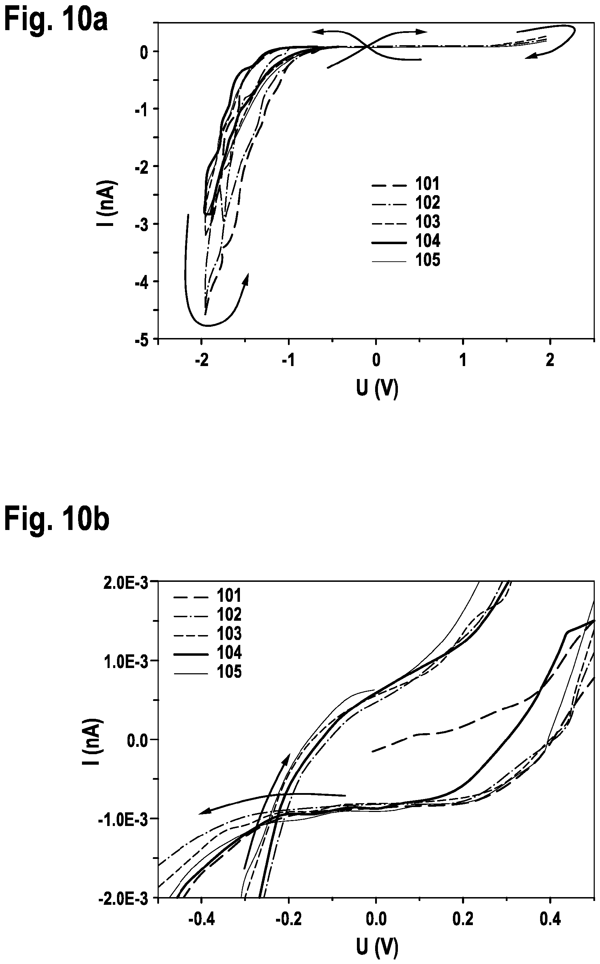

Figur 10a zeigt fünf ausgewählte Strom-Spannungs-Kurven aus 20 Meßzyklen (101, 102, 103, 104 und 105) an einem Schaltelement der Größe 50 × 50 µm², das wie oben beschrieben unter Verwendung von CCY-5-O3P hergestellt wurde. Die Spannung wurde gemäß Figur 4 variiert (Dreiecksform) und zwar pro Zyklus ausgehend von 0 V nach +2 V, von +2V nach 0 V, dann von 0 V nach -2 V und zurück nach 0 V. Fig. 10b zeigt eine Vergrößerung eines Ausschnittes aus Figur 10a im Bereich niedriger Vorspannung. Alle Kurven außer der ersten (beginnend bei 0 V und 0 A) sind aufgrund von Ladeströmen gegenüber 0 A verschoben, jeweils in Abhängigkeit von Richtung und Geschwindigkeit der Spannungsänderung. Wie durch die Pfeile in Figur 10a angezeigt, zeigen die zyklischen Strom-Spannungskurven eine deutliche, stark asymmetrische memristive Charakteristik.

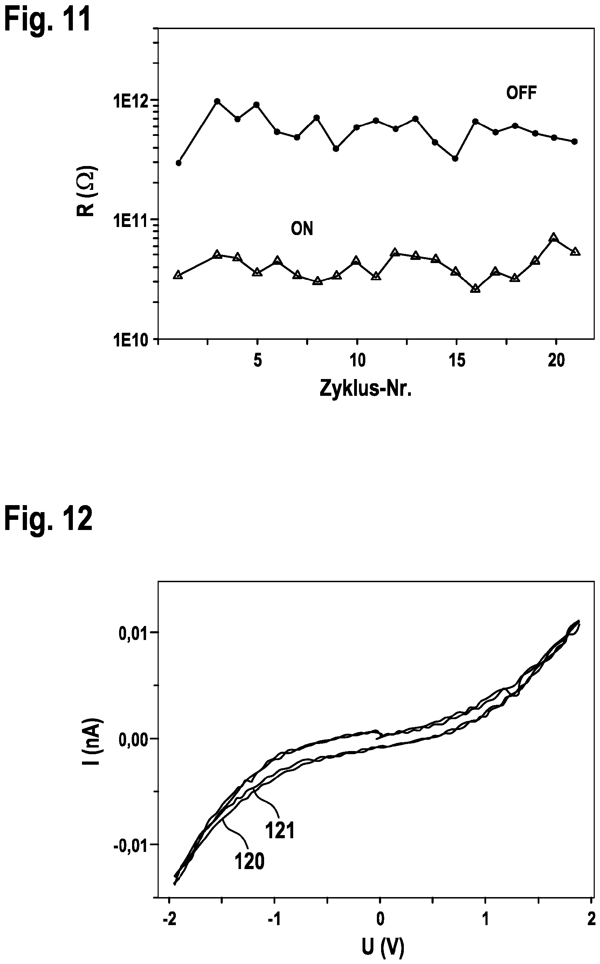

Figur 11 zeigt die aus den Werten der in Figur 10b dargestellten 20 Strom-Spannungskurven abgeleiteten Widerstandswerte bei geringen Vorspannungen (lineare Regression von 0 V bis -150 mV). Das untersuchte Schaltelement weist im Rahmen der Meßgenauigkeit konstante Widerstandswerte von Zyklus zu Zyklus auf. Die Widerstandwerte im ausgeschalteten Zustand (OFF) sind etwa um den Faktor 14 höher als im eingeschalteten Zustand (ON). Die Schaltelemente sind hervorragend für die Herstellung memristiver Speicher geeignet.

### Referenzmessung:

Figur 12 zeigt den aufgezeichneten Strom für zwei Zyklen (120, 121) der I-U-Charakteristik einer Alkylphosphonat-funktionalisierten ("C18-PA") Si-Probe als passives Referenzsystem. Es wird kein Schalt- bzw. für memristive Systeme charakteristisches, hysteretisches Verhalten beobachtet. Die schwach ausgeprägte Hysterese über den gesamten Spannungsbereich ist auf kapazitive Ladeströme zurückzuführen. Insbesondere bleiben die Widerstandswerte bei kleinen Auslesespannungen im Rahmen der Meßgenauigkeit konstant (=unabhängig von der Vorgeschichte des Bauelements).

### Charakterisierung von Phosphonsäuremonoschichten auf Titannitrid als alternatives Substratmaterial für die erste Elektrode (20)

### Herstellung der Monoschichten

Ein mit einer 30 nm dicken Schicht Titannitrid beschichteter p+-Si(100)-Wafer wird in Chips der Größe 8 mm × 8 mm zerteilt und im Ultraschallbad jeweils 5 min in Aceton und Isopropanol gereinigt. Anschließend werden die Chips 3 min mit Sauerstoffplasma (200 W) behandelt und sofort danach in eine 1 mM Lösung von CCY-5-O3P in Tetrahydrofuran/Methanol (1:1) gegeben. Nach 72 h wurden die Chips aus der Lösung genommen, mit Ethanol gewaschen und untersucht.

Die folgenden Eigenschaften wurden bestimmt:

| Parameter | | |
|---|---|---|
| | Vergleich TiN ohne SAM nach Plasmabehandlung | Eigenschaften SAM auf TiN |
| Kontaktwinkel von Wasser | <10° | 100 - 102 ° |
| Oberflächenrauhigkeit | 0.28 nm | 0.31 nm (0.32 nm für C18 Referenz) |
| Schichtdicke (ellipsometrisch) | (ohne) | 1.2 ± 0.2 nm [1] |
| Änderung der Austrittsarbeit (Kelvin-Sonde) | Referenz | - 200 meV, in Bezug auf Referenz |

| | | |
|---|---|---|
| ^{[1]} angenommener Brechungsindex n = 1.55 | | |

Die Werte weisen auf eine erfolgreiche Derivatisierung der TiN-Oberfläche mit einer selbstorganisierten Monoschicht von CCY-5-O3P hin.

### Charakterisierung von Phosphonsäuremonoschichten unter Verwendung einer Aluminiumoxid-Zwischenschicht auf der ersten Elektrode (20)

### Herstellung der Monoschichten

Ausgangsmaterial ist ein 525 µm dicker Silizium-Wafer mit einem Durchmesser von 6 Zoll, mit [100]-Orientierung, der mit Bor stark p-dotiert ist und einen spezifischen Widerstand von ca. 0,005 Ω·cm hat.

Dieser Silizium-Wafer wird durch ein ALD-Verfahren (engl.: atomic layer deposition) mit einer etwa 2 nm dicken Al₂O₃ Schicht beschichtet. Vor der Abscheidung von Al₂O₃ wurde der Wafer mit dem nasschemischen Reinigungsverfahren "RCA", das dem Fachmann bekannt ist, gereinigt und in 1 %-ige HF-Lösung getaucht. Direkt im Anschluss wird der Wafer in eine Vakuumkammer transferiert, in der mit Hilfe der Vorläufersubstanzen (precursor) Trimethyl-Aluminium und Wasser Al₂O₃ auf der Waferoberfläche abgeschieden wird. Nach etwa 20 Reaktionszyklen bei 200°C ist die gewünschte Schichtdicke von 2 nm erreicht.

Nach der Abscheidung von Al₂O₃ wird der Wafer in quadratische Teile ("Chips") der Größe 8 mm × 8 mm zerteilt. Die Chips werden jeweils 5 min im Ultraschallbad zunächst in Aceton und anschließend in Isopropanol gereinigt. Danach werden die Chips mit Sauerstoffplasma (100 W, 1 min) behandelt.

Die Chips werden anschließend in eine Lösung (0.04 mmol) der Substanz CY-5-O11P in THF getaucht. Nach 72 Stunden wird der Chip aus der Lösung herausgenommen, mit THF abgespült, mit Stickstoff abgetrocknet, im Ofen für 24 Stunden bei 120°C getempert, nochmals mit einer 1:1 Mischung von THF und Methanol abgespült und erneut mit Stickstoff abgetrocknet.

Im Anschluss an diese Vorbehandlung werden die Chips mit einem Hg-Tropfen (32) als zweite Elektrode elektrisch charakterisiert (Figur 3). Als erste Elektrode dient ein Kupferblock (30), der mit der Unterseite der Chips (stark p-dotiertes Silizium) leitend verbunden ist.

### Meßergebnisse

An die beiden Elektroden (30,32) wird über das Meßgerät (34) eine Gleichspannung angelegt, welche über die Zeit in einer zyklischen Abfolge zwischen einem maximalen negativen (hier -3 V) und maximalen positiven Spannungswert (hier -3 V) bei konstanter Rate (hier 20 mV/s) variiert. In der Figur 4 ist ein solcher Zyklus (41) eingezeichnet. Der resultierende Strom durch die zu charakterisierende Probe (40) wird gemessen und aufgezeichnet.

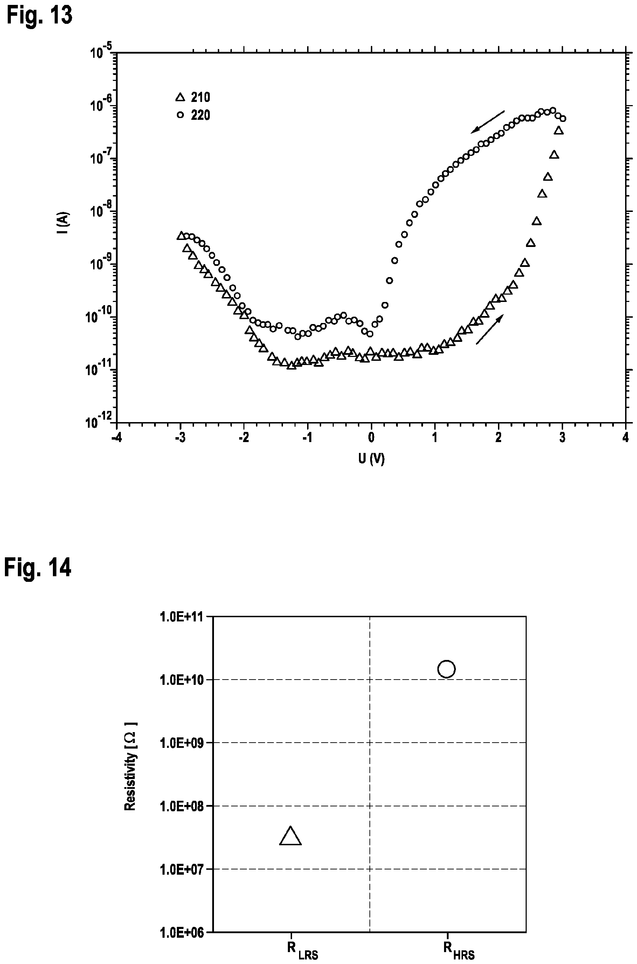

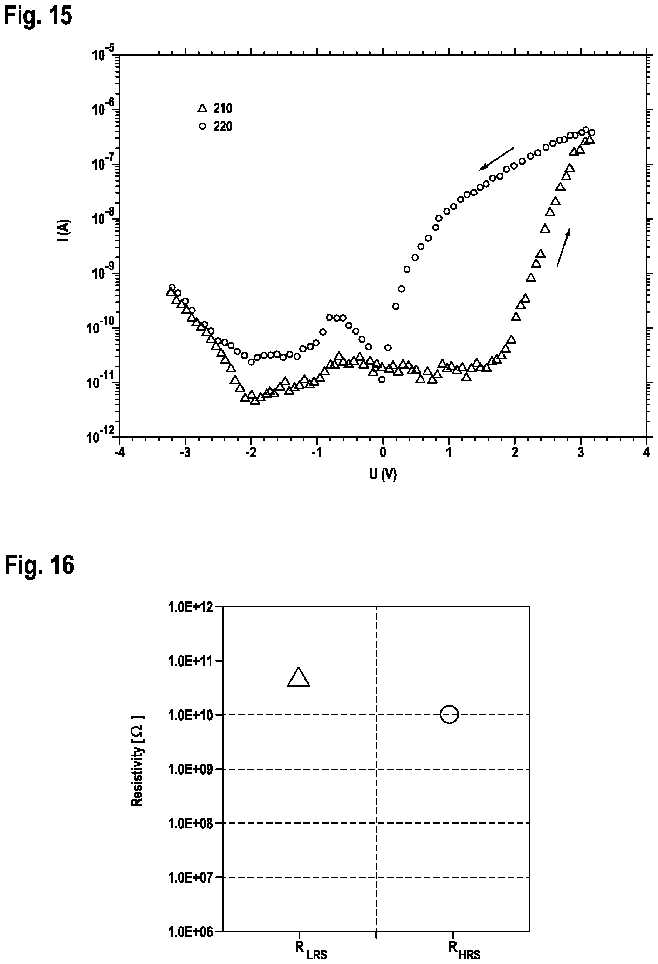

Die aufgezeichneten Ströme für verschiedene Proben (40) werden in Figur 13 und Figur 15 dargestellt und nachfolgend näher erläutert.

Figur 13 zeigt den aufgezeichneten Strom in Abhängigkeit der angelegten Spannung (Strom-Spannungs-Kurve) durch eine Probe (40) mit dem Monolagensystem CY-5-O11P. Die Strom-Spannungs-Kurve stellt die Mittelung der Stromwerte über drei aufeinanderfolgende Zyklen dar.

Man erkennt ein charakteristisches hysteretisches Verhalten im Bereich positiver Vorspannungen des Substrates. Im Bereich negativer Vorspannungen sind geringere Ströme als für positive Vorspannungen und kaum hysteretisches Verhalten zu erkennen. Die Strom-Spannungs-Charakteristik ähnelt der einer Diode. Ein diodenartiges Verhalten, wie es in Figur 13 zu sehen ist, ist für ein memristives Bauteil innerhalb einer integrierten Schaltung besonders wünschenswert.

Die hier gemessenen, geringen Ströme können besonders vorteilhaft für Speicheranwendungen sein. Typische bei Memristoren auf Grundlage der Bildung von Metallfilamenten gemessene ON-Ströme sind sehr hoch (100-mA-Bereich) und stellen eine besondere Problematik in den elektronischen Schaltungen dar (z.B. Leistungsverbrauch, Wärmeentwicklung).

Das mittels Hg-Elektrode gemessene Verhältnis von einem Zustand mit relativ hohem Widerstand (R_{HRS}) zu einem Zustand mit demgegenüber relativ niedrigem Widerstand, nachfolgend R_{HRS}:R_{LRS}-Verhältnis genannt, liegt für das System CY-5-O11P bei ca. 430. Zur Bestimmung des R_{LRS}-Wertes wird die Spannung von 0 V auf 3 V erhöht und von 3 V wieder auf 0 V gefahren. Im Anschluss wird die Spannung in einem Zyklus bei konstanter Rate (hier 20 mV/s) mit einer maximalen negativen Spannung von -0.1 V und einer maximalen positiven Spannung von 0.1 V variiert. Anhand der Steigung der Strom-Spannungs-Kurve von diesem Spannungszyklus kann der Widerstand abgelesen werden. Für den R_{HRS}-Wert wird die Spannung von 0 V auf -3 V, dann von -3 V wieder auf 0 V gefahren. Anschließend wird auf analoge Weise wie für den R_{LRS}-Wert der Widerstand bestimmt. Die R_{LRS}- und R_{HRS}-Werte für das Monolagensystem CY-5-O11P sind in Figur 14 aufgetragen.

Die oben beschriebene Messung zur Bestimmung der Widerstände ist unabhängig von der Messung der in Figur 13 aufgezeigten Strom-Spannungs-Kurven. Zwischen beiden Messungen liegen mehrere Minuten, in denen keine Spannung an dem Bauteil anliegt. Die resistiven Zustände R_{LRS} und R_{HRS} sind unter Umgebungsbedingungen stabil. Die Ergebnisse zeigen, daß sich das System von einem Zustand mit hohem Widerstand zu einem Zustand mit niedrigem Widerstand reversibel schalten läßt. Die Schaltelemente sind somit für die Herstellung nicht-flüchtiger, memristiver Speicher geeignet.

### Referenzmessung

Als Vergleich wurde eine in analoger Weise hergestellte Probe (40) unter Verwendung der nicht lateral fluorierten Referenzverbindung CP-5-O11P untersucht.

Figur 15 zeigt den aufgezeichneten und gemittelten Strom für drei Zyklen der Strom-Spannungs-Charakteristik einer CP-5-O11P-Monoschicht auf einem Al₂O₃-derivatisierten Chip als unfluoriertes Referenzsystem.

Es wird kein Zustand mit niedrigem Widerstand und also kein für memristive Systeme charakteristisches Schaltverhalten beobachtet. Die Hysterese über den gesamten Spannungsbereich ist auf kapazitive Ladeströme zurückzuführen. Insbesondere bleiben die Widerstandswerte (Figur 16) bei kleinen Auslesespannungen im Rahmen der Meßgenauigkeit konstant (unabhängig vom Durchlaufen eines Schaltzyklus gemäß Figur 15).

### Bezugszeichenliste

- 1: Schaltelement
- 10: elektronisches Bauelement (mit Kreuzdrahtvorrichtung)
- 12: äußeres Substrat
- 14: Isolator
- 16: zweite Elektrode
- 18: molekulare Schicht
- 20: erste Elektrode
- 22: Diode
- 24: n+-Schicht
- 26: p+-Schicht
- 30: Kupferplatte
- 32: Hg-Tropfen
- 34: Meßgerät
- 36: Strommeßeinheit
- 38: Spannungsquelle
- 40: Probe
- 41: Meßzyklus
- 50: erste Probe
- 51: zweite Probe
- 52: dritte Probe
- 70: Widerstand
- 71: Spannung
- 90: Kontaktnadel
- 101: 1. Zyklus
- 102: 5. Zyklus
- 103: 10. Zyklus
- 104: 15. Zyklus
- 105: 20. Zyklus
- 120: 1. Zyklus
- 121: 2. Zyklus
- 160: Kontaktfläche der zweiten Elektrode
- 200: Kontaktfläche der ersten Elektrode
- 210: steigende Vorspannung
- 220: sinkende Vorspannung

## Patentansprüche

1. Memristives Bauelement (10) enthaltend ein oder mehrere Schaltelemente (1) umfassend, in dieser Reihenfolge,
eine erste Elektrode (20),
eine molekulare Schicht (18), gebunden an ein Substrat, und
eine zweite Elektrode (16),
wobei die molekulare Schicht durch Chemisorption oder kovalent an das Substrat gebunden ist,
und wobei das Substrat aus der ersten Elektrode (20) und optional einer oder mehreren darüberliegenden Schichten besteht, und wobei die molekulare Schicht im wesentlichen aus einer oder mehreren Verbindungen der Formel I, wie nachfolgend definiert, gebildet wird:
R¹-(A¹-Z¹)ᵣ-B¹-(Z²-A²)ₛ-Sp-G (I)
worin
R¹ H, einen Alkyl- oder Alkoxyrest mit 1 bis 15 C-Atomen, wobei in diesen Resten auch eine oder mehrere CH₂-Gruppen jeweils unabhängig voneinander durch -C≡C- , -CH=CH-, 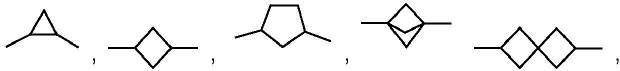 -O-, -S-, -CF₂O-, -OCF₂-, -CO-O-, -O-CO-, -SiR⁰R⁰⁰-, -NH-, -NR⁰- oder -SO₂- so ersetzt sein können, daß O-Atome nicht direkt miteinander verknüpft sind, und worin auch ein oder mehrere H-Atome durch Halogen, CN, SCN oder SF₅ ersetzt sein können,
R⁰, R⁰⁰ gleich oder verschieden einen Alkyl- oder Alkoxyrest mit 1 bis 15 C-Atomen, worin auch ein oder mehrere H-Atome durch Halogen ersetzt sein können,
A¹, A² bei jedem Auftreten gleich oder verschieden einen aromatischen, heteroaromatischen, alicyclischen oder heteroaliphatischen Ring mit 4 bis 25 Ringatomen, der auch kondensierte Ringe enthalten kann und der ein- oder mehrfach durch Y substituiert sein kann,
Y bei jedem Auftreten gleich oder verschieden F, Cl, CN, SCN, SF₅ oder geradkettiges oder verzweigtes, jeweils optional fluoriertes, Alkyl, Alkoxy, Alkylcarbonyl, Alkoxycarbonyl, Alkylcarbonyloxy oder Alkoxycarbonyloxy mit 1 bis 12 C-Atomen,
B¹ 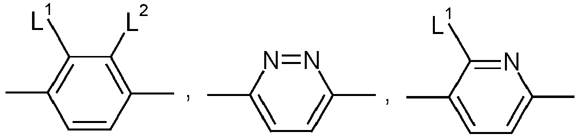 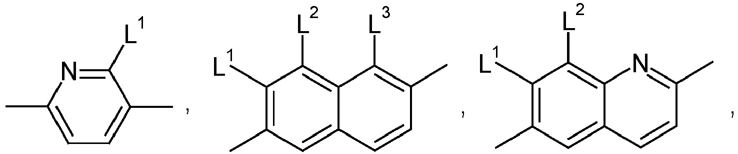 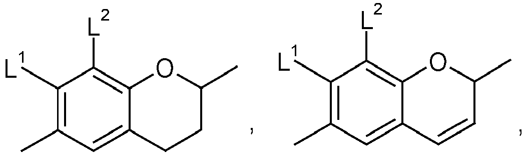 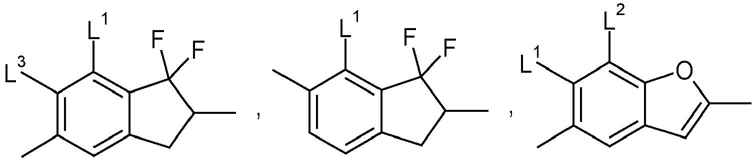 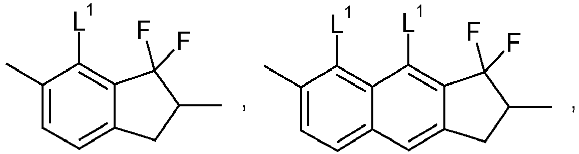 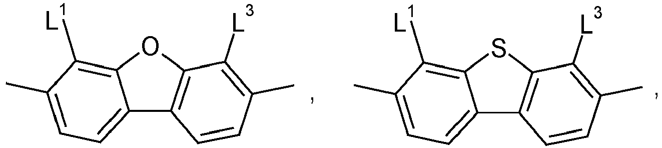 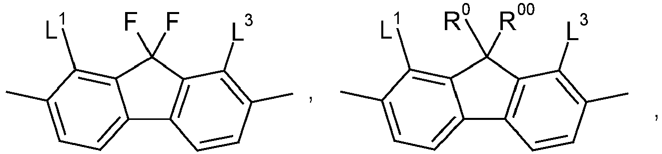 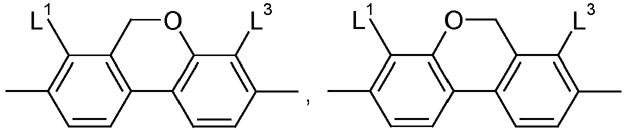 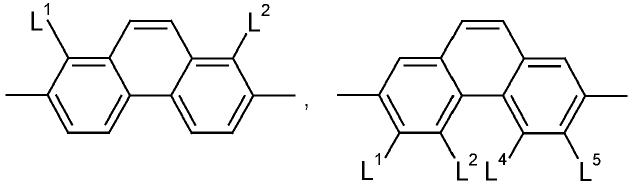 wobei die Gruppen in beide Richtungen orientiert sein können,
L¹ bis L⁵ unabhängig voneinander F, Cl, Br, I, CN, SF₅, CF₃, OCF₃, bevorzugt Cl oder F, wobei L³ alternativ auch H bedeuten kann,
Z¹, Z² bei jedem Auftreten gleich oder verschieden eine Einfachbindung, -CF₂O-, -OCF₂-, -CF₂S-, -SCF₂-, -CH₂O-, -OCH₂- ,-C(O)O-, -OC(O)-, -C(O)S-, -SC(O)-, -CH₂-, -(CH₂)₂-, -(CH₂)₃-, -(CH₂)₄-, -CF₂-, -CF₂-CF₂-, -CF₂-CH₂-, -CH₂-CF₂-, -CH=CH-, -CF=CF- , -CF=CH-, -CH=CF-, -(CH₂)₃O-, -O(CH₂)₃- -C≡C-, -O-, -S-, -C=N-, -N=C-, -N=N-, -N=N(O)-, -N(O)=N-, -N=C-C=N-,
Sp eine Abstandsgruppe oder eine Einfachbindung,
G -CH=CH₂, -OH, -SH, -SO₂OH, -OP(O)(OH)₂,-PO(OH)2, -C(OH)(PO(OH)₂)₂, -COOH, -Si(OR^{x})₃, -SiCl₃,
R^{x} geradkettiges oder verzweigtes Alkyl mit 1 bis 6 C-Atomen, und
r und s unabhängig voneinander 0, 1, 2 oder 3, wobei r + s ≤ 4 ist,
bedeuten.

2. Memristives Bauelement (10) nach Anspruch 1, **dadurch gekennzeichnet, daß** die erste Elektrode (20) das Substrat für die molekulare Schicht darstellt.

3. Memristives Bauelement (10) nach Anspruch 1, **dadurch gekennzeichnet, daß** zwischen der ersten Elektrode (20) und der molekularen Schicht (18) eine Diode (22) angeordnet ist, die das Substrat für die molekulare Schicht (18) darstellt.

4. Memristives Bauelement (10) nach Anspruch 3, **dadurch gekennzeichnet, daß** die Diode (22) eine n+ dotierte Schicht (24) und eine p+ dotierte Schicht (26) umfaßt, wobei die n+ dotierte Schicht (24) oder die p+ dotierte Schicht (26) gemeinsam mit einer halbleitenden ersten Elektrode (20) als kombinierte Elektrode ausgeführt ist.

5. Memristives Bauelement (10) nach Anspruch 1 oder 2, **dadurch gekennzeichnet, daß** zwischen der zweiten Elektrode (16) und der molekularen Schicht (18) eine Diode (22) angeordnet ist.

6. Memristives Bauelement (10) nach einem der Ansprüche 1 bis 5, **dadurch gekennzeichnet, daß** zwischen erster Elektrode (20) und der molekularen Schicht (18) eine Zwischenschicht angeordnet ist, wobei die Zwischenschicht ausgewählt ist aus einem oxidischen oder fluoridischen Material und daß die molekulare Schicht (18) an diese oxidische oder fluoridische Zwischenschicht gebunden ist.

7. Memristives Bauelement (10) nach Anspruch 6, **dadurch gekennzeichnet, daß** die oxidische oder fluoridische Zwischenschicht aus TiO₂, Al₂O₃, HfO₂, SiO₂ oder LiF gebildet wird.

8. Memristives Bauelement (10) nach einem oder mehreren der Ansprüche 1 bis 7, **dadurch gekennzeichnet, daß** die Ankergruppe (G) der Verbindungen der Formel I, wie in Anspruch 1 definiert, ausgewählt ist aus den Gruppen -CH=CH₂, -PO(OH)₂ und -C(OH)(PO(OH)₂)₂.

9. Memristives Bauelement (10) nach einem der Ansprüche 1 bis 8, **dadurch gekennzeichnet, daß** die molekulare Schicht eine molekulare Monoschicht ist.

10. Memristives Bauelement (10) nach einem der Ansprüche 1 bis 9, **dadurch gekennzeichnet, daß** die erste Elektrode (20) aus einem Material besteht ausgewählt aus Elementhalbleitern, Verbindungshalbleitern der Gruppen III-V, Verbindungshalbleitern der Guppen II-VI, Metallen, sowie leitfähigen, oxidischen Materialien.

11. Memristives Bauelement (10) nach Anspruch 10, **dadurch gekennzeichnet, daß** die erste Elektrode (20) aus einem Material besteht ausgewählt aus den Elementhalbleitern Si, Ge, Diamant, Graphit, Graphen, Sn in der alpha Modifikation und Se, den Verbindungshalbleitern GaAs, InAs, InP, GaSb, TaN, TiN, MoN, WN, GaN, CdSe und ZnS, den Metallen Au, Ag, Cu, Al, W, Ta, Ti, Co, Mo, Pt, Ru und Mg sowie den leitfähigen oxidischen Materialien ITO, IGO, IGZO, AZO und FTO.

12. Memristives Bauelement (10) nach einem oder mehreren der Ansprüche 1 bis 11, **dadurch gekennzeichnet, daß** die zweite Elektrode (16) aus einem leitenden oder halbleitenden Material oder einer Kombination mehrerer dieser Materialien gewählt aus Hg, In, Ga, InGa, Ag, Au, Cr, Pt, Pd, Au, Pb, Al, Mg, Zn, Yb, W, CNT, Graphen und leitfähigen Polymeren besteht.

13. Memristives Bauelement (10) nach einem oder mehreren der Ansprüche 1 bis 12, **dadurch gekennzeichnet, daß** die erste Elektrode (20) und die zweite Elektrode (16) metallische Leiter sind oder daß die erste Elektrode (20) halbleitend ist und die zweite Elektrode (16) ein metallischer Leiter ist.

14. Memristives Bauelement (10) nach einem oder mehreren der Ansprüche 1 bis 13, **dadurch gekennzeichnet, daß** das Bauelement (10) eine Vielzahl von Schaltelementen (1) aufweist, wobei die ersten Elektroden (20) und zweiten Elektroden (16) der Schaltelemente (1) eine Kreuzdrahtvorrichtung ausbilden.

15. Memristives Bauelement (10) nach einem oder mehreren der Ansprüche 1 bis 14, **dadurch gekennzeichnet, daß** die Schaltelemente eingerichtet sind, zwischen einem Zustand mit hohem elektrischen Widerstand und einem Zustand mit geringem elektrischen Widerstand zu wechseln, wobei der Quotient zwischen hohem elektrischen Widerstand und niedrigem elektrischen Widerstand zwischen 10 und 100000 liegt.

16. Verfahren zum Betrieb eines memristiven Bauelements (10) nach einem oder mehreren der Ansprüche 1 bis 15, **dadurch gekennzeichnet, daß** ein Schaltelement (1) des memristiven Bauelements (10) in einen Zustand hohen elektrischen Widerstands geschaltet wird, indem eine korrespondierende erste Elektrode (20) auf ein erstes elektrisches Potential und eine korrespondierende zweite Elektrode (16) auf ein zweites elektrisches Potential gelegt wird, wobei der Betrag der Spannung zwischen den beiden Elektroden (16, 20) größer ist, als eine erste Schaltspannung und das erste Potential größer als das zweite Potential ist, ein Schaltelement (1) des elektronischen Bauelements (10) in einen Zustand geringen elektrischen Widerstands geschaltet wird, indem eine korrespondierende erste Elektrode (20) auf ein drittes elektrisches Potential und eine korrespondierende zweite Elektrode (16) auf ein viertes elektrisches Potential gelegt wird, wobei der Betrag der Spannung zwischen den beiden Elektroden (16, 20) größer ist, als eine zweite Schaltspannung und das vierte Potential größer als das dritte Potential ist, und der Zustand eines Schaltelements bestimmt wird, indem zwischen korrespondierenden Elektroden (16, 20) eine Auslesespannung angelegt wird, deren Betrag kleiner als die erste und die zweite Schaltspannung ist und der fließende Strom gemessen wird.

17. Verwendung einer molekularen Schicht wie in Anspruch 1 angegeben in einem memristiven elektronischen Bauelement.

18. Verfahren zur Herstellung eines Bauelements nach einem oder mehreren der Ansprüche 1 bis 15, enthaltend mindestens die folgenden Schritte:
I. Herstellung einer ersten Elektrode (20),
II. Abscheidung einer Monolage (18) enthaltend eine oder mehrere Verbindungen der Formel I,
III. Aufbringen einer zweiten Elektrode (16).

19. Verbindungen der Formeln Ia bis If:
R¹-B¹-Sp-G Ia
R¹-(A¹-Z¹)-B¹-Sp-G Ib
R¹-(A¹-Z¹)₂-B¹-Sp-G Ic
R¹-B¹-( -Z²-A²)-Sp-G Id
R¹-B¹-( Z²-A²)₂-Sp-G Ie
R¹-(A¹-Z¹)-B¹-( Z²-A²-)-Sp-G If
worin
A¹ und A² 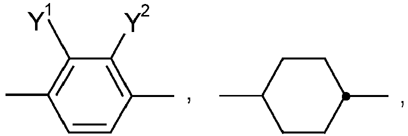
B¹ 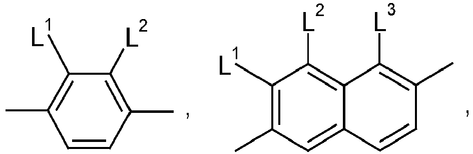 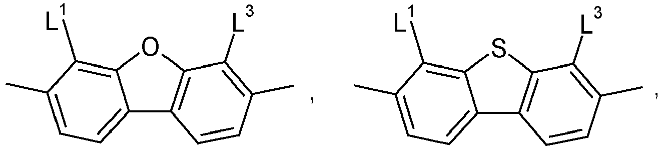 wobei die Gruppen in beide Richtungen orientiert sein können,
R¹ Alkyl mit 1-15 C-Atomen,
L¹ und L² unabhängig voneinander Cl oder F, wobei mindestens einer der Reste L¹ und L² F bedeutet,
L³ F,
Y¹ und Y² unabhängig voneinander H, Cl oder F,
Z¹, Z² unabhängig voneinander eine Einfachbindung, -CF₂O-, -OCF₂-, -CH₂O-, OCH₂-, -CH₂CH₂-,
Sp unverzweigtes 1,ω-Alkylen mit 1 bis 12 C-Atomen,
G -OP(O)(OH)₂, -P(O)(OH)₂, -C(OH)(PO(OH)₂)₂,
bedeuten.

20. Verbindungen der Formeln Ia bis If
R¹-B¹-Sp-G Ia
R¹-(A¹-Z¹)-B¹-Sp-G Ib
R¹-(A¹-Z¹)₂-B¹-Sp-G Ic
R¹-B¹-(Z²-A²)-Sp-G Id
R¹-B¹-(Z²-A²)₂-Sp-G Ie
R¹-(A¹-Z¹)-B¹⁻(Z²⁻A²⁻)-Sp-G If
worin
Sp -O(CF₂)ₚ₁- oder -(CF₂)ₚ₁- und
p1 eine ganze Zahl von 1 bis 12
bedeuten, und die übrigen Gruppen und Parameter die in Anspruch 19 angegebenen Bedeutungen haben.

## Claims

1. Memristive component (10) containing one or more switching elements (1) comprising, in this sequence,
a first electrode (20),
a molecular layer (18), bonded to a substrate, and
a second electrode (16),
where the molecular layer is bonded to the substrate by chemisorption or covalently,
and where the substrate consists of the first electrode (20) and optionally one or more layers lying on top of this, and
where the molecular layer is essentially formed from one or more compounds of the formula I, as defined below:
R¹-(A¹-Z¹)ᵣ-B¹-(Z²-A²)ₛ-Sp-G (I)
in which
R¹ denotes H, an alkyl or alkoxy radical having 1 to 15 C atoms, where, in addition, one or more CH₂ groups in these radicals may each be replaced, independently of one another, by -C≡C- , -CH=CH-, 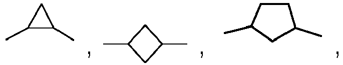 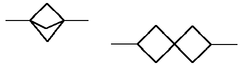 , -O-, -S-, -CF₂O-, -OCF₂-, -CO-O-, -O-CO- , -SiR⁰R⁰⁰- , -NH- , -NR⁰- or -SO₂- in such a way that O atoms are not linked directly to one another, and in which, in addition, one or more H atoms may be replaced by halo-gen, CN, SCN or SF₅,
R⁰, R⁰⁰, identically or differently, denote an alkyl or alkoxy radical having 1 to 15 C atoms, in which, in addition, one or more H atoms may be replaced by halogen
A¹, A² on each occurrence, identically or differently, denote an aromatic, heteroaromatic, alicyclic or heteroaliphatic ring having 4 to 25 ring atoms, which may also contain condensed rings and which may be mono- or polysubstituted by Y
Y on each occurrence, identically or differently, denotes F, Cl, CN, SCN, SF₅ or straight-chain or branched, in each case optionally fluorinated alkyl, alkoxy, alkylcarbonyl, alkoxy-carbonyl, alkylcarbonyloxy or alkoxycarbonyloxy having 1 to 12 C atoms,
B¹ denotes 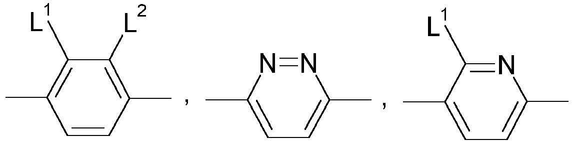 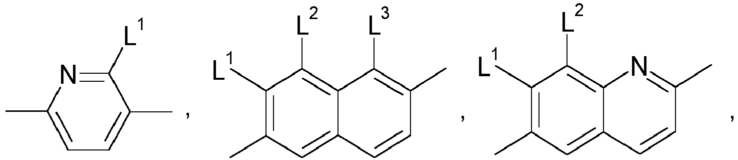 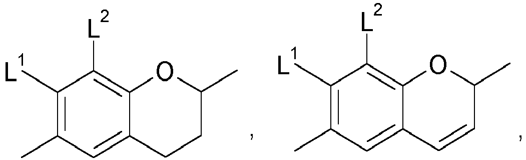 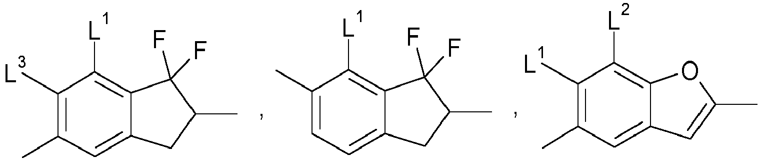 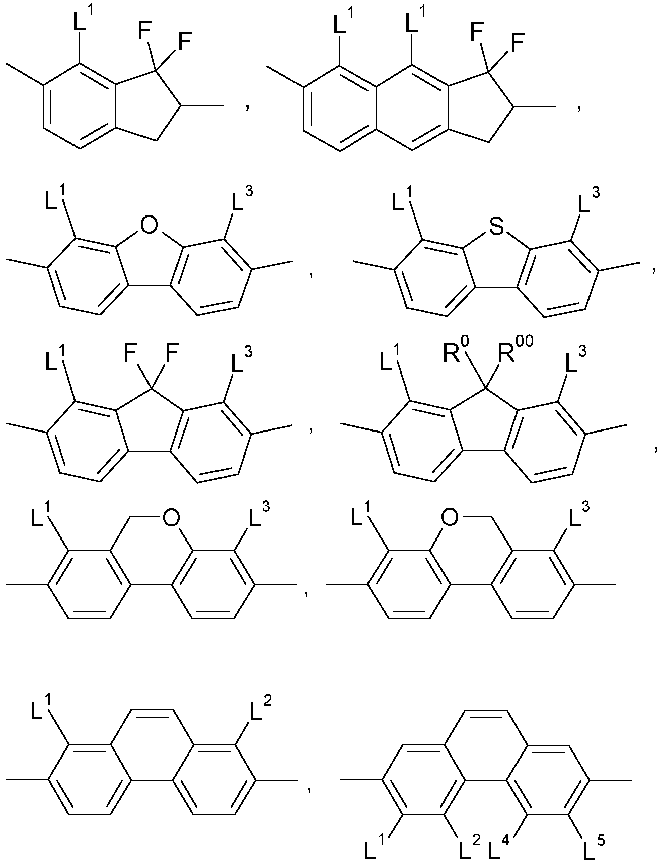 where the groups may be oriented in both directions,
L¹ to L⁵, independently of one another, denote F, Cl, Br, I, CN, SF₅, CF₃, OCF₃, preferably Cl or F, where L³ may alternatively also denote H,
Z¹, Z² on each occurrence, identically or differently, denote a single bond, -CF₂O-, -OCF₂-, -CF₂S-, -SCF₂-, -CH₂O-, -OCH₂-, -C(O)O-, -OC(O)-, -C(O)S-, -SC(O)-, -CH₂-, -(CH₂)₂-, -(CH₂)₃-, -(CH₂)₄-, -CF₂-, -CF₂-CF₂-, -CF₂-CH₂-, -CH₂-CF₂-, -CH=CH-, -CF=CF- , -CF=CH-, -CH=CF-, -(CH₂)₃O-, -O(CH₂)₃-, -C≡C-, -O-, -S-, -C=N-, -N=C-, -N=N-, -N=N(O)-, -N(O)=N-, -N=C-C=N-,
Sp denotes a spacer group or a single bond,
G denotes -CH=CH₂, -OH, -SH, -SO₂OH, -OP(O)(OH)₂, -PO(OH)₂, -C(OH)(PO(OH)₂)₂, -COOH, -Si(OR^{x})₃, -SiCl₃,
R^{x} denotes straight-chain or branched alkyl having 1 to 6 C atoms, and
r and s, independently of one another, denote 0, 1, 2 or 3, where r + s ≤ 4.

2. Memristive component (10) according to Claim 1, **characterised in that** the first electrode (20) represents the substrate for the molecular layer.

3. Memristive component (10) according to Claim 1, **characterised in that** a diode (22), which represents the substrate for the molecular layer (18), is arranged between the first electrode (20) and the molecular layer (18).

4. Memristive component (10) according to Claim 3, **characterised in that** the diode (22) comprises an n+-doped layer (24) and a p+-doped layer (26), where the n+-doped layer (24) or the p+-doped layer (26) is implemented as a combined electrode together with a semiconducting first electrode (20).

5. Memristive component (10) according to Claim 1 or 2, **characterised in that** a diode (22) is arranged between the second electrode (16) and the molecular layer (18).

6. Memristive component (10) according to one of Claims 1 to 5, **characterised in that** an interlayer is arranged between the first electrode (20) and the molecular layer (18), where the interlayer is selected from an oxidic or fluoridic material and **in that** the molecular layer (18) is bonded to this oxidic or fluoridic interlayer.

7. Memristive component (10) according to Claim 6, **characterised in that** the oxidic or fluoridic interlayer is formed from TiO₂, Al₂O₃, HfO₂, SiO₂ or LiF.

8. Memristive component (10) according to one or more of Claims 1 to 7, **characterised in that** the anchor group (G) of the compounds of the formula I, as defined in Claim 1, is selected from the groups -CH=CH₂, -PO(OH)₂ and -C(OH)(PO(OH)₂)₂.

9. Memristive component (10) according to one of claims 1 to 8, **characterised in that** the molecular layer is a molecular monolayer.

10. Memristive component (10) according to one of Claims 1 to 9, **characterised in that** the first electrode (20) consists of a material selected from element semiconductors, compound semiconductors from groups III-V, compound semiconductors from groups II-VI, metals, and conductive, oxidic materials.

11. Memristive component (10) according to Claim 10, **characterised in that** the first electrode (20) consists of a material selected from the element semiconductors Si, Ge, diamond, graphite, graphene, Sn in the alpha modification and Se, the compound semiconductors GaAs, InAs, InP, GaSb, TaN, TiN, MoN, WN, GaN, CdSe and ZnS, the metals Au, Ag, Cu, Al, W, Ta, Ti, Co, Mo, Pt, Ru and Mg and the conductive oxidic materials ITO, IGO, IGZO, AZO and FTO.

12. Memristive component (10) according to one or more of Claims 1 to 11, **characterised in that** the second electrode (16) consists of a conducting or semiconducting material or a combination of a plurality of these materials selected from Hg, In, Ga, InGa, Ag, Au, Cr, Pt, Pd, Au, Pb, Al, Mg, Zn, Yb, W, CNT, graphene and conductive polymers.

13. Memristive component (10) according to one or more of Claims 1 to 12, **characterised in that** the first electrode (20) and the second electrode (16) are metallic conductors or **in that** the first electrode (20) is semiconducting and the second electrode (16) is a metallic conductor.

14. Memristive component (10) according to one or more of Claims 1 to 13, **characterised in that** the component (10) has a multiplicity of switching elements (1), where the first electrodes (20) and second electrodes (16) of the switching elements (1) form a crossbar array.

15. Memristive component (10) according to one or more of Claims 1 to 14, **characterised in that** the switching elements are set up to change between a state having high electrical resistance and a state having low electrical resistance, where the quotient between high electrical resistance and low electrical resistance is between 10 and 100,000.

16. Method for operating a memristive component (10) according to one or more of Claims 1 to 15, **characterised in that** a switching element (1) of the memristive component (10) is switched into a state of high electrical resistance by setting a corresponding first electrode (20) to a first electrical potential and setting a corresponding second electrode (16) to a second electrical potential, where the value of the voltage between the two electrodes (16, 20) is greater than a first switching voltage and the first potential is greater than the second potential, a switching element (1) of the electronic component (10) is switched into a state of low electrical resistance by setting a corresponding first electrode (20) to a third electrical potential and setting a corresponding second electrode (16) to a fourth electrical potential, where the value of the voltage between the two electrodes (16, 20) is greater than a second switching voltage and the fourth potential is greater than the third potential, and the state of a switching element is determined by applying a reading voltage, whose value is smaller than the first and second switching voltages, between corresponding electrodes (16, 20) and measuring the current flowing.

17. Use of a molecular layer as indicated in Claim 1 in a memristive electronic component.

18. Process for the production of a component according to one or more of Claims 1 to 15, comprising at least the following steps:
I. production of a first electrode (20),
II. deposition of a monolayer (18) comprising one or more compounds of the formula I,
III. application of a second electrode (16).

19. Compounds of the formulae Ia to If:
R¹-B¹-Sp-G Ia
R¹-(A¹-Z¹)-B¹-Sp-G Ib
R¹-(A¹-Z¹)₂-B¹-Sp-G Ic
R¹-B¹-( -Z²-A²)-Sp-G Id
R¹-B¹-( Z²-A²)₂-Sp-G le
R¹-(A¹-Z¹)-B¹-( Z²-A²-)-Sp-G If
in which
A¹ and A² denote 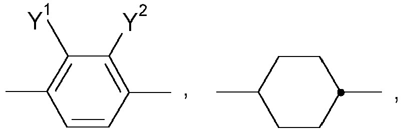
B¹ denotes 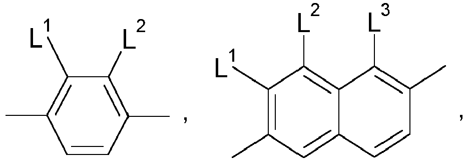 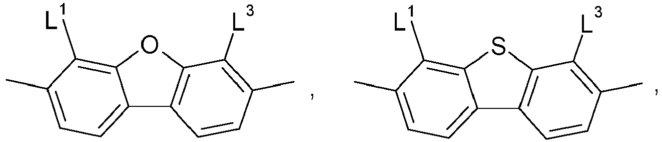 where the groups may be oriented in both directions,
R¹ denotes alkyl having 1-15 C atoms,
L¹ and L², independently of one another, denote Cl or F, where at least one of the radicals L¹ and L² denotes F,
L³ denotes F,
Y¹ and Y², independently of one another, denote H, Cl or F,
Z¹, Z², independently of one another, denote a single bond, -CF₂O-, -OCF₂-, -CH₂O-, OCH₂-, -CH₂CH₂-,
Sp denotes unbranched 1,ω-alkylene having 1 to 12 C atoms,
G denotes -OP(O)(OH)₂, -PO(OH)₂, -COH(PO(OH)₂)₂.

20. Compounds of the formulae Ia to If
R¹-B¹-Sp-G Ia Ia
R¹-(A¹-Z¹)-B¹-Sp-G Ib
R¹-(A¹-Z¹)₂-B¹-Sp-G Ic
R¹-B¹-( -Z²-A²)-Sp-G Id
R¹-B¹-( Z²-A²)₂-Sp-G le
R¹-(A¹-Z¹)-B¹-( Z²-A²-)-Sp-G If
in which
Sp denotes -O(CF₂)ₚ₁- or -(CF₂)ₚ₁- and
p1 denotes an integer from 1 to 12,
and the other groups and parameters have the meanings indicated in Claim 19.

## Revendications

1. Composant memristif contenant un ou plusieurs élément(s) de commutation (10) comprenant, selon cette séquence,
une première électrode (20),
une couche moléculaire (18), qui est liée à un substrat, et
une seconde électrode (16),
dans lequel la couche moléculaire est liée au substrat par chemisorption ou de façon covalente,
et dans lequel le substrat est constitué par la première électrode (20) et en option, par une ou plusieurs couche(s) qui est/sont étendue(s) sur le sommet afférent, et
dans lequel la couche moléculaire est essentiellement formée à partir d'un ou de plusieurs composé(s) de la formule I, telle que définie ci-après :
R¹-(A¹-Z¹)ᵣ-B¹-(Z²-A²)ₛ-Sp-G (I)
dans laquelle
R¹ représente H, un radical alkyle ou alcoxy qui comporte de 1 à 15 atome(s) de C, où, en outre, un ou plusieurs groupe(s) CH₂ dans ces radicaux peut/peuvent chacun être remplacé(s), de manière indépendante les uns des autres, par -C≡C- , -CH=CH-, 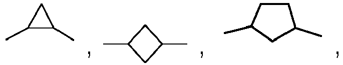 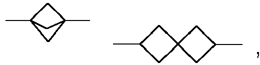 -O-, -S-, -CF₂O-, -OCF₂-, -CO-O-, -O-CO- , -SiR⁰R⁰⁰- , -NH-, -NR⁰-, -SO₂- de telle sorte que des atomes de O ne soient pas liés directement les uns aux autres, et où, en outre, un ou plusieurs atome(s) de H peut/ peuvent être remplacé(s) par halogène, CN, SCN ou SF₅,
R⁰, R⁰⁰ représentent, de manière identique ou différente, un radical alkyle ou alcoxy qui comporte de 1 à 15 atome(s) de C, où, en outre, un ou plusieurs atome(s) de H peut/peuvent être remplacé(s) par halogène,
A¹, A² représentent pour chaque occurrence, de manière identique ou différente, un cycle aromatique, hétéroaromatique, alicyclique ou hétéroaliphatique qui comporte de 4 à 25 atomes de cycle, lequel peut également contenir des cycles condensés et lequel peut être mono- ou polysubstitué par Y,
Y représente pour chaque occurrence, de manière identique ou différente, F, Cl, CN, SCN, SF₅ ou alkyle, alcoxy, alkylcarbonyle, alcoxycarbonyle, alkylcarbonyloxy ou alcoxycarbonyloxy en chaîne droite ou ramifié, dans chaque cas en option fluoré qui comporte de 1 à 12 atome(s) de C,
B¹ représente 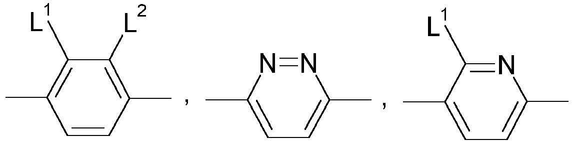 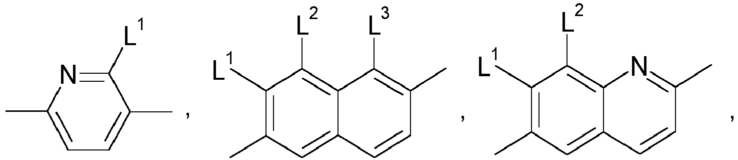 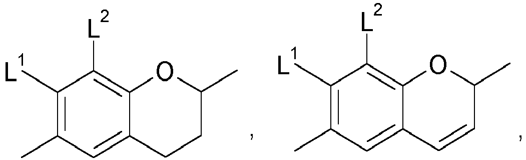 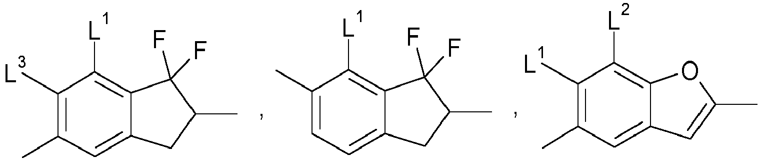 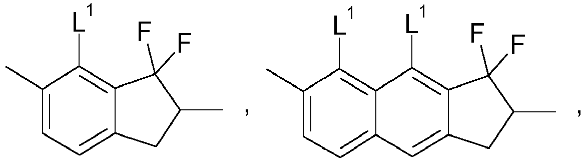 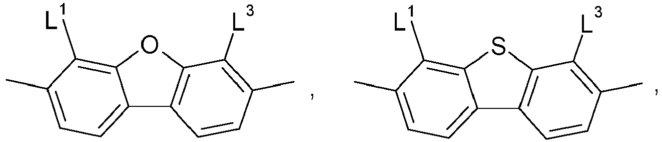 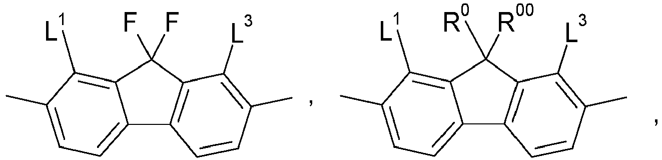 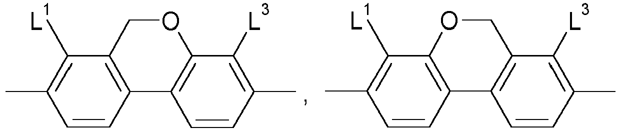 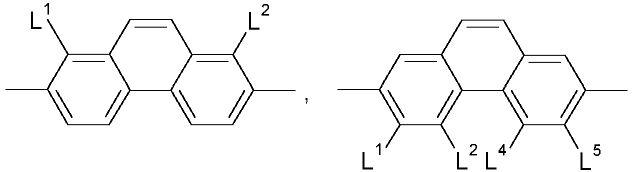 où les groupes peuvent être orientés dans les deux directions,
L¹ à L⁵ représentent, de manière indépendante les uns des autres, F, Cl, Br, I, CN, SF₅, CF₃, OCF₃, de préférence Cl ou F, où L³ peut à titre d'alternative représenter également H,
Z¹, Z² représentent pour chaque occurrence, de manière identique ou différente, une liaison simple, -CF₂O-, -OCF₂-, -CF₂S-, -SCF₂-, -CH₂O-, -OCH₂-, -C(O)O-, -OC(O)-, -C(O)S-, -SC(O)-, -CH₂-, -(CH₂)₂-, -(CH₂)₃-, -(CH₂)₄-, -CF₂-, -CF₂-CF₂-, -CF₂-CH₂-, -CH₂-CF₂-, -CH=CH-, -CF=CF- , -CF=CH-, -CH=CF-, -(CH₂)₃O-, -O(CH₂)₃-, -C≡C-, -O-, -S-, -C=N-, -N=C-, -N=N-, -N=N(O)-, -N(O)=N-, -N=C-C=N-,
Sp représente un groupe d'espaceur ou une liaison simple,
G représente -CH=CH₂, -OH, -SH, -SO₂OH, -OP(O)(OH)₂, -PO(OH)₂, -C(OH)(PO(OH)₂)₂, -COOH, -Si(OR^{x})₃, -SiCl₃,
R^{x} représente alkyle en chaîne droite ou ramifié qui comporte de 1 à 6 atome(s) de C, et
r et s représentent, de manière indépendante l'un de l'autre, 0, 1, 2 ou 3,
où r + s ≤ 4.

2. Composant memristif (10) selon la revendication 1, **caractérisé en ce que** la première électrode (20) représente le substrat pour la couche moléculaire.

3. Composant memristif (10) selon la revendication 1, **caractérisé en ce qu'**une diode (22), qui représente le substrat pour la couche moléculaire (18), est agencée entre la première électrode (20) et la couche moléculaire (18).

4. Composant memristif (10) selon la revendication 3, **caractérisé en ce que** la diode (22) comprend une couche dopée n+ (24) et une couche dopée p+ (26), dans lequel la couche dopée n+ (24) ou la couche dopée p+ (26) est mise en oeuvre en tant qu'électrode combinée en association avec une première électrode de semiconduction (20).

5. Composant memristif (10) selon la revendication 1 ou 2, **caractérisé en ce qu'**une diode (22) est agencée entre la seconde électrode (16) et la couche moléculaire (18).

6. Composant memristif (10) selon l'une des revendications 1 à 5, **caractérisé en ce qu'**une inter-couche est agencée entre la première électrode (20) et la couche moléculaire (18), dans lequel l'inter-couche est sélectionnée parmi un matériau oxydique ou fluorhydrique et **en ce que** la couche moléculaire (18) est liée à cette inter-couche oxydique ou fluorhydrique.

7. Composant memristif (10) selon la revendication 6, **caractérisé en ce que** l'inter-couche oxydique ou fluorhydrique est formée à partir de TiO₂, d'Al₂O₃, de HfO₂, de SiO₂ ou de LiF.

8. Composant memristif (10) selon une ou plusieurs des revendications 1 à 7, **caractérisé en ce que** le groupe d'ancrage (G) des composés de la formule I, telle que définie selon la revendication 1, est sélectionné parmi les groupes -CH=CH₂, -PO(OH)₂ et -C(OH)(PO(OH)₂)₂.

9. Composant memristif (10) selon une ou plusieurs des revendications 1 à 8, **caractérisé en ce que** la couche moléculaire est une monocouche moléculaire.

10. Composant memristif (10) selon une ou plusieurs des revendications 1 à 9, **caractérisé en ce que** la première électrode (20) est constituée par un matériau qui est sélectionné parmi les semiconducteurs élémentaires, les semiconducteurs composites des groupes III-V, les semiconducteurs composites des groupes II-VI, les métaux, et les matériaux oxydiques conducteurs.

11. Composant memristif (10) selon la revendication 10, **caractérisé en ce que** la première électrode (20) est constituée par un matériau qui est sélectionné parmi les semiconducteurs élémentaires Si, Ge, le diamant, le graphite, le graphène, le Sn selon la modification alpha et le Se, les semiconducteurs composites GaAs, InAs, InP, GaSb, TaN, TiN, MoN, WN, GaN, CdSe et ZnS, les métaux Au, Ag, Cu, Al, W, Ta, Ti, Co, Mo, Pt, Ru et Mg et les matériaux oxydiques conducteurs ITO, IGO, IGZO, AZO et FTO.

12. Composant memristif (10) selon une ou plusieurs des revendications 1 à 11, **caractérisé en ce que** la seconde électrode (16) est constituée par un matériau de conduction ou de semiconduction ou par une combinaison d'une pluralité de ces matériaux sélectionnés parmi Hg, In, Ga, InGa, Ag, Au, Cr, Pt, Pd, Au, Pb, Al, Mg, Zn, Yb, W, CNT, le graphène et les polymères conducteurs.

13. Composant memristif (10) selon une ou plusieurs des revendications 1 à 12, **caractérisé en ce que** la première électrode (20) et la seconde électrode (16) sont des conducteurs métalliques ou **en ce que** la première électrode (20) est de semiconduction et la seconde électrode (16) est un conducteur métallique.

14. Composant memristif (10) selon une ou plusieurs des revendications 1 à 13, **caractérisé en ce que** le composant (10) comporte une multiplicité d'éléments de commutation (1), où les premières électrodes (20) et les secondes électrodes (16) des éléments de commutation (1) forment un réseau crossbar.

15. Composant memristif (10) selon une ou plusieurs des revendications 1 à 14, **caractérisé en ce que** les éléments de commutation sont élaborés de manière à ce qu'ils réalisent une modification entre un état qui présente une résistance électrique élevée et un état qui présente une résistance électrique faible, dans lequel le quotient entre la résistance électrique élevée et la résistance électrique faible est entre 10 et 100 000.

16. Procédé pour faire fonctionner un composant memristif (10) selon une ou plusieurs des revendications 1 à 15, **caractérisé en ce qu'**un élément de commutation (1) du composant memristif (10) est commuté dans un état de résistance électrique élevée en établissant une première électrode correspondante (20) à un premier potentiel électrique et en établissant une seconde électrode correspondante (16) à un deuxième potentiel électrique, dans lequel la valeur de la tension entre les deux électrodes (16, 20) est supérieure à une première tension de commutation et le premier potentiel est supérieur au deuxième potentiel, un élément de commutation (1) du composant électronique (10) est commuté dans un état de résistance électrique faible en établissant une première électrode correspondante (20) à un troisième potentiel électrique et en établissant une seconde électrode correspondante (16) à un quatrième potentiel électrique, dans lequel la valeur de la tension entre les deux électrodes (16, 20) est supérieure à une seconde tension de commutation et le quatrième potentiel est supérieur au troisième potentiel, et l'état d'un élément de commutation est déterminé en appliquant une tension de lecture, dont la valeur est inférieure aux première et seconde tensions de commutation, entre des électrodes correspondantes (16, 20) et en mesurant le courant qui circule.

17. Utilisation d'une couche moléculaire telle qu'indiquée selon la revendication 1 dans un composant électronique memristif.

18. Procédé pour la fabrication d'un composant selon une ou plusieurs des revendications 1 à 15, comprenant au moins les étapes qui suivent :
I. la fabrication d'une première électrode (20),
II. le dépôt d'une monocouche (18) qui comprend un ou plusieurs composé(s) de la formule I,
III. l'application d'une seconde électrode (16).

19. Composés des formules la à If :
R¹-B¹-Sp-G la
R¹-(A¹-Z¹)-B¹-Sp-G Ib
R¹-(A¹-Z¹)₂-B¹-Sp-G Ic
R¹-B¹-( -Z²-A²)-Sp-G Id
R¹-B¹-( Z²-A²)₂-Sp-G Ie
R¹-(A¹-Z¹)-B¹-( Z²-A²-)-Sp-G If
dans lesquelles
A¹ et A² représentent 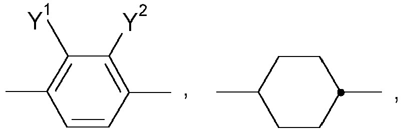
B¹ représente 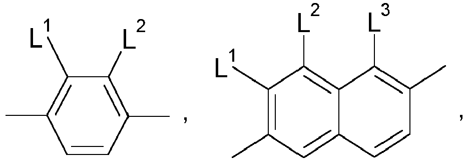 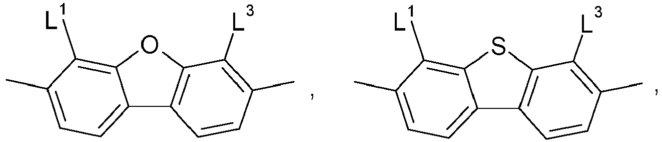 où les groupes peuvent être orientés dans les deux directions,
R¹ représente alkyle qui comporte de 1 à 15 atome(s) de C,
L¹ et L² représentent, de manière indépendante l'un de l'autre, Cl ou F, où au moins l'un des radicaux L¹ et L² représente F,
L³ représente F,
Y¹ et Y² représentent, de manière indépendante l'un de l'autre, H, Cl ou F,
Z¹, Z² représentent, de manière indépendante l'un de l'autre, une liaison simple, -CF₂O-, -OCF₂-, -CH₂O-, OCH₂-, -CH₂CH₂-,
Sp représente 1,ω-alkylène non ramifié qui comporte de 1 à 12 atome(s) de C,
G représente -OP(O)(OH)₂, -PO(OH)₂, -COH(PO(OH)₂)₂.

20. Composés des formules la à If
R¹-B¹-Sp-G la
R¹-(A¹-Z¹)-B¹-Sp-G Ib
R¹-(A¹-Z¹)₂-B¹-Sp-G Ic
R¹-B¹-( -Z²-A²)-Sp-G Id
R¹-B¹-( Z²-A²)₂-Sp-G Ie
R¹-(A¹-Z¹)-B¹-( Z²-A²-)-Sp-G If
dans lesquelles
Sp représente -O(CF₂)ₚ₁- ou -(CF₂)ₚ₁- et
p1 représente un entier de 1 à 12,
et les autres groupes et paramètres présentent les significations qui ont été indiquées selon la revendication 19.
